# EUROPEAN PATENT APPLICATION

(11) **EP 4 524 248 A1**
(43) Date of publication of application: **19.03.2025**
(21) Application number: 23803596.8
(22) Date of filing: 11.05.2023
(51) Int. Cl.: C12N 15/13, A61K 39/395, A61P 35/00, C07K 16/28, C07K 16/30, C07K 16/32, C07K 16/46, C12N 1/15, C12N 1/19, C12N 1/21, C12N 5/10, C12N 15/62, C12N 15/63, C12P 21/02

(54) **ANTI-TAA/ANTI-CD3 MULTISPECIFIC ANTIBODY**

(30) Priority: 12.05.2022 JP 2022078632
(71) Applicant: Astellas Pharma, Inc., Chuo-ku Tokyo 103-8411 (JP)
(72) Inventor: SOGA, Shinji, Tokyo 103-8411 (JP); YAGI, Shigenori, Tokyo 103-8411 (JP); SATAKE, Yoshiki, Tokyo 103-8411 (JP); HONDA, Takashi, Tokyo 103-8411 (JP); SATO, Masahito, Tokyo 103-8411 (JP); CHAEN, Takashi, Tokyo 103-8411 (JP)
(74) Representative: Hoffmann Eitle
(86) International application number: PCT/JP2023/017663
(87) International publication number: WO 2023/219121

(57) **Abstract**

An object of the present invention is to provide an anti-TAA/anti-CD3 multispecific antibody that can be used in treatment of humans. In order to produce an anti-TAA/anti-CD3 multispecific antibody targeting various tumor associated antigens (TAAs), the present inventors utilized known information on anti-CD3 scFv and their own techniques for analyzing protein structure information to obtain 16 types of anti-CD3 scFv having affinity to various CD3 and improved stability. Furthermore, a multispecific antibody combining a plurality of anti-TAA antibodies such as TSPAN8 and CD37 was produced to confirm that the obtained multispecific antibody maintained various binding activities to CD3 and stability. Thus, the obtained 16 types of anti-CD3 scFv can be used in production of an anti-TAA/anti-CD3 multispecific antibody for an arbitrary TAA.

## Description

### TECHNICAL FIELD

The present invention relates to an anti-TAA/anti-CD3 multispecific antibody useful as an active ingredient in a pharmaceutical composition to be used in treatment of a human.

### BACKGROUND ART

Cluster of differentiation 3 (CD3) is a protein transmitting an activation signal to a T cell by forming, on the surface of the T cell, a complex together with a T cell receptor (TCR). The CD3 is a complex consisting of five subunits of gamma (γ), delta (δ), epsilon (ε), zeta (ζ) and eta (η) chains, and the subunits form three dimers, εγ, εδ, and ζζ. CD3 expression is specific to T cells and occurs at all stages of T cell differentiation. Moreover, the CD3 is expressed in both a normal T cell and a tumor T cell, and hence is widely used as a T cell marker (NPL 1).

Cluster of differentiation 137 (CD137, also known as 4-1BB) is a molecule belonging to the tumor necrosis factor receptor superfamily (TNFRSF), which has been reported to be expressed on the surface of an immune cell such as a T cell, a B cell, a natural killer (NK) cell, a dendritic cell, an eosinophil, and a mast cell. In particular, CD137 on a T cell is known to bind to a CD137 ligand on an antigen presenting cell and to be involved in activation and survival of the T cell as a costimulatory molecule (NPL 2).

As a novel cancer therapy using *in vivo* immune system, a bispecific T-cell recruiting antibody has been developed. The most common aspect of the bispecific T-cell recruiting antibody involves simultaneous binding to a tumor associated antigen (TAA) expressed on a cancer cell and CD3 expressed on a T cell, thereby reducing the physical distance between the T cell and the cancer cell and simultaneously inputting a CD3-mediated activation signal to the T cell to induce a cytotoxic effect of the T cell on the cancer cell. Blinatumomab, which targets CD3 and cluster of differentiation 19 (CD19) as antigens, has already been used in the treatment of relapsed or refractory acute lymphoblastic leukemia (ALL) in clinical practice and excellent therapeutic effects have been demonstrated. In recent years, a bispecific T-cell recruiting antibody that binds to both TAA expressed on a cancer cell and CD137 expressed on a T cell has also been developed (NPL 3). Various TAAs are known as targets of the bispecific T-cell recruiting antibody, and for example, bispecific T-cell recruiting antibodies targeting TAAs such as CD33, BCMA, PSMA, and EGFRvIII have been studied and developed as therapeutic agents for various cancers (NPL 4).

The bioactivity of the bispecific T-cell recruiting antibody is known to be affected by various factors, such as formats of the antibody, expression levels of targeted TAAs, epitopes on TAAs recognized by the antibody, affinities to TAA and CD3, and *in vivo* kinetics, and it is considered essential to appropriately control these factors in order to develop a bispecific T-cell recruiting antibody that achieves both efficacy and safety (NPL 5). Regarding safety, generally known issues common to bispecific T-cell recruiting antibodies include side effects (on-target/ off-tumor toxicities) resulting from expression of the targeted TAA in normal tissue other than tumors and cytokine release syndrome (CRS), which is based on activation of the immune system. Potential avenues for avoiding the on-target/off-tumor toxicities include, for example, targeting a molecule, a variant, a post-translational modification, or the like specifically present in cancer tissue as an antigen; providing an antibody with a plurality of binding sites for TAA to increase selectivity for cancer cells expressing an antigen at high density through an avidity effect; and developing an antibody that can be locally activated by a protease or the like present in tumor tissue. A potential way to reduce the risk of CRS has been proposed which involves modulating the affinity of bispecific T-cell recruiting antibodies to CD3 to reduce cytokine production while maintaining cytotoxic activity (NPL 5), for example,and there are reports on the development of anti-CD3 antibodies with different affinities (PTLs 2 to 4).

To further improve therapeutic efficacy, studies have been conducted, in recent years, on various multispecific antibodies, such as a trispecific T-cell recruiting antibody that binds to two different TAAs and one antigen on T cells, a trispecific T-cell recruiting antibody that binds to one TAA and two different antigens on T cells, and a trispecific antibody that binds to antigens on one TAA and respective antigens on T cells and NK cells (NPLs 6 to 8).

### CITATION LIST

### PATENT LITERATURE

PTL 1: Japanese Patent No. 5686953
PTL 2: U.S. Patent No. 9587021
PTL 3: U.S. Patent No. 9701759
PTL 4: U.S. Patent No. 10259887

### NON PATENT LITERATURE

NPL 1: Blood Research, 2022; 57: p.55-61
NPL 2: Cancer Science, 2020; 111(5): p.1461-1467
NPL 3: Science Translational Medicine, 2019; 11(496): eaav5989
NPL 4: Biomarker Research, 2021; 9(1): p.38
NPL 5: Methods, 2019; 154: p.102
NPL 6: Biomecidines, 2020; 8(7): 204
NPL 7: Nature, 2022;603: p.328-334
NPL 8: Theranostics 2023; 13(3): p.1028-1041

### SUMMARY OF INVENTION

### TECHNICAL PROBLEM

An object of the present invention is to provide an anti-TAA/anti-CD3 multispecific antibody that can be used in treatment of a human.

### SOLUTION TO PROBLEM

In the course of investigating production of an anti-TAA/anti-CD3 bispecific antibody targeting various TAAs, the present inventors considered that the optimal affinity of an anti-CD3 single chain variable fragment (hereafter referred to as "anti-CD3 scFv") would vary depending on the expression level of the TAA to be combined and the affinity of an anti-TAA antibody for the TAA. Therefore, the present inventors obtained anti-CD3 scFv antibodies having diverse affinity by using public information and their own techniques for analyzing protein structure information, which were combined with anti-TSPAN8 antibodies to obtain multiple anti-TSPAN8/anti-CD3 bispecific antibodies (Example 1), and also introduced multiple mutations to improve the stability of anti-CD3 scFv to obtain anti-CD3 scFv having both diverse affinity and stability, thus successfully obtaining multiple anti-TSPAN8/anti-CD3 bispecific antibodies having various binding activities to CD3 and stability (Example 2).

In order to confirm that the obtained anti-CD3 scFv could also be combined with any anti-TAA antibody other than anti-TSPAN8 to produce a bispecific antibody having both diverse affinity and stability, the present inventors then produced an anti-CD37/anti-CD3 bispecific antibody by combining an anti-CD37 antibody and anti-CD3 scFv, an anti-SLC34A2/anti-CD3 bispecific antibody, an anti-CLDN4/anti-CD3 bispecific antibody, and an anti-PD-L1/anti-CD3 bispecific antibody, and thus confirmed that these bispecific antibodies maintained both diverse affinity and stability (Examples 3 to 7). In addition, the present inventors confirmed that trispecific antibodies could be provided by combining these bispecific antibodies with an anti-CD137 antibody that binds to CD137 (Example 7).

Specifically, the present invention relates to [1] to [25].
[1] A multispecific antibody including: a heavy chain variable region and a light chain variable region of an anti-tumor associated antigen (TAA) antibody that binds to TAA; and an anti-CD3 scFv region including a heavy chain variable region and a light chain variable region of an anti-CD3 antibody that binds to CD3, wherein the anti-CD3 scFv region is any one of (1) to (16):
   (1) an anti-CD3 scFv region consisting of an amino acid sequence at amino acid positions 1 to 254 of SEQ ID NO: 14;
   (2) an anti-CD3 scFv region consisting of an amino acid sequence at amino acid positions 1 to 254 of SEQ ID NO: 16;
   (3) an anti-CD3 scFv region consisting of an amino acid sequence at amino acid positions 1 to 254 of SEQ ID NO: 18;
   (4) an anti-CD3 scFv region consisting of an amino acid sequence at amino acid positions 1 to 254 of SEQ ID NO: 20;
   (5) an anti-CD3 scFv region consisting of an amino acid sequence at amino acid positions 1 to 254 of SEQ ID NO: 22;
   (6) an anti-CD3 scFv region consisting of an amino acid sequence at amino acid positions 1 to 254 of SEQ ID NO: 24;
   (7) an anti-CD3 scFv region consisting of an amino acid sequence at amino acid positions 1 to 254 of SEQ ID NO: 26;
   (8) an anti-CD3 scFv region consisting of an amino acid sequence at amino acid positions 1 to 254 of SEQ ID NO: 28;
   (9) an anti-CD3 scFv region consisting of an amino acid sequence at amino acid positions 1 to 254 of SEQ ID NO: 30;
   (10) an anti-CD3 scFv region consisting of an amino acid sequence at amino acid positions 1 to 254 of SEQ ID NO: 32;
   (11) an anti-CD3 scFv region consisting of an amino acid sequence at amino acid positions 1 to 254 of SEQ ID NO: 34;
   (12) an anti-CD3 scFv region consisting of an amino acid sequence at amino acid positions 1 to 254 of SEQ ID NO: 36;
   (13) an anti-CD3 scFv region consisting of an amino acid sequence at amino acid positions 1 to 254 of SEQ ID NO: 38;
   (14) an anti-CD3 scFv region consisting of an amino acid sequence at amino acid positions 1 to 254 of SEQ ID NO: 40;
   (15) an anti-CD3 scFv region consisting of an amino acid sequence at amino acid positions 1 to 254 of SEQ ID NO: 42; and
   (16) an anti-CD3 scFv region consisting of an amino acid sequence at amino acid positions 1 to 254 of SEQ ID NO: 44.
[2] The multispecific antibody according to [1], wherein the multispecific antibody is a bispecific antibody having structures (a) to (c):
   (a) a Fab region of an anti-TAA antibody consisting of a heavy chain fragment including a heavy chain variable region of the anti-TAA antibody and a light chain including a light chain variable region of the anti-TAA antibody;
   (b) an anti-CD3 scFV region including a heavy chain variable region and a light chain variable region of an anti-CD3 antibody; and
   (c) an Fc region consisting of a first Fc polypeptide linked to the heavy chain fragment of the Fab region (a) and a second Fc polypeptide linked to the anti-CD3 scFv region (b).
[3] The multispecific antibody according to [2], wherein the Fc region comprises knobs-into-holes mutation or LALA mutation, L234A and L235A, where positions of the mutation are amino acid positions in human Igγ1 constant region according to EU index.
[4] The multispecific antibody according to [2], wherein the Fc region comprises knobs-into-holes mutation and LALA mutation.
[5] The multispecific antibody according to any one of [2] to [4], wherein the Fc region comprises P329A mutation and/or P331G mutation, where a position of the mutation is an amino acid position in human Igγ1 constant region according to EU index.
[6] The multispecific antibody according to any one of [2] to [5], wherein the heavy chain fragment of the Fab region of the anti-TAA antibody and the first Fc polypeptide are linked via a hinge region; and the anti-CD3 scFv region and the second Fc polypeptide are linked via a hinge region.
[7] The multispecific antibody according to [6], wherein the hinge region linking the anti-CD3 scFv region to the second Fc polypeptide comprises C220S mutation, where a position of the mutation is an amino acid position in human Igγ1 constant region according to EU index.
[8] The multispecific antibody according to [6], wherein the Fc region comprises knobs-into-holes mutation, LALA mutation, and P329A and/or P331G mutation, wherein the hinge region linking anti-CD3 scFv region to the second Fc polypeptide comprises C220S mutation, where a position of the mutation is an amino acid position in human Igγ1 constant region according to EU index.
[9] A multispecific antibody which is an anti-tumor associated antigen (TAA)/anti-CD3 bispecific antibody, including: a heavy chain of an anti-TAA antibody that binds to TAA, in which a first Fc polypeptide is linked to a heavy chain fragment including a heavy chain variable region of the anti-TAA antibody; a light chain including a light chain variable region of the anti-TAA antibody; and a polypeptide in which an anti-CD3 scFv region including a heavy chain variable region and a light chain variable region of an anti-CD3 antibody that binds to CD3 and a second Fc polypeptide are linked, wherein the anti-CD3 scFv region is any one of (1) to (16):
   (1) an anti-CD3 scFv region consisting of an amino acid sequence at amino acid positions 1 to 254 of SEQ ID NO: 14;
   (2) an anti-CD3 scFv region consisting of an amino acid sequence at amino acid positions 1 to 254 of SEQ ID NO: 16;
   (3) an anti-CD3 scFv region consisting of an amino acid sequence at amino acid positions 1 to 254 of SEQ ID NO: 18;
   (4) an anti-CD3 scFv region consisting of an amino acid sequence at amino acid positions 1 to 254 of SEQ ID NO: 20;
   (5) an anti-CD3 scFv region consisting of an amino acid sequence at amino acid positions 1 to 254 of SEQ ID NO: 22;
   (6) an anti-CD3 scFv region consisting of an amino acid sequence at amino acid positions 1 to 254 of SEQ ID NO: 24;
   (7) an anti-CD3 scFv region consisting of an amino acid sequence at amino acid positions 1 to 254 of SEQ ID NO: 26;
   (8) an anti-CD3 scFv region consisting of an amino acid sequence at amino acid positions 1 to 254 of SEQ ID NO: 28;
   (9) an anti-CD3 scFv region consisting of an amino acid sequence at amino acid positions 1 to 254 of SEQ ID NO: 30;
   (10) an anti-CD3 scFv region consisting of an amino acid sequence at amino acid positions 1 to 254 of SEQ ID NO: 32;
   (11) an anti-CD3 scFv region consisting of an amino acid sequence at amino acid positions 1 to 254 of SEQ ID NO: 34;
   (12) an anti-CD3 scFv region consisting of an amino acid sequence at amino acid positions 1 to 254 of SEQ ID NO: 36;
   (13) an anti-CD3 scFv region consisting of an amino acid sequence at amino acid positions 1 to 254 of SEQ ID NO: 38;
   (14) an anti-CD3 scFv region consisting of an amino acid sequence at amino acid positions 1 to 254 of SEQ ID NO: 40;
   (15) an anti-CD3 scFv region consisting of an amino acid sequence at amino acid positions 1 to 254 of SEQ ID NO: 42; and
   (16) an anti-CD3 scFv region consisting of an amino acid sequence at amino acid positions 1 to 254 of SEQ ID NO: 44.
[10] A multispecific antibody which is a trispecific antibody obtained by further adding an anti-CD137 scFv region including a heavy chain variable region and a light chain variable region of an anti-CD137 antibody that binds to CD137 to the multispecific antibody according to any one of [2] to [9], wherein the anti-CD137 scFv region is any one of (a) to (e):
   (a) an anti-CD137 scFv region consisting of an amino acid sequence at amino acid positions 1 to 242 of SEQ ID NO: 46;
   (b) an anti-CD137 scFv region consisting of an amino acid sequence at amino acid positions 1 to 244 of SEQ ID NO: 48;
   (c) an anti-CD137 scFv region consisting of an amino acid sequence at amino acid positions 1 to 249 of SEQ ID NO: 50;
   (d) an anti-CD137 scFv region consisting of an amino acid sequence at amino acid positions 1 to 244 of SEQ ID NO: 52; and
   (e) an anti-CD137 scFv region consisting of an amino acid sequence at amino acid positions 1 to 247 of SEQ ID NO: 54.
[11] The multispecific antibody according to [10], wherein the anti-CD137 scFv region is linked to one or both of carboxy terminals of the first Fc polypeptide and the second Fc polypeptide.
[12] A multispecific antibody which is an anti-tumor associated antigen (TAA)/anti-CD3/anti-CD137 trispecific antibody including: a heavy chain of an anti-TAA antibody that binds to TAA, in which a first Fc polypeptide is linked to a heavy chain fragment including a heavy chain variable region of the anti-TAA antibody; a light chain including a light chain variable region of the anti-TAA antibody; and a polypeptide in which an anti-CD3 scFv region including a heavy chain variable region and a light chain variable region of an anti-CD3 antibody that binds to CD3 and a second Fc polypeptide are linked, wherein an anti-CD137 scFv region including a heavy chain variable region and a light chain variable region of an anti-CD137 antibody is also linked to one or both of carboxy terminals of the first Fc polypeptide and the second Fc polypeptide,
   wherein the anti-CD3 scFv region is any one of (1) to (16):
      (1) an anti-CD3 scFv region consisting of an amino acid sequence at amino acid positions 1 to 254 of SEQ ID NO: 14;
      (2) an anti-CD3 scFv region consisting of an amino acid sequence at amino acid positions 1 to 254 of SEQ ID NO: 16;
      (3) an anti-CD3 scFv region consisting of an amino acid sequence at amino acid positions 1 to 254 of SEQ ID NO: 18;
      (4) an anti-CD3 scFv region consisting of an amino acid sequence at amino acid positions 1 to 254 of SEQ ID NO: 20;
      (5) an anti-CD3 scFv region consisting of an amino acid sequence at amino acid positions 1 to 254 of SEQ ID NO: 22;
      (6) an anti-CD3 scFv region consisting of an amino acid sequence at amino acid positions 1 to 254 of SEQ ID NO: 24;
      (7) an anti-CD3 scFv region consisting of an amino acid sequence at amino acid positions 1 to 254 of SEQ ID NO: 26;
      (8) an anti-CD3 scFv region consisting of an amino acid sequence at amino acid positions 1 to 254 of SEQ ID NO: 28;
      (9) an anti-CD3 scFv region consisting of an amino acid sequence at amino acid positions 1 to 254 of SEQ ID NO: 30;
      (10) an anti-CD3 scFv region consisting of an amino acid sequence at amino acid positions 1 to 254 of SEQ ID NO: 32;
      (11) an anti-CD3 scFv region consisting of an amino acid sequence at amino acid positions 1 to 254 of SEQ ID NO: 34;
      (12) an anti-CD3 scFv region consisting of an amino acid sequence at amino acid positions 1 to 254 of SEQ ID NO: 36;
      (13) an anti-CD3 scFv region consisting of an amino acid sequence at amino acid positions 1 to 254 of SEQ ID NO: 38;
      (14) an anti-CD3 scFv region consisting of an amino acid sequence at amino acid positions 1 to 254 of SEQ ID NO: 40;
      (15) an anti-CD3 scFv region consisting of an amino acid sequence at amino acid positions 1 to 254 of SEQ ID NO: 42; and
      (16) an anti-CD3 scFv region consisting of an amino acid sequence at amino acid positions 1 to 254 of SEQ ID NO: 44, and
   wherein the anti-CD137 scFv region is any one of (a) to (e):
      (a) an anti-CD137 scFv region consisting of an amino acid sequence at amino acid positions 1 to 242 of SEQ ID NO: 46;
      (b) an anti-CD137 scFv region consisting of an amino acid sequence at amino acid positions 1 to 244 of SEQ ID NO: 48;
      (c) an anti-CD137 scFv region consisting of an amino acid sequence at amino acid positions 1 to 249 of SEQ ID NO: 50;
      (d) an anti-CD137 scFv region consisting of an amino acid sequence at amino acid positions 1 to 244 of SEQ ID NO: 52; and
      (e) an anti-CD137 scFv region consisting of an amino acid sequence at amino acid positions 1 to 247 of SEQ ID NO: 54.
[13] The multispecific antibody according to any one of [1] to [12], wherein the anti-TAA antibody is an anti-TAA antibody that binds to any one of TAAs:
   HER2, HER3, HER4, CD19, CD20, CD22, CD33, CD38, CD40, CD44, CD70, CD123, CD138, CXCR3, CXCR5, CCR3, CCR4, CCR9, CRTH2, PMCH, CD4, CD25, CD200, endoplasmin, BCMA, CD276, TSPAN8, CLDN4, CLDN6, CLDN18.2, ENPP3, SLC34A2, TROP2, MerTK, Nectin-4, ASGR1, mesothelin, PSMA, LIV-1, MUC1, MUC2, MUC4, MUC16, CDH6, CEACAM1, CEACAM3, CEACAM4, CEACAM5, CEACAM6, CEACAM7, CEACAM16, CEACAM18, CEACAM19, CEACAM20, CEACAM21, URLC10, NY-ESO-1, GAA, OFA, cyclinB1, WT-1, CEF, VEGRR1, VEGFR2, TTK, HPV16, HPV16E7, HPV18, CEAIMA910, KOC1, SL-701, MART-1, gp100, tyrosinase, survivin, MAGE-3.1, MAGE-10.A2, OVA BiP, gp209-2M, melan-A, NA17.A2, KOC1, CO16, DEPDC1, MPHOSPH1, MAGE12, ONT-10, GD2L, GD3L, URLC10, CDCA1, TF, PSA, TERT, STF-II, G17DT, ICT-107, Dex2, hTERT, PAP, TRP2, LRRC15, c-Met, PD-L1, FAP, EGFR, B3-H3, GPC3, and 5T4.
[14] The multispecific antibody according to any one of [1] to [13], which is post-translationally modified.
[15] The multispecific antibody according to [14], wherein the post-translational modification is pyroglutamylation at an amino terminal of the heavy chain variable region and/or lysine deletion at a heavy chain carboxy terminal.
[16] A polynucleotide for use in production of the multispecific antibody according to any one of [1] to [15], the polynucleotide being selected from the group consisting of (1) to (16):
   (1) a polynucleotide having a nucleotide sequence encoding the anti-CD3 scFv region consisting of an amino acid sequence at amino acid positions 1 to 254 of SEQ ID NO: 14;
   (2) a polynucleotide having a nucleotide sequence encoding the anti-CD3 scFv region consisting of an amino acid sequence at amino acid positions 1 to 254 of SEQ ID NO: 16;
   (3) a polynucleotide having a nucleotide sequence encoding the anti-CD3 scFv region consisting of an amino acid sequence at amino acid positions 1 to 254 of SEQ ID NO: 18;
   (4) a polynucleotide having a nucleotide sequence encoding the anti-CD3 scFv region consisting of an amino acid sequence at amino acid positions 1 to 254 of SEQ ID NO: 20;
   (5) a polynucleotide having a nucleotide sequence encoding the anti-CD3 scFv region consisting of an amino acid sequence at amino acid positions 1 to 254 of SEQ ID NO: 22;
   (6) a polynucleotide having a nucleotide sequence encoding the anti-CD3 scFv region consisting of an amino acid sequence at amino acid positions 1 to 254 of SEQ ID NO: 24;
   (7) a polynucleotide having a nucleotide sequence encoding the anti-CD3 scFv region consisting of an amino acid sequence at amino acid positions 1 to 254 of SEQ ID NO: 26;
   (8) a polynucleotide having a nucleotide sequence encoding the anti-CD3 scFv region consisting of an amino acid sequence at amino acid positions 1 to 254 of SEQ ID NO: 28;
   (9) a polynucleotide having a nucleotide sequence encoding the anti-CD3 scFv region consisting of an amino acid sequence at amino acid positions 1 to 254 of SEQ ID NO: 30;
   (10) a polynucleotide having a nucleotide sequence encoding the anti-CD3 scFv region consisting of an amino acid sequence at amino acid positions 1 to 254 of SEQ ID NO: 32;
   (11) a polynucleotide having a nucleotide sequence encoding the anti-CD3 scFv region consisting of an amino acid sequence at amino acid positions 1 to 254 of SEQ ID NO: 34;
   (12) a polynucleotide having a nucleotide sequence encoding the anti-CD3 scFv region consisting of an amino acid sequence at amino acid positions 1 to 254 of SEQ ID NO: 36;
   (13) a polynucleotide having a nucleotide sequence encoding the anti-CD3 scFv region consisting of an amino acid sequence at amino acid positions 1 to 254 of SEQ ID NO: 38;
   (14) a polynucleotide having a nucleotide sequence encoding the anti-CD3 scFv region consisting of an amino acid sequence at amino acid positions 1 to 254 of SEQ ID NO: 40;
   (15) a polynucleotide having a nucleotide sequence encoding the anti-CD3 scFv region consisting of an amino acid sequence at amino acid positions 1 to 254 of SEQ ID NO: 42; and
   (16) a polynucleotide having a nucleotide sequence encoding the anti-CD3 scFv region consisting of an amino acid sequence at amino acid positions 1 to 254 of SEQ ID NO: 44.
[17] A polynucleotide for use in production of the multispecific antibody according to any one of [10] to [15], the polynucleotide being selected from the group consisting of (a) to (e):
   (a) a polynucleotide having a nucleotide sequence encoding an anti-CD137 scFv region consisting of an amino acid sequence at amino acid positions 1 to 242 of SEQ ID NO: 46;
   (b) a polynucleotide having a nucleotide sequence encoding an anti-CD137 scFv region consisting of an amino acid sequence at amino acid positions 1 to 244 of SEQ ID NO: 48;
   (c) a polynucleotide having a nucleotide sequence encoding an anti-CD137 scFv region consisting of an amino acid sequence at amino acid positions 1 to 249 of SEQ ID NO: 50;
   (d) a polynucleotide having a nucleotide sequence encoding an anti-CD137 scFv region consisting of an amino acid sequence at amino acid positions 1 to 244 of SEQ ID NO: 52; and
   (e) a polynucleotide having a nucleotide sequence encoding an anti-CD137 scFv region consisting of an amino acid sequence at amino acid positions 1 to 247 of SEQ ID NO: 54.
[18] An expression vector including the polynucleotide according to [16] or [17].
[19] A host cell including the polynucleotide according to [16] or [17] or the expression vector according to [18].
[20] A production method including a step of culturing the host cell according to [19].
[21] A pharmaceutical composition including the multispecific antibody according to any one of [1] to [15] and a pharmaceutically acceptable excipient.
[22] The multispecific antibody according to any one of [1] to [15], for use of treatment cancer.
[23] The pharmaceutical composition according to [21], for use of treatment cancer.
[24] A method for treating cancer including administering, to a subject, a therapeutically effective amount of the multispecific antibody according to any one of [1] to [15].
[25] Use of the multispecific antibody according to any one of [1] to [15] in production of a pharmaceutical composition for the treatment of cancer.

### ADVANTAGEOUS EFFECTS OF INVENTION

The anti-CD3 scFv of the present invention can be combined with an antibody for an arbitrary TAA (anti-TAA antibody) to provide an anti-TAA/anti-CD3 multispecific antibody (including a bispecific antibody or a trispecific antibody), which can bind to both an arbitrary TAA and CD3 and enhance a killing effect of a T cell on a cancer cell by reducing the physical distance between the cancer cell and the T cell. Accordingly, the anti-TAA/anti-CD3 multispecific antibody of the present invention and a pharmaceutical composition containing the multispecific antibody can be used for treating cancer.

### BRIEF DESCRIPTION OF DRAWINGS

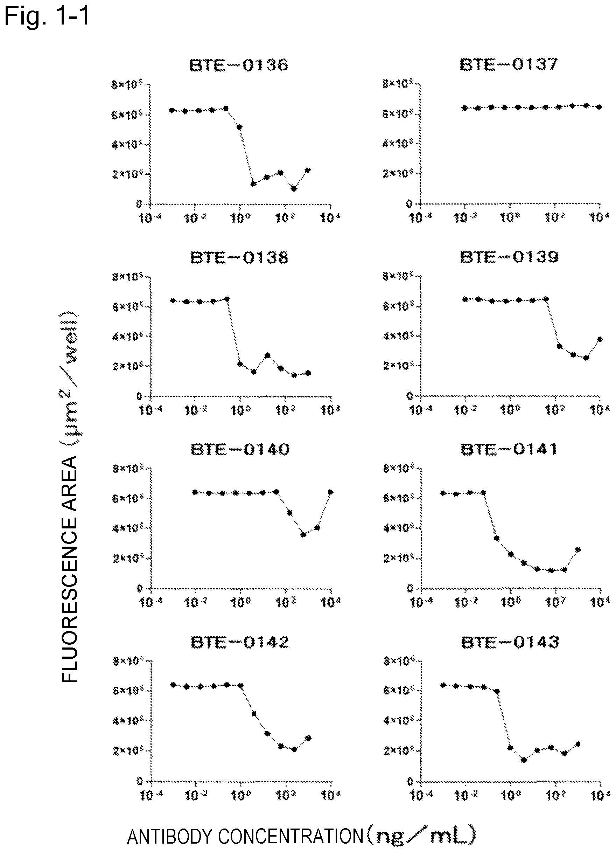

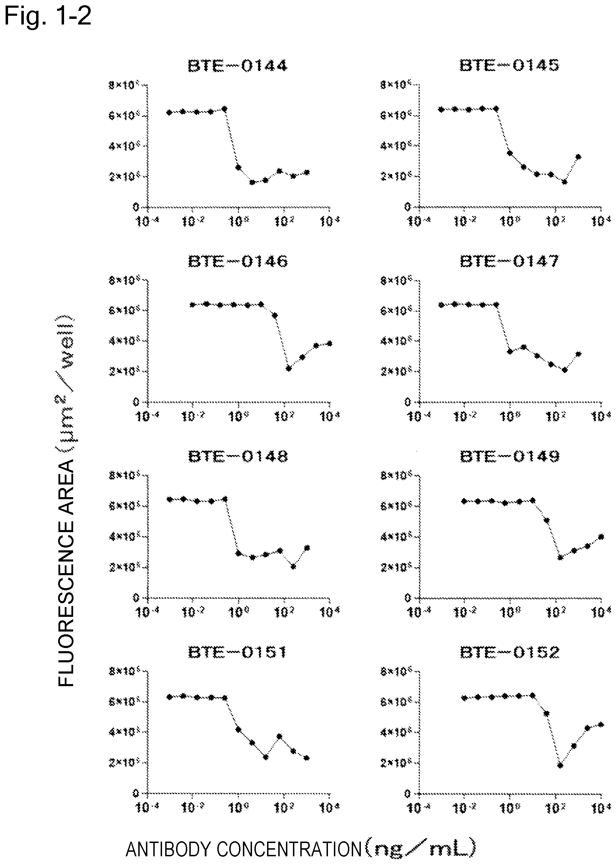

[Fig. 1-1] Fig. 1-1 shows evaluation results of cytotoxic activity of an anti-CD37/anti-CD3 bispecific antibody in a co-culture system of CHO-K1_human CD37/GFPSpark cell and expanded pan T cell. The abscissa indicates the antibody concentration, and the ordinate indicates the GFPSpark fluorescence area of each well. Experiments were conducted in triplicate, and average values for each condition were indicated by symbols and connected by a polygonal line.
[Fig. 1-2] Fig. 1-2 shows evaluation results of cytotoxic activity of an anti-CD37/anti-CD3 bispecific antibody in a co-culture system of CHO-K1_human CD37/GFPSpark cell and expanded pan T cell. The abscissa indicates the antibody concentration, and the ordinate indicates the GFPSpark fluorescence area of each well. Experiments were conducted in triplicate, and average values for each condition were indicated by symbols and connected by a polygonal line.

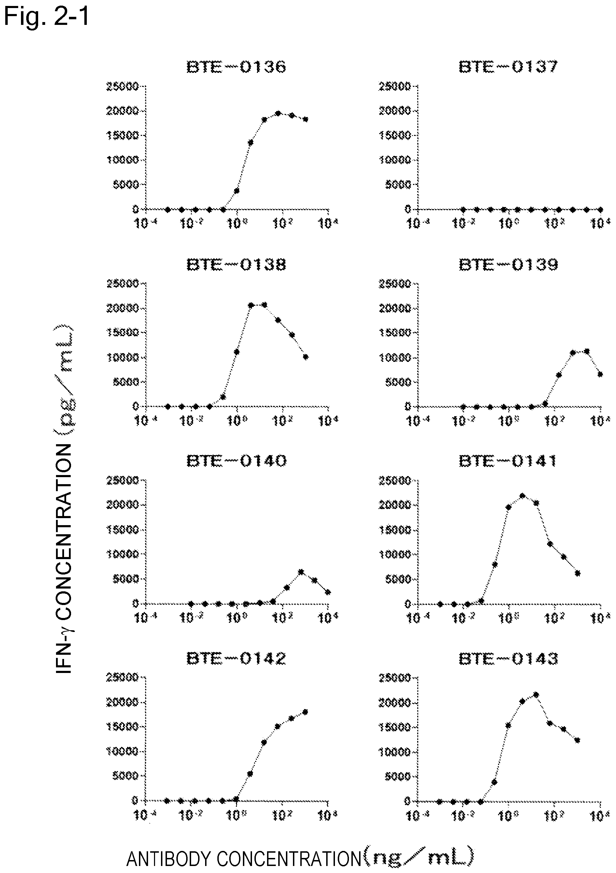

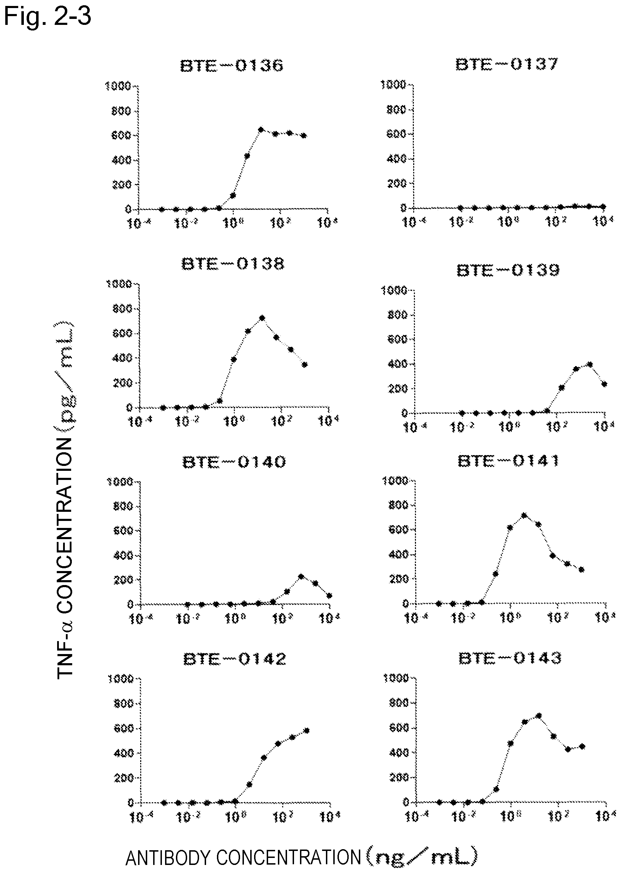

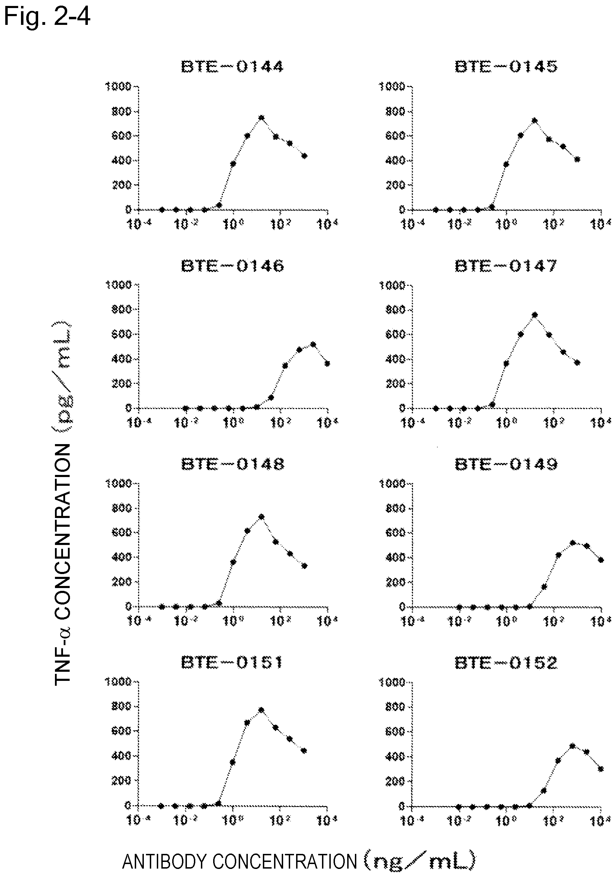

[Fig. 2-1] Fig. 2-1 shows evaluation results of IFN-y secretion-inducing activity of an anti-CD37/anti-CD3 bispecific antibody in a co-culture system of CHO-K1_human CD37/GFPSpark cell and expanded pan T cell. The abscissa indicates the antibody concentration, and the ordinate indicates the IFN-y concentration in a culture supernatant. Experiments were conducted in triplicate, and average values for each condition were indicated by symbols and connected by a polygonal line.

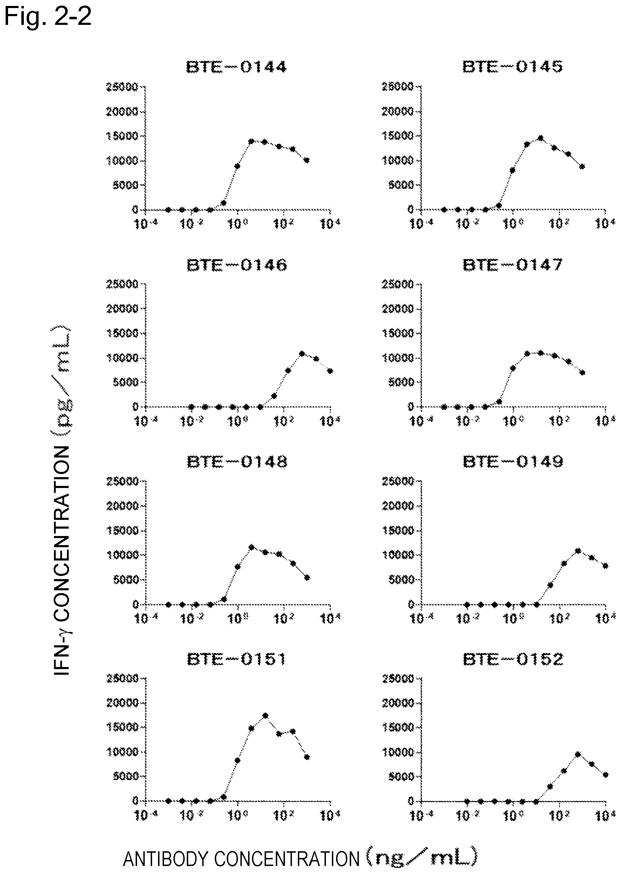

[Fig. 2-2] Fig. 2-2 shows evaluation results of IFN-y secretion-inducing activity of an anti-CD37/anti-CD3 bispecific antibody in a co-culture system of CHO-K1_human CD37/GFPSpark cell and expanded pan T cell. The abscissa indicates the antibody concentration, and the ordinate indicates the IFN-y concentration in a culture supernatant. Experiments were conducted in triplicate, and average values for each condition were indicated by symbols and connected by a polygonal line.
[Fig. 2-3] Fig. 2-3 shows evaluation results of TNF-α secretion-inducing activity of an anti-CD37/anti-CD3 bispecific antibody in a co-culture system of CHO-K1_human CD37/GFPSpark cell and expanded pan T cell. The abscissa indicates the antibody concentration, and the ordinate indicates the TNF-α concentration in a culture supernatant. Experiments were conducted in triplicate, and average values for each condition were indicated by symbols and connected by a polygonal line.
[Fig. 2-4] Fig. 2-4 shows evaluation results of TNF-α secretion-inducing activity of an anti-CD37/anti-CD3 bispecific antibody in a co-culture system of CHO-K1_human CD37/GFPSpark cell and expanded pan T cell. The abscissa indicates the antibody concentration, and the ordinate indicates the TNF-α concentration in a culture supernatant. Experiments were conducted in triplicate, and average values for each condition were indicated by symbols and connected by a polygonal line.

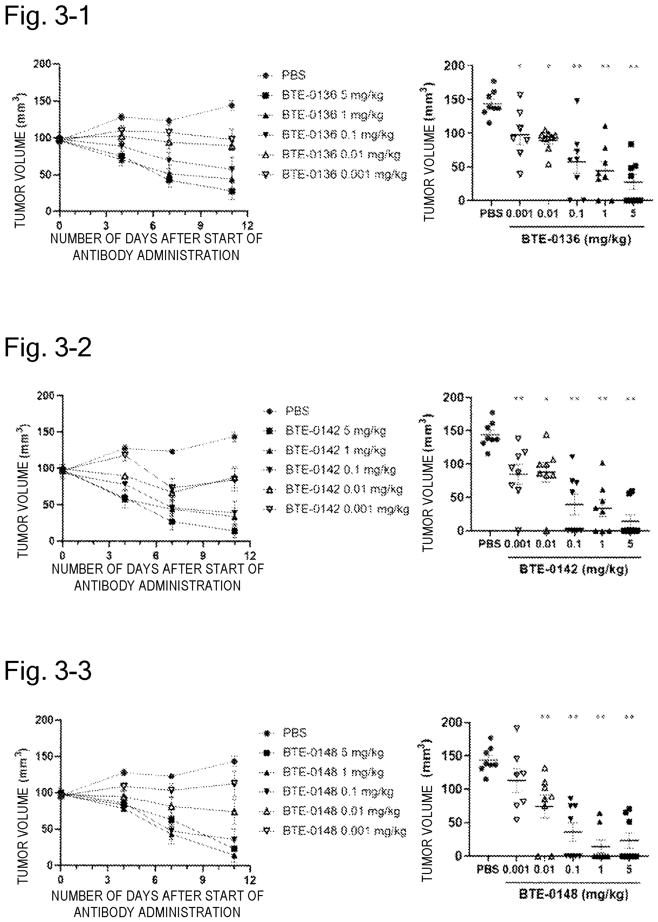

[Fig. 3-1] Fig. 3-1 shows an anti-tumor effect of BTE-0136 in a human PBMC-transferred SU-DHL-6 cell subcutaneous cancer-bearing model. The abscissa in the left graph indicates the number of days after the start of antibody administration, the ordinate indicates tumor volume, and an error bar indicates a standard error. The right graph shows the value of tumor volume of each individual 11 days after the start of administration. The abscissa indicates a dose of BTE-0136, and the ordinate indicates tumor volume. The horizontal line indicates an average value, and an error bar indicates a standard error. A significant probability, p-value, was obtained by comparing a tumor volume in a PBS-treated group with a tumor volume in a BTE-0136-treated group at each dose by Dunnett's multiple comparison test. In the graph, ** indicates a group having a p-value smaller than a significance level of 0.01, and * indicates a group having a p-value smaller than a significance level of 0.05.
[Fig. 3-2] Fig. 3-2 shows an anti-tumor effect of BTE-0142 in a human PBMC-transferred SU-DHL-6 cell subcutaneous cancer-bearing model. The abscissa in the left graph indicates the number of days after the start of antibody administration, the ordinate indicates tumor volume, and an error bar indicates a standard error. The right graph shows the value of tumor volume of each individual 11 days after the start of administration. The abscissa indicates a dose of BTE-0142, and the ordinate indicates tumor volume. The horizontal line indicates an average value, and an error bar indicates a standard error. A significant probability, p-value, was obtained by comparing a tumor volume in a PBS-treated group with a tumor volume in a BTE-0142-treated group at each dose by Dunnett's multiple comparison test. In the graph, ** indicates a group having a p-value smaller than a significance level of 0.01, and * indicates a group having a p-value smaller than a significance level of 0.05.
[Fig. 3-3] Fig. 3-3 shows an anti-tumor effect of BTE-0148 in a human PBMC-transferred SU-DHL-6 cell subcutaneous cancer-bearing model. The abscissa in the left graph indicates the number of days after the start of antibody administration, the ordinate indicates tumor volume, and an error bar indicates a standard error. The right graph shows the value of tumor volume of each individual 11 days after the start of administration, the abscissa indicates a dose of BTE-0148, and the ordinate indicates tumor volume. The horizontal line indicates an average value, and an error bar indicates a standard error. A significant probability, p-value, was obtained by comparing a tumor volume in a PBS-treated group with a tumor volume in a BTE-0148-treated group at each dose by Dunnett's multiple comparison test. In the graph, ** indicates a group having a p-value smaller than a significance level of 0.01.

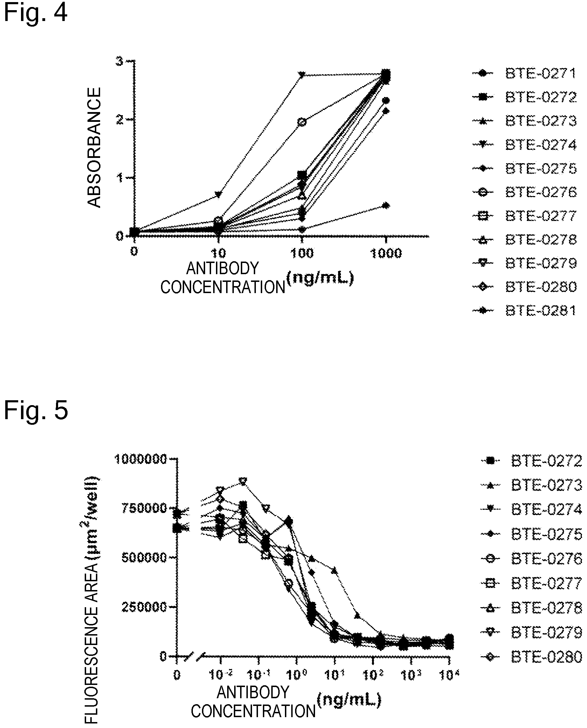

[Fig. 4] Fig. 4 indicates binding activity of 11 types of anti-SLC34A2/anti-CD3 bispecific antibodies to CD3. The abscissa indicates the antibody concentration, and the ordinate indicates the absorbance (binding amount of the antibody).
[Fig. 5] Fig. 5 shows evaluation results of cytotoxic activity of an anti-SLC34A2/anti-CD3 bispecific antibody in a co-culture system of OVCAR-3_GFPSpark cell and expanded pan T cell. The abscissa indicates the antibody concentration, and the ordinate indicates the GFPSpark fluorescence area of each well. Experiments were conducted in triplicate, and average values for each condition were indicated by symbols and connected by a polygonal line.

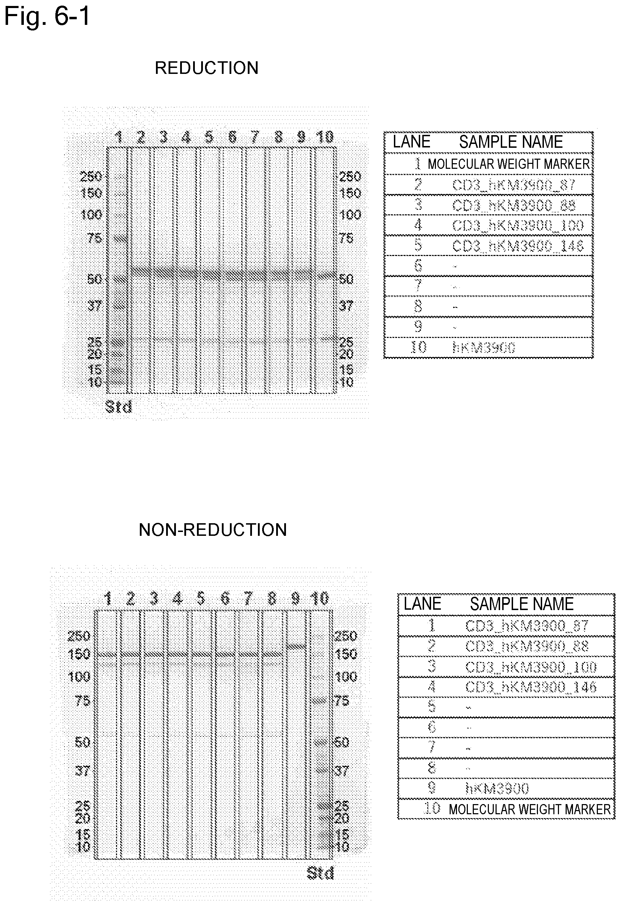

[Fig. 6-1] Fig. 6-1 shows results of SDS-PAGE analysis of anti-CLDN4/anti-CD3 bispecific antibody samples treated under reducing and non-reducing conditions.

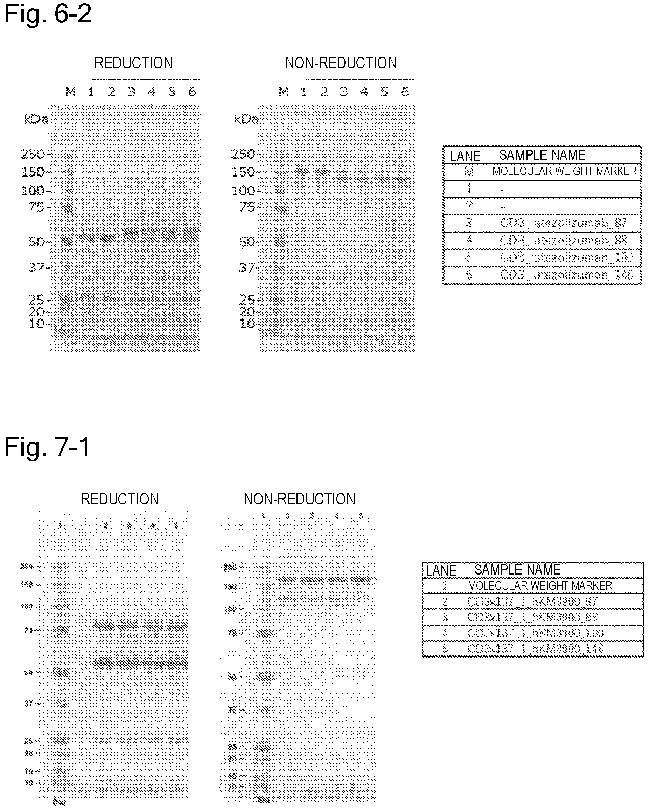

[Fig. 6-2] Fig. 6-2 shows results of SDS-PAGE analysis of anti-PD-L1/anti-CD3 bispecific antibody samples treated under reducing and non-reducing conditions.
[Fig. 7-1] Fig. 7-1 shows results of SDS-PAGE analysis of samples anti-CLDN4/anti-CD3/anti-CD137 trispecific antibody treated under reducing and non-reducing conditions.

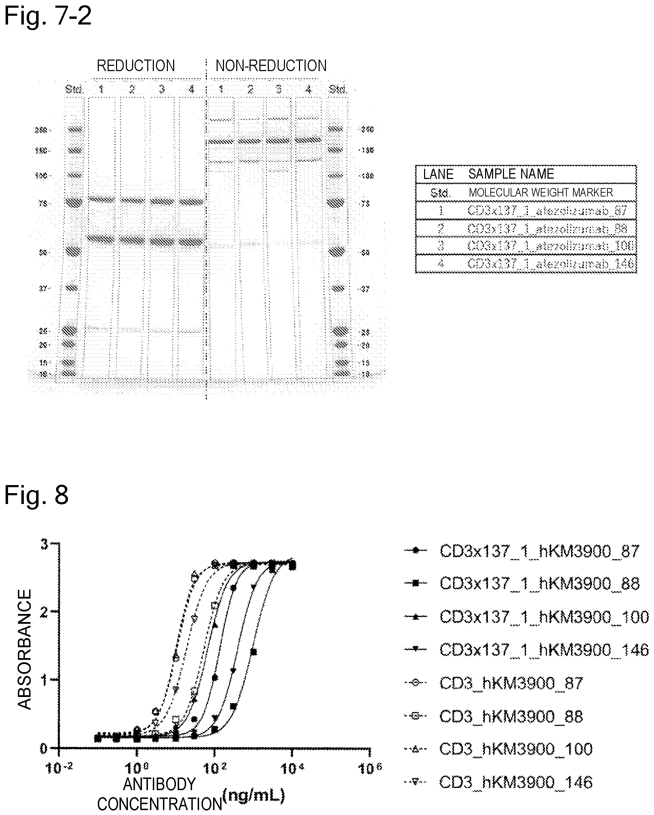

[Fig. 7-2] Fig. 7-2 shows results of SDS-PAGE analysis of anti-PD-L1/anti-CD3/anti-CD137 trispecific antibody samples treated under reducing and non-reducing conditions.
[Fig. 8] Fig. 8 shows binding activity of test antibodies to CD3 as measured and evaluated by ELISA. The ordinate of the figure represents a value obtained by subtracting the absorbance at 570 nm from the absorbance at 450 nm, and the abscissa represents the concentration of the test antibodies. Symbols indicate the average absorbance at addition of each test antibody.

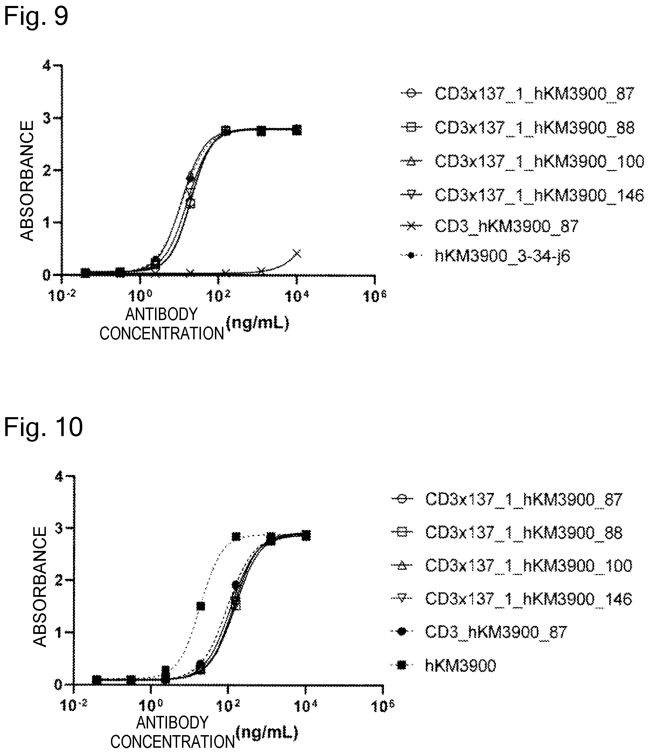

[Fig. 9] Fig. 9 shows binding activity of test antibodies to CD137 as measured and evaluated by ELISA. The ordinate of the figure represents a value obtained by subtracting the absorbance at 570 nm from the absorbance at 450 nm, and the abscissa represents the concentration of the test antibodies. Symbols indicate the average absorbance at addition of each test antibody.
[Fig. 10] Fig. 10 shows binding activity of test antibodies to CLDN4 as measured and evaluated by cell-based ELISA. The ordinate of the figure represents a value obtained by subtracting the absorbance at 570 nm from the absorbance at 450 nm, and the abscissa represents the concentration of the test antibodies. Symbols indicate the average absorbance at addition of each test antibody.

### DESCRIPTION OF EMBODIMENTS

The present invention will be described in detail below.

### <Definitions>

Terms used herein have the meaning commonly used by those skilled in the art in this technical field unless otherwise specified below.

An antibody (or immunoglobulin) refers to a glycoprotein having a four-chain structure of a symmetric Y-shaped structure consisting of two heavy chains having a single sequence and two light chains having a single sequence as a basic structure. An antibody is divided into five classes IgG, IgM, IgA, IgD, and IgE. The basic structure of an antibody molecule is common among the classes, and two heavy chains having a molecular weight of 50,000 to 70,000 and two light chains having a molecular weight of 20,000 to 30,000 are bonded through a disulfide bond or a noncovalent bond to form an antibody molecule having the Y-shaped four chain structure having a molecular weight of 150,000 to 190,000. A heavy chain is composed of a polypeptide chain usually containing about 440 amino acids with a structure characteristic to each class and is designated as Igγ, Igµ, Igα, Igδ, and Igε corresponding to IgG, IgM, IgA, IgD, and IgE, respectively. IgG is further divided into subclasses IgG1, IgG2, IgG3, and IgG4, and heavy chains corresponding to these subclasses are respectively designated as Igγ1, Igγ2, Igγ3, and Igγ4. A light chain is composed of a polypeptide chain usually containing about 220 amino acids and known to be divided into two types, λ light chain and κ light chain, which are respectively designated as Igλ, and Igκ. The two types of light chains can pair with any type of heavy chains.

A heavy chain has four (five in Igµ, and Igε) intrachain disulfide bonds of an antibody molecule, and a light chain has two intrachain disulfide bonds, and one loop is formed with every 100 to 110 amino acid residues. The three-dimensional structure is similar among loops, which is designated as a structural unit or a domain. In both a heavy chain and a light chain, a domain positioned at the N-terminal is designated as a variable region, which is known to have various amino acid sequences even when the antibody is produced from the same class (or subclass) of animals of the same species and to be involved in antibody-antigen binding specificity. An amino acid sequence of a domain on the C-terminal side downstream from the variable region is substantially constant in each class or subclass, and the domain is designated as a constant region. The heavy chain has, from the N-terminal toward the C-terminal, a heavy chain variable region (VH) and a heavy chain constant region (CH). The CH is further divided into three domains of CH1 domain, CH2 domain and CH3 domain disposed in this order from the N-terminal side. The light chain has, from the N-terminal toward the C-terminal, a light chain variable region (VL) and a light chain constant region (CL).

The amino acid sequence of three complementarity determining regions (CDRs) present in each of the VH and VL are highly variable, contributing to variability of the variable regions. The CDRs are regions consisting of about 5 to 10 amino acid residues at the N-terminal of each of the heavy chain and the light chain in the order of CDR1, CDR2 and CDR3 and form an antigen-binding site. On the other hand, a portion excluding the CDRs in the variable region is designated as a frame work region (FR), which consists of FR1 to FR4, with relatively little variation in the amino acid sequence.

Treatment of the antibody with the proteolytic enzyme papain gives three antibody fragments. The two fragments on the N-terminal side are designated as Fab (antigen-binding fragments; fragment, antigen binding) regions. As used herein, the term "Fab region" refers to a region consisting of the VH and the CH1 domain of the heavy chain and the light chain (VL and CL), which binds to an antigen at a tip portion in an antigen-binding site formed by the Fab region. As used herein, the term "heavy chain fragment" refers to a fragment consisting of the VH and the CH1 domain of the heavy chain composing the Fab region.

The fragment on the C-terminal side is designated as an Fc (crystallizable fragment; fragment, crystallizable) region. As used herein, the term "Fc polypeptide" refers to a polypeptide consisting of the CH2 domain and the CH3 domain of the heavy chain, and the term "Fc region" refers to a complex consisting of two Fc polypeptides.

The heavy chain fragment and the Fc polypeptide are linked via a portion designated as a hinge region. Furthermore, the two heavy chains of the antibody are bound together by a disulfide bond in the hinge region.

As used herein, the term "antigen" is used in a generally used sense, particularly as a term for a molecule or a part of a molecule to which an antigen-binding protein such as an antibody or an antigen-binding fragment can specifically bind. The antigen can be a molecule such as proteins and nucleic acids. One antigen may have one or more epitopes capable of interacting with different antibodies and the like.

As used herein, the term "antibody" refers to a polypeptide that specifically binds to an antigen and may have any structure as long as it can specifically bind to an antigen. For example, the antibody comprises, in addition to a polypeptide having a four chain structure as a basic structure such as IgG, polypeptides in various forms, such as an antigen-binding fragment, a multispecific antibody, a bispecific antibody, and a trispecific antibody described below.

As used herein, the term "antigen-binding fragment" refers to a molecule having antigen binding activity derived from an antibody and including at least one polypeptide chain. Representative examples of the antigen-binding fragment include a single-chain variable region fragment (scFv), a Fab fragment, a Fab' fragment, and a F(ab')₂ fragment. The scFv is a monovalent antigen-binding fragment composed of the VH and the VL linked via a linker. The Fab fragment is a monovalent antigen-binding fragment composed of a fragment containing the light chain and the VH and the CH1 domain of the heavy chain. The Fab' fragment is a monovalent antigen-binding fragment composed of a fragment containing the light chain, the VH and the CH1 domain of the heavy chain, and a part of the hinge region, and in this part of the hinge region, a cysteine residue forming a S-S bond between the heavy chains is included. The F(ab')₂ fragment is a bivalent molecule in which Fab' fragments are linked to each other via a disulfide bond. The term "monovalent" means that one antigen binding site is included, and the term "bivalent" means that two antigen binding sites are included. As used herein, the term "scFv region" refers to a region including scFv.

As used herein, the term "polypeptide" means a plurality of amino acid linked to each other with peptide bonds. The amino acids used in the polypeptide may be either natural or artificial amino acids. The number of amino acid residues contained in the polypeptide may be 2 or more, preferably 10 or more.

As used herein, the term "multispecific antibody" refers to an antibody capable of specifically binding to two or more different antigens, which is, for example, referred to as a bispecific antibody or a trispecific antibody depending on the number of antigens to be bound. Multispecific antibodies include a complex of two or more antibodies and/or antigen-binding fragments each capable of binding to a different antigen, and the term "antibodies" used herein comprises multispecific antibodies, unless otherwise specified in the context.

As used herein, the term "bispecific antibody" refers to an antibody capable of specifically binding to two different antigens, and the term "trispecific antibody" refers to an antibody capable of specifically binding to three different antigens.

As used herein, the term "human antibody" refers to an antibody having a human immunoglobulin amino acid sequence. As used herein, the term "humanized antibody" refers to an antibody in which a part of, most of, or all of amino acid residues excluding the CDRs have been replaced with amino acid residues derived from a human immunoglobulin molecule. A humanizing method is not particularly limited, and a humanized antibody can be produced with reference to, for example, U.S. Patent Nos. 5225539 and 6180370.

As used herein, the term "post-translational modification" means that an antibody undergoes a post-translational modification when the antibody is expressed in a cell. Examples of the post-translational modification include modification such as pyroglutamylation, glycosylation, oxidation, deamidation, and glycation of glutamine or glutamic acid at a heavy chain N-terminal, and lysine deletion by cleavage of lysine at a heavy chain C-terminal with carboxypeptidase. It is known that such post-translational modification is caused in various antibodies (J. Pharm. Sci., 2008, Vol. 97, p. 2426-2447).

As used herein, the term "polynucleotide" refers to a polymer of nucleotides and comprises RNA or DNA. The RNA or DNA to be used may be naturally occurring or artificially synthesized.

An amino acid residue number of the antibody to be used herein can be prescribed by specifying Kabat numbering or EU index (Kabat et al., Sequences of Proteins of Immunological Interest, 5th Ed., 1991, NIH Publication No. 91-3242) according to these numbering systems.

As used herein, the term "first" or "second" is used for conveniently distinguishing two or more portions. Use of such a term does not intend to impart a specific order or meaning unless clearly mentioned.

As used herein, the term "link" or "linked" means that a plurality of components (e.g., a Fab region and an Fc polypeptide) are linked to one another directly or via one or more intermediaries (examples of which include, but are not limited to, a peptide linker and a hinge region). As used herein, the term "peptide linker" means one or more arbitrary amino acid residues that can be introduced by genetic engineering for linking variable regions to each other. A length of the peptide linker to be used in the present invention is not particularly limited but can be appropriately selected depending on purpose by those skilled in the art.

As used herein, the term "identity" means a value of identity obtained using EMBOSS Needle (Nucleic Acids Res., 2015, Vol. 43, p. W580-W584) with parameters prepared as default. The parameters are as follows:
Gap Open Penalty = 10
Gap Extend Penalty = 0.5
Matrix = EBLOSUM62
End Gap Penalty = false

As used herein, the term "subject" means a human or other animals in need of prevention or treatment and is, in one aspect, a human in need of treatment or prevention.

Tetraspanin-8 (TSPAN8) is a four-pass transmembrane protein belonging to the tetraspanin family with two extracellular loop regions and three cytoplasmic domains. TSPAN8 is known to be involved in cell adhesion, cell motility, and cell activation and growth, and is expressed at a high level in gastric cancer, pancreatic cancer, colorectal cancer, liver cancer, and other cancer. For example, an association between increased expression of TSPAN8 and progression or metastasis of cancer has been reported (Biomolecules, 2020, Vol. 10, p. 383).

Cluster of differentiation 37 (CD37) is a four-pass transmembrane protein belonging to the tetraspanin family with two extracellular regions and three cytoplasmic regions. Analysis using knockout mice has revealed that CD37 is also involved in immune functions such as antibody production and regulation of T-cell functions (Int. J Mol. Sci., 2020, Vol. 21, p. 9531). Expression of the CD37 in normal tissue is limited to blood cells, particularly at high levels in mature B cells. Furthermore, CD37 is also known to be frequently and highly expressed in blood cancers, such as non-Hodgkin's lymphoma and chronic lymphocytic leukemia, which are derived from mature B cells (Blood Adv., 2019, Vol. 3, p. 1230).

Solute carrier family 34 member 2 (SLC34A2, also known as NaPi2b) is a pH-sensitive sodium-dependent phosphate transporter, which is expressed, for example, in the lung, salivary gland, mammary gland, and small intestine, and involved in the absorption of dietary phosphate. Furthermore, abnormalities in SLC34A2 genes are known to cause alveolar microlithiasis (Pflugers Arch., 2019, Vol. 471, p. 165). SLC34A2, which is highly expressed in lung cancer and ovarian cancer, has attracted attention as a target molecule of therapeutic agents for these cancers (Mol. Cancer Ther., 2021, Vol. 20, p. 896).

Claudin-4 (CLDN4) is a four-pass transmembrane protein belonging to the claudin family, which is expressed in an epithelial cell and an endothelial cell, and plays a significant role as a main molecule composing a tight junction. CLDN4 is highly expressed in cancer tissue of, for example, colorectal cancer, bladder cancer, and ovarian cancer, suggesting that an anti-CLDN4 antibody may be applicable to treatment or diagnosis of cancer (Cancer Science, 2009, Vol. 100, p. 1623-1630).

Programmed death-ligand 1 (PD-L1) is a ligand of programmed cell death-1 (PD-1). PD-1, expression of which is induced in T cells with sustained activation, regulates T cell activation in an inhibitory manner by binding to the ligand PD-L1 or programmed death-ligand 2 (PD-L2) (Annu. Rev. Immunol., 2008, Vol. 26, p. 677-704). In general, such a T cell activation regulatory mechanism is designated as an immune checkpoint and known as one of the negative feedback mechanisms to prevent excessive immune responses.

### <Anti-CD37/Anti-CD3 Bispecific Antibody of Invention>

The present invention provides a bispecific antibody that binds to CD37 and CD3 (also referred to as the "anti-CD37/anti-CD3 bispecific antibody") with structures (a) to (c).

The bispecific antibody that binds to CD37 and CD3 comprises:
(a) a Fab region of an anti-CD37 antibody consisting of a heavy chain fragment comprising a heavy chain variable region of the anti-CD37 antibody and a light chain comprising a light chain variable region of the anti-CD37 antibody;
(b) an anti-CD3 scFV region comprising a heavy chain variable region and a light chain variable region of an anti-CD3 antibody; and
(c) an Fc region consisting of a first Fc polypeptide linked to the heavy chain fragment of the Fab region (a) and a second Fc polypeptide linked to the anti-CD3 scFv region (b).

The anti-CD37/anti-CD3 bispecific antibody of the present invention comprises an anti-CD3 scFV region comprising a heavy chain variable region and a light chain variable region of an anti-CD3 antibody. An anti-CD3 scFv region known in this technical field or an anti-CD3 scFv region produced based on sequence information on the heavy chain variable region and the light chain variable region of an anti-CD3 antibody known in this technical field can be used for the anti-CD37/anti-CD3 bispecific antibody of the present invention. The known anti-CD3 antibody include clones such as OKT3, UTCH1, L2K, and TR66, sequences of which are used as bispecific antibodies (Pharmacol. Ther., 2018, Vol. 182, p. 161-175).

In one embodiment, the heavy chain variable region and the light chain variable region of the anti-CD3 antibody in the anti-CD37/anti-CD3 bispecific antibody of the present invention are any one of (1) to (3):
(1) a heavy chain variable region of an anti-CD3 antibody consisting of an amino acid sequence at amino acid positions 1 to 125 of SEQ ID NO: 14 and a light chain variable region of an anti-CD3 antibody consisting of an amino acid sequence at amino acid positions 146 to 254 of SEQ ID NO: 14;
(2) a heavy chain variable region of an anti-CD3 antibody consisting of an amino acid sequence at amino acid positions 1 to 125 of SEQ ID NO: 16 and a light chain variable region of an anti-CD3 antibody consisting of an amino acid sequence at amino acid positions 146 to 254 of SEQ ID NO: 16; and
(3) a heavy chain variable region of an anti-CD3 antibody consisting of an amino acid sequence at amino acid positions 1 to 125 of SEQ ID NO: 18 and a light chain variable region of an anti-CD3 antibody consisting of an amino acid sequence at amino acid positions 146 to 254 of SEQ ID NO: 18.

In the anti-CD3-scFv region, the type and the length of a peptide linker linking the heavy chain variable region and the light chain variable region of the anti-CD3 antibody are not particularly limited but can be appropriately selected by those skilled in the art. The length is preferably 5 or more amino acids (the upper limit is not particularly limited but is usually 30 or less amino acids, and preferably 20 or less amino acids), particularly preferably 15 amino acids. As the peptide linker, for example, a glycine-serine linker (GS linker) or a glycine-lysine-proline-glycine-serine linker (GKPGS linker) can be used. Examples of such a linker include the following:
Ser
Gly-Ser
Gly-Gly-Ser
Ser-Gly-Gly
Gly-Gly-Gly-Ser (SEQ ID NO: 55)
Ser-Gly-Gly-Gly (SEQ ID NO: 56)
Gly-Gly-Gly-Gly-Ser (SEQ ID NO: 57)
Ser-Gly-Gly-Gly-Gly (SEQ ID NO: 58)
Gly-Gly-Gly-Gly-Gly-Ser (SEQ ID NO: 59)
Ser-Gly-Gly-Gly-Gly-Gly (SEQ ID NO: 60)
Gly-Gly-Gly-Gly-Gly-Gly-Ser (SEQ ID NO: 61)
Ser-Gly-Gly-Gly-Gly-Gly-Gly (SEQ ID NO: 62)
(Gly-Gly-Gly-Gly-Ser)ₙ
(Ser-Gly-Gly-Gly-Gly)ₙ
Gly-Lys-Pro-Gly-Ser (SEQ ID NO: 63)
(Gly-Lys-Pro-Gly-Ser)ₙ

In the above, n represents an integer of 1 or more. The length and the sequence of the peptide linker can be appropriately selected depending on purpose by those skilled in the art.

In one embodiment, the linker used for the anti-CD3 scFv region is a linker having the sequence (Gly-Lys-Pro-Gly-Ser)ₙ. In one embodiment, the linker used for the anti-CD3 scFv region is preferably a linker having the sequence (Gly-Lys-Pro-Gly-Ser)₄.

In one embodiment, the anti-CD3 scFv region is any one of (1) to (3):
(1) an anti-CD3 scFv region consisting of an amino acid sequence at amino acid positions 1 to 254 of SEQ ID NO: 14;
(2) an anti-CD3 scFv region consisting of an amino acid sequence at amino acid positions 1 to 254 of SEQ ID NO: 16; and
(3) an anti-CD3 scFv region consisting of an amino acid sequence at amino acid positions 1 to 254 of SEQ ID NO: 18.

In one embodiment, the heavy chain variable region of the anti-CD37 antibody comprises a CDR1 consisting of an amino acid sequence at amino acid positions 31 to 35 of SEQ ID NO: 10, a CDR2 consisting of an amino acid sequence at amino acid positions 50 to 65 of SEQ ID NO: 10, and a CDR3 consisting of an amino acid sequence at amino acid positions 98 to 104 of SEQ ID NO: 10; and the light chain variable region of the anti-CD37 antibody comprises a CDR1 consisting of an amino acid sequence at amino acid positions 24 to 34 of SEQ ID NO: 12, a CDR2 consisting of an amino acid sequence at amino acid positions 50 to 56 of SEQ ID NO: 12, and a CDR3 consisting of an amino acid sequence at amino acid positions 89 to 95 of SEQ ID NO: 12.

In one embodiment, the heavy chain variable region of the anti-CD37 antibody comprises NO: 10, and the light chain variable region of the anti-CD37 antibody consists of an amino acid sequence at amino acid positions 1 to 105 of SEQ ID NO: 12.

As a heavy chain constant region from which the CH1 domain of the heavy chain fragment of the anti-CD37 antibody is derived, any one of the constant regions Igγ, Igµ Igα, Igδ, and Igε can be selected. The Igγ can be selected from, for example, Igγ1, Igγ2, Igγ3, and Igγ4. In one embodiment, the heavy chain fragment of the anti-CD37 antibody Fab region comprises a CH1 domain derived from human Igγ1 constant region.

As the CL of the light chain of the anti-CD37 antibody Fab region, the constant region of either Igλ or Igκ can be selected. In one embodiment, the light chain of the anti-CD37 antibody Fab region comprises a CL. In one embodiment, the light chain of the anti-CD37 antibody Fab region comprises a human Igκ constant region, CL.

In one embodiment, the anti-CD37 antibody Fab region is composed of a heavy chain fragment consisting of an amino acid sequence at amino acid positions 1 to 213 of SEQ ID NO: 10 and a light chain consisting of an amino acid sequence of SEQ ID NO: 12.

In the present specification, a polypeptide in which a heavy chain fragment including the heavy chain variable region of an ant-CD37 antibody and a first Fc polypeptide are linkedis also referred to as the "heavy chain of an anti CD37 antibody". In the anti-CD37/anti-CD3 bispecific antibody of the present invention, the heavy chain fragment of the heavy chain variable region of the anti-CD37 antibody and the Fc polypeptide (first Fc polypeptide) may be linkeddirectly or via a peptide linker or a hinge region. Alternatively, in the anti-CD37/anti-CD3 bispecific antibody of the present invention, the anti-CD3 scFv region and the Fc polypeptide (second Fc polypeptide) may be linkeddirectly or via a peptide linker or a hinge region.

In one embodiment, the anti-CD37/anti-CD3 bispecific antibody of the present invention is an anti-CD37/anti-CD3 bispecific antibody according to any one of (a) to (c):
(a) a bispecific antibody containing a heavy chain of an anti-CD37 antibody in which a first Fc polypeptide is linked to a heavy chain fragment of the anti-CD37 antibody comprising a heavy chain variable region containing a CDR1 consisting of an amino acid sequence at amino acid positions 31 to 35 of SEQ ID NO: 10, a CDR2 consisting of an amino acid sequence at amino acid positions 50 to 65 of SEQ ID NO: 10, and a CDR3 consisting of an amino acid sequence at amino acid positions 98 to 104 of SEQ ID NO: 10; a light chain of an anti-CD37 antibody including a light chain variable region comprising a CDR1 consisting of an amino acid sequence at amino acid positions 24 to 34 of SEQ ID NO: 12, a CDR2 consisting of an amino acid sequence at amino acid positions 50 to 56 of SEQ ID NO: 12, and a CDR3 consisting of an amino acid sequence at amino acid positions 89 to 95 of SEQ ID NO: 12; and a polypeptide in which a second Fc polypeptide is linked to an anti-CD3 scFv region comprising a heavy chain variable region of an anti-CD3 antibody consisting of an amino acid sequence at amino acid positions 1 to 125 of SEQ ID NO: 14 and a light chain variable region of an anti-CD3 antibody consisting of an amino acid sequence at amino acid positions 146 to 254 of SEQ ID NO: 14;
(b) a bispecific antibody comprising a heavy chain of an anti-CD37 antibody in which a first Fc polypeptide is linked to a heavy chain fragment of the anti-CD37 antibody including a heavy chain variable region containing a CDR1 consisting of an amino acid sequence at amino acid positions 31 to 35 of SEQ ID NO: 10, a CDR2 consisting of an amino acid sequence at amino acid positions 50 to 65 of SEQ ID NO: 10, and a CDR3 consisting of an amino acid sequence at amino acid positions 98 to 104 of SEQ ID NO: 10; a light chain of an anti-CD37 antibody comprising a light chain variable region comprising a CDR1 consisting of an amino acid sequence at amino acid positions 24 to 34 of SEQ ID NO: 12, a CDR2 consisting of an amino acid sequence at amino acid positions 50 to 56 of SEQ ID NO: 12, and a CDR3 consisting of an amino acid sequence at amino acid positions 89 to 95 of SEQ ID NO: 12; and a polypeptide in which a second Fc polypeptide is linked to an anti-CD3 scFv region including a heavy chain variable region of an anti-CD3 antibody consisting of an amino acid sequence at amino acid positions 1 to 125 of SEQ ID NO: 16 and a light chain variable region of an anti-CD3 antibody consisting of an amino acid sequence at amino acid positions 146 to 254 of SEQ ID NO: 16; and
(c) a bispecific antibody comprising a heavy chain of an anti-CD37 antibody in which a first Fc polypeptide is linked to a heavy chain fragment of the anti-CD37 antibody including a heavy chain variable region containing a CDR1 consisting of an amino acid sequence at amino acid positions 31 to 35 of SEQ ID NO: 10, a CDR2 consisting of an amino acid sequence at amino acid positions 50 to 65 of SEQ ID NO: 10, and a CDR3 consisting of an amino acid sequence at amino acid positions 98 to 104 of SEQ ID NO: 10; a light chain of an anti-CD37 antibody comprising a light chain variable region containing a CDR1 consisting of an amino acid sequence at amino acid positions 24 to 34 of SEQ ID NO: 12, a CDR2 consisting of an amino acid sequence at amino acid positions 50 to 56 of SEQ ID NO: 12, and a CDR3 consisting of an amino acid sequence at amino acid positions 89 to 95 of SEQ ID NO: 12; and a polypeptide in which a second Fc polypeptide is linked to an anti-CD3 scFv region including a heavy chain variable region of an anti-CD3 antibody consisting of an amino acid sequence at amino acid positions 1 to 125 of SEQ ID NO: 18 and a light chain variable region of an anti-CD3 antibody consisting of an amino acid sequence at amino acid positions 146 to 254 of SEQ ID NO: 18;

In one embodiment, the anti-CD37/anti-CD3 bispecific antibody of the present invention is an anti-CD37/anti-CD3 bispecific antibody according to any one of (a) to (c):
(a) a bispecific antibody comprising a heavy chain of an anti-CD37 antibody in which a first Fc polypeptide is linked to a heavy chain fragment of the anti-CD37 antibody comprising a heavy chain variable region consisting of an amino acid sequence at amino acid positions 1 to 115 of SEQ ID NO: 10; a light chain of an anti-CD37 antibody comprising a light chain variable region consisting of an amino acid sequence at amino acid positions 1 to 105 of SEQ ID NO: 12; and a polynucleotide in which a second Fc polypeptide is linked to an anti-CD3 scFV region comprising a heavy chain variable region of an anti-CD3 antibody consisting of an amino acid sequence at amino acid positions 1 to 125 of SEQ ID NO: 14 and a light chain variable region of an anti-CD3 antibody consisting of an amino acid sequence at amino acid positions 146 to 254 of SEQ ID NO: 14;
(b) a bispecific antibody comprising a heavy chain of an anti-CD37 antibody in which a first Fc polypeptide is linked to a heavy chain fragment of the anti-CD37 antibody comprising a heavy chain variable region consisting of an amino acid sequence at amino acid positions 1 to 115 of SEQ ID NO: 10; a light chain of an anti-CD37 antibody comprising a light chain variable region consisting of an amino acid sequence at amino acid positions 1 to 105 of SEQ ID NO: 12; and a polynucleotide in which a second Fc polypeptide is linked to an anti-CD3 scFV region comprising a heavy chain variable region of an anti-CD3 antibody consisting of an amino acid sequence at amino acid positions 1 to 125 of SEQ ID NO: 16 and a light chain variable region of an anti-CD3 antibody consisting of an amino acid sequence at amino acid positions 146 to 254 of SEQ ID NO: 16; and
(c) a bispecific antibody comprising a heavy chain of an anti-CD37 antibody in which a first Fc polypeptide is linked to a heavy chain fragment of the anti-CD37 antibody comprising a heavy chain variable region consisting of an amino acid sequence at amino acid positions 1 to 115 of SEQ ID NO: 10; a light chain of an anti-CD37 antibody comprising a light chain variable region consisting of an amino acid sequence at amino acid positions 1 to 105 of SEQ ID NO: 12; and a polynucleotide in which a second Fc polypeptide is linked to an anti-CD3 scFV region comprising a heavy chain variable region of an anti-CD3 antibody consisting of an amino acid sequence at amino acid positions 1 to 125 of SEQ ID NO: 18 and a light chain variable region of an anti-CD3 antibody consisting of an amino acid sequence at amino acid positions 146 to 254 of SEQ ID NO: 18.

In one embodiment, the anti-CD37/anti-CD3 bispecific antibody of the present invention is an anti-CD37/anti-CD3 bispecific antibody according to any one of (a) to (c):
(a) a bispecific antibody comprising a heavy chain of an anti-CD37 antibody in which a first Fc polypeptide is linked to a heavy chain fragment consisting of an amino acid sequence at amino acid positions 1 to 213 of SEQ ID NO: 10; a light chain consisting of an amino acid sequence of SEQ ID NO: 12; and a polypeptide in which a second Fc polypeptide is linked to an anti-CD3 scFv region consisting of amino acid sequence at amino acid positions 1 to 254 of SEQ ID NO: 14;
(b) a bispecific antibody comprising a heavy chain of an anti-CD37 antibody in which a first Fc polypeptide is linked to a heavy chain fragment consisting of an amino acid sequence at amino acid positions 1 to 213 of SEQ ID NO: 10; a light chain consisting of an amino acid sequence of SEQ ID NO: 12; and a polypeptide in which a second Fc polypeptide is linked to an anti-CD3 scFv region consisting of amino acid sequence at amino acid positions 1 to 254 of SEQ ID NO: 16; and
(c) a bispecific antibody comprising a heavy chain of an anti-CD37 antibody in which a first Fc polypeptide is linked to a heavy chain fragment consisting of an amino acid sequence at amino acid positions 1 to 213 of SEQ ID NO: 10; a light chain consisting of an amino acid sequence of SEQ ID NO: 12; and a polypeptide in which a second Fc polypeptide is linked to an anti-CD3 scFv region consisting of amino acid sequence at amino acid positions 1 to 254 of SEQ ID NO: 18.

In the anti-CD37/anti-CD3 bispecific antibody of the present invention, any one of the constant regions Igγ, Igµ Igα, Igδ, and Igε can be selected as a heavy chain constant region from which the first Fc polypeptide and the second Fc polypeptide composing the Fc region are derived. The Igγ can be selected from, for example, Igγ1, Igγ2, Igγ3, and Igγ4. In one embodiment, the first Fc polypeptide and the second Fc polypeptide are Fc polypeptides derived from the human Igγ1 constant region.

The Fc region of the anti-CD37/anti-CD3 bispecific antibody of the present invention may contain mutation that reduces antibody-dependent cytotoxicity (ADCC) or complement-dependent cytotoxicity (CDC). L234A is a replacement of leucine with alanine at amino acid position 234 in human Igyl constant region according to EU index. L235A is a replacement of leucine with alanine at amino acid position 235 in human Igyl constant region according to EU index. The amino acid mutation of L234A and L235A of human Igyl constant region is designated as "LALA mutation". This mutation is known to reduce antibody-dependent cytotoxicity and complement-dependent cytotoxicity of an antibody (Mol. Immunol., 1992, Vol. 29, p. 633-639).

The Fc region in the anti-CD37/anti-CD3 bispecific antibody of the present invention may further contain mutation based on another known technique. For example, the Fc region may contain P329A mutation (J Biol Chem., 2001, Vol. 276, Issue 9, p. 6591-6604) or mutation based on knobs-into-holes technique (hereinafter also referred to as "knobs-into-holes mutation"). The P329A mutation has the effect of keeping ADCC and CDC activities low by attenuating the binding to FcyR and C1q. According to the knobs-into-holes technique, an amino acid side chain present in the CH3 region of one heavy chain is replaced with a larger side chain (knob), and an amino acid side chain present in the CH3 region of another heavy chain is replaced with a smaller side chain (hole), and thus, heterodimerization of the heavy chains is accelerated with the knob disposed within the hole, and in this manner, a heterodimerized antibody molecule of interest can be efficiently obtained (Nature, 1994, Vol. 372, p. 379-383).

In one embodiment, the anti-CD37/anti-CD3 bispecific antibody of the present invention include an Fc region comprising LALA mutation. In one embodiment, the anti-CD37/anti-CD3 bispecific antibody of the present invention include an Fc region comprising P329A mutation. In one embodiment, the anti-TAA/anti-CD3 bispecific antibody of the present invention include an Fc region comprising knobs-into-holes mutation. In one embodiment, the anti-CD37/anti-CD3 bispecific antibody of the present invention include an Fc region comprising one or more mutations of LALA mutation, P329A mutation, and knobs-into-holes mutation. In one embodiment, the anti-CD37/anti-CD3 bispecific antibody of the present invention include an Fc region comprising LALA mutation and knobs-into-holes mutation. In one embodiment, the knobs-into-holes mutation contained in the anti-CD37/anti-CD3 bispecific antibody of the present invention comprises T366W mutation in one Fc polypeptide forming the Fc region, and T366S, L368A, and Y407V mutations in another Fc polypeptide forming the Fc region (see, International Publication No. WO 1998/050431).

In one embodiment, the anti-CD37/anti-CD3 bispecific antibody of the present invention comprises a first Fc polypeptide consisting of an amino acid sequence at amino acid positions 229 to 445 of SEQ ID NO: 10. In one embodiment, the anti-CD37/anti-CD3 bispecific antibody of the present invention comprises a second Fc polypeptide consisting of an amino acid sequence at amino acid positions 270 to 486 of SEQ ID NO: 14, an amino acid sequence at amino acid positions 270 to 486 of SEQ ID NO: 16, or an amino acid sequence at amino acid positions 270 to 486 of SEQ ID NO: 18.

In one embodiment, the anti-CD37/anti-CD3 bispecific antibody of the present invention comprises a polynucleotide described in any one of (a) to (c):
(a) an Fc region consisting of a first Fc polypeptide consisting of an amino acid sequence at amino acid positions 229 to 445 of SEQ ID NO: 10 and a second Fc polypeptide consisting of an amino acid sequence at amino acid positions 270 to 486 of SEQ ID NO: 14;
(b) an Fc region consisting of a first Fc polypeptide consisting of an amino acid sequence at amino acid positions 229 to 445 of SEQ ID NO: 10 and a second Fc polypeptide consisting of an amino acid sequence at amino acid positions 270 to 486 of SEQ ID NO: 16; and
(c) an Fc region consisting of a first Fc polypeptide consisting of an amino acid sequence at amino acid positions 229 to 445 of SEQ ID NO: 10 and a second Fc polypeptide consisting of an amino acid sequence at amino acid positions 270 to 486 of SEQ ID NO: 18.

In the anti-CD37/anti-CD3 bispecific antibody of the present invention, the heavy chain fragment comprising the heavy chain variable region of the anti-CD37 antibody and the first Fc polypeptide may be linked via a hinge region to form a heavy chain of the anti-CD37 antibody.

Alternatively, in the anti-CD37/anti-CD3 bispecific antibody of the present invention, the anti-CD3 scFv region and the second Fc polypeptide may be linked via a hinge region.

In one embodiment, the anti-CD37/anti-CD3 bispecific antibody of the present invention comprises a heavy chain of an anti-CD37 antibody in which the heavy chain fragment of the Fab region of the anti-CD37 antibody and the first Fc polypeptide is linked via a hinge region. In one embodiment, the anti-CD37/anti-CD3 bispecific antibody of the present invention comprises a polypeptide in which the anti-CD3 scFv region and the second Fc polypeptide are linked via a hinge region. In one embodiment, the anti-CD37/anti-CD3 bispecific antibody of the present invention comprises a heavy chain of an anti-CD37 antibody in which a heavy chain fragment of the Fab region of the anti-CD37 antibody and the first Fc polypeptide are linked via a hinge region; and a polypeptide in which the anti-CD3 scFv region and the second Fc polypeptide are linked via a hinge region.

In one embodiment, the anti-CD37/anti-CD3 bispecific antibody of the present invention comprises a heavy chain in which the heavy chain fragment of the Fab region of the anti-CD37 antibody and the first Fc polypeptide are linked via a hinge region; a light chain of the anti-CD37 scFv region; and a polypeptide in which the anti-CD3 scFv region and the second Fc polypeptide are linked via a hinge region.

In the case of comprising a hinge region, the anti-CD37/anti-CD3 bispecific antibody of the present invention may contain a mutation in the hinge region based on a known technique. For example, the hinge region may contain C220S mutation (Methods, 2019, Vol. 154, p. 38-50). The C220S mutation aims to remove the cysteine at position 220, which has become free as a result of loss of the paired cysteine due to loss of the cysteine present in the light chain constant region. When the anti-CD37/anti-CD3 bispecific antibody of the present invention comprises a hinge region, in one embodiment, the hinge region linking the anti-CD3 scFv region to the second Fc polypeptide.

In one embodiment of the anti-CD37/anti-CD3 bispecific antibody of the present invention, the Fc region comprises LALA mutation, P329A mutation, and knobs-into-holes mutation, and the hinge region linking the anti-CD3 scFv region to the second Fc polypeptide comprises C220S mutation.

In one embodiment, the anti-CD37/anti-CD3 bispecific antibody of the present invention comprises a polypeptide comprising a hinge region consisting of the amino acid sequence at amino acid positions 214 to 445 of SEQ ID NO: 10 and the first Fc polypeptide. In one embodiment, the anti-CD37/anti-CD3 bispecific antibody of the present invention comprises a polypeptide comprising a hinge region consisting of an amino acid sequence at amino acid positions 255 to 486 of SEQ ID NO: 14, an amino acid sequence at amino acid positions 255 to 486 of SEQ ID NO: 16, or an amino acid sequence at amino acid positions 255 to 486 of SEQ ID NO: 18; and the second polypeptide.

In one embodiment, the anti-CD37/anti-CD3 bispecific antibody of the present invention comprises polypeptides described in any one of (a) to (c):
(a) a polypeptide comprising a hinge region consisting of an amino acid sequence at amino acid positions 214 to 445 of SEQ ID NO: 10 and the first Fc polypeptide; and a polypeptide comprising a hinge region consisting of an amino acid sequence at amino acid positions 255 to 486 of SEQ ID NO: 14 and the second Fc polypeptide;
(b) a polypeptide consisting of a hinge region consisting of an amino acid sequence at amino acid positions 214 to 445 of SEQ ID NO: 10 and the first Fc polypeptide; and a polypeptide comprising a hinge region consisting of an amino acid sequence at amino acid positions 255 to 486 of SEQ ID NO: 16 and the second Fc polypeptide; and
(c) a polypeptide consisting of a hinge region consisting of an amino acid sequence at amino acid positions 214 to 445 of SEQ ID NO: 10 and the first Fc polypeptide; and a polypeptide comprising a hinge region consisting of an amino acid sequence at amino acid positions 255 to 486 of SEQ ID NO: 18 and the second Fc polypeptide.

In the present specification, the amino acid mutations such as LALA mutation, P329A mutation, knobs-into-holes mutation, and C220S mutation are described based on amino acid positions in human Igyl constant region according to EU index. For example, as described above, L234A is a replacement of leucine with alanine at amino acid position 234 in human Igyl constant region according to EU index.

In one embodiment, the anti-CD37/anti-CD3 bispecific antibody of the present invention is any one of (a) to (c):
(a) a bispecific antibody comprising a heavy chain of an anti-CD37 antibody consisting of an amino acid sequence of SEQ ID NO: 10; a light chain of an anti-CD3 antibody consisting of an amino acid sequence of SEQ ID NO: 12; and a polypeptide consisting of an amino acid sequence of SEQ ID NO: 14 in which the anti-CD3 scFv region and the second Fc polypeptide are linked;
(b) a bispecific antibody comprising a heavy chain of an anti-CD37 antibody consisting of an amino acid sequence of SEQ ID NO: 10; a light chain of an anti-CD3 antibody consisting of an amino acid sequence of SEQ ID NO: 12; and a polypeptide consisting of an amino acid sequence of SEQ ID NO: 16 in which the anti-CD3 scFv region and the second Fc polypeptide are linked; and
(c) a bispecific antibody comprising a heavy chain of an anti-CD37 antibody consisting of an amino acid sequence of SEQ ID NO: 10; a light chain of an anti-CD3 antibody consisting of an amino acid sequence of SEQ ID NO: 12; and a polypeptide consisting of an amino acid sequence of SEQ ID NO: 18 in which the anti-CD3 scFv region and the second Fc polypeptide are linked.

In one embodiment, the anti-CD37/anti-CD3 bispecific antibody of the present invention is a bispecific antibody comprising a heavy chain of an anti-CD37 antibody consisting of an amino acid sequence of SEQ ID NO: 10; a light chain of an anti-CD37 antibody consisting of an amino acid sequence of SEQ ID NO: 12; and a polypeptide consisting of an amino acid sequence of SEQ ID NO: 14 in which the anti-CD3 scFv region and the second Fc polypeptide are linked.

In one embodiment, the anti-CD37/anti-CD3 bispecific antibody of the present invention is a bispecific antibody comprising a heavy chain of an anti-CD37 antibody consisting of an amino acid sequence of SEQ ID NO: 10; a light chain of an anti-CD37 antibody consisting of an amino acid sequence of SEQ ID NO: 12; and a polypeptide consisting of an amino acid sequence of SEQ ID NO: 16 in which the anti-CD3 scFv region and the second Fc polypeptide are linked.

In one embodiment, the anti-CD37/anti-CD3 bispecific antibody of the present invention is a bispecific antibody comprising a heavy chain of an anti-CD37 antibody consisting of an amino acid sequence of SEQ ID NO: 10; a light chain of an anti-CD37 antibody consisting of an amino acid sequence of SEQ ID NO: 12; and a polypeptide consisting of an amino acid sequence of SEQ ID NO: 18 in which the anti-CD3 scFv region and the second Fc polypeptide are linked.

In one embodiment, the anti-CD37/anti-CD3 bispecific antibody of the present `invention may be post-translationally modified. In one embodiment, the post-translational modification is pyroglutamylation at the N-terminal of the heavy chain variable region and/or lysine deletion at the heavy chain C-terminal. It is known in the art that the post-translational modification by pyroglutamylation at the N-terminal or lysine deletion at the C-terminal does not affect the activity of the antibody (Analytical Biochemistry, 2006, Vol. 348, p. 24-39).

The anti-CD37/anti-CD3 bispecific antibody of the present invention binds to human CD37 (GenBank Accession No. NM_001774.3) and human CD3εδ complex protein (CD3ε: GenBank Accession No. NM_000733.3 and CD3δ: GenBank Accession No. NM_000732.4 or NM_001040651.1). It is possible to confirm whether the antibody binds to human CD37 and human CD3εδ complex protein using a known binding activity measurement method. Examples of a method of measuring binding activity include Enzyme-Linked ImmunoSorbent Assay (ELISA) and flow cytometry. In the case of using ELISA, for example, a method described in Example 1-4 can be employed.

The anti-CD37/anti-CD3 bispecific antibody of the present invention can be produced by those skilled in the art by a method known in the art based on sequence information and the like on the anti-CD37 antibody and the heavy chain variable region and the light chain variable region of the anti-CD3 scFv region disclosed herein. Furthermore, the anti-CD3 scFv region of the anti-CD37/anti-CD3 bispecific antibody of the present invention can be produced by those skilled in the art by a method known in the art based on sequence information and the like on the heavy chain variable region and the light chain variable region of a known anti-CD3 antibody.

In one embodiment, the anti-CD37/anti-CD3 bispecific antibody of the present invention is a humanized antibody or a human antibody. In production of a humanized antibody, a back mutation may be appropriately introduced by employing a method well known to those skilled in the art (Bioinformatics, 2015, Vol. 31, p. 434-435). The anti-CD37/anti-CD3 bispecific antibody of the present invention can be produced by, but not particularly limited to, a method described later in the section of <Method for Producing Bispecific Antibody of Invention and Bispecific Antibody of Invention Produced by the Method>, for example.

### <Polynucleotide of Anti-CD37/Anti-CD3 Bispecific Antibody of Invention>

The present invention also provides a polynucleotide for use in the production of the anti-CD37/anti-CD3 bispecific antibody of the present invention (also referred to as the "polynucleotide of the anti-CD37/anti-CD3 bispecific antibody of the present invention").

In one embodiment, the polynucleotide of the anti-CD37/anti-CD3 bispecific antibody of the present invention is selected from the group consisting of (a) to (j):
(a) a polynucleotide having a nucleotide sequence encoding a heavy chain fragment of an anti-CD37 antibody comprising a heavy chain variable region consisting of an amino acid sequence at amino acid positions 1 to 115 of SEQ ID NO: 10;
(b) a polynucleotide having a nucleotide sequence encoding a light chain of an anti-CD37 antibody comprising a light chain variable region consisting of an amino acid sequence at amino acid positions 1 to 105 of SEQ ID NO: 12;
(c) a polynucleotide having a nucleotide sequence encoding an anti-CD3 scFv region consisting of an amino acid sequence at amino acid positions 1 to 254 of SEQ ID NO: 14;
(d) a polynucleotide having a nucleotide sequence encoding an anti-CD3 scFv region consisting of an amino acid sequence at amino acid positions 1 to 254 of SEQ ID NO: 16;
(e) a polynucleotide having a nucleotide sequence encoding an anti-CD3 scFv region consisting of an amino acid sequence at amino acid positions 1 to 254 of SEQ ID NO: 18;
(f) a polynucleotide having a nucleotide sequence encoding a heavy chain of an anti-CD37 antibody consisting of an amino acid sequence of SEQ ID NO: 10;
(g) a polynucleotide having a nucleotide sequence encoding a light chain of an anti-CD37 antibody consisting of an amino acid sequence of SEQ ID NO: 12;
(h) a polynucleotide comprising a nucleotide sequence encoding a polypeptide consisting of an amino acid sequence of SEQ ID NO: 14 in which the anti-CD3 scFv region and the second Fc polypeptide are linked;
(i) a polynucleotide having a nucleotide sequence encoding a polypeptide consisting of an amino acid sequence of SEQ ID NO: 16 in which the anti-CD3 scFv region and the second Fc polypeptide are linked; and
(j) a polynucleotide having a nucleotide sequence encoding a polypeptide consisting of an amino acid sequence of SEQ ID NO: 18 in which the anti-CD3 scFv region and the second Fc polypeptide are linked.

In one embodiment, the polynucleotide of the anti-CD37/anti-CD3 bispecific antibody of the present invention, which encodes the Fab region of an anti-CD37 antibody, comprises a polynucleotide having a nucleotide sequence encoding a heavy chain fragment of an anti-CD37 antibody comprising a heavy chain variable region consisting of an amino acid sequence at amino acid positions 1 to 115 of SEQ ID NO: 10; and a polynucleotide having a nucleotide sequence encoding a light chain of an anti-CD37 antibody comprising a light chain variable region consisting of an amino acid sequence at amino acid positions 1 to 105 of SEQ ID NO: 12.

In an embodiment, the polynucleotide of the anti-CD37/anti-CD3 bispecific antibody of the present invention is selected from the group consisting of (k) to (p):
(k) a polynucleotide having a nucleotide sequence encoding an anti-CD3 scFV region comprising a heavy chain variable region of an anti-CD3 antibody consisting of an amino acid sequence at amino acid positions 1 to 125 of SEQ ID NO: 14 and a light chain variable region of an anti-CD3 antibody consisting of an amino acid sequence at amino acid positions 146 to 254 of SEQ ID NO: 14;
(l) a polynucleotide having a nucleotide sequence encoding an anti-CD3 scFV region comprising a heavy chain variable region of an anti-CD3 antibody consisting of an amino acid sequence at amino acid positions 1 to 125 of SEQ ID NO: 16 and a light chain variable region of an anti-CD3 antibody consisting of an amino acid sequence at amino acid positions 146 to 254 of SEQ ID NO: 16;
(m) a polynucleotide having a nucleotide sequence encoding an anti-CD3 scFV region comprising a heavy chain variable region of an anti-CD3 antibody consisting of an amino acid sequence at amino acid positions 1 to 125 of SEQ ID NO: 18 and a light chain variable region of an anti-CD3 antibody consisting of an amino acid sequence at amino acid positions 146 to 254 of SEQ ID NO: 18;
(n) a polynucleotide having a nucleotide sequence encoding a polypeptide in which a second Fc polypeptide is linked to an anti-CD3 scFV region comprising a heavy chain variable region of an anti-CD3 antibody consisting of an amino acid sequence at amino acid positions 1 to 125 of SEQ ID NO: 14 and a light chain variable region of an anti-CD3 antibody consisting of an amino acid sequence at amino acid positions 146 to 254 of SEQ ID NO: 14;
(o) a polynucleotide having a nucleotide sequence encoding a polypeptide in which a second Fc polypeptide is linked to an anti-CD3 scFV region comprising a heavy chain variable region of an anti-CD3 antibody consisting of an amino acid sequence at amino acid positions 1 to 125 of SEQ ID NO: 16 and a light chain variable region of an anti-CD3 antibody consisting of an amino acid sequence at amino acid positions 146 to 254 of SEQ ID NO: 16; and
(p) a polynucleotide having a nucleotide sequence encoding a polypeptide in which a second Fc polypeptide is linked to an anti-CD3 scFV region comprising a heavy chain variable region of an anti-CD3 antibody consisting of an amino acid sequence at amino acid positions 1 to 125 of SEQ ID NO: 18 and a light chain variable region of an anti-CD3 antibody consisting of an amino acid sequence at amino acid positions 146 to 254 of SEQ ID NO: 18.

In one embodiment, polynucleotides of the anti-CD37/anti-CD3 bispecific antibody of the present invention are selected from the group consisting of (a) to (c):
(a) a polynucleotide having a nucleotide sequence encoding a heavy chain fragment of an anti-CD37 antibody comprising a heavy chain variable region consisting of an amino acid sequence at amino acid positions 1 to 115 of SEQ ID NO: 10, a polynucleotide having a nucleotide sequence encoding a light chain of an anti-CD37 antibody comprising a light chain variable region consisting of an amino acid sequence at amino acid positions 1 to 105 of SEQ ID NO: 12, and a polynucleotide having a nucleotide sequence encoding an anti-CD3 scFV region comprising a heavy chain variable region of an anti-CD3 antibody consisting of an amino acid sequence at amino acid positions 1 to 125 of SEQ ID NO: 14 and a light chain variable region of an anti-CD3 antibody consisting of an amino acid sequence at amino acid positions 146 to 254 of SEQ ID NO: 14;
(b) a polynucleotide having a nucleotide sequence encoding a heavy chain fragment of an anti-CD37 antibody comprising a heavy chain variable region consisting of an amino acid sequence at amino acid positions 1 to 115 of SEQ ID NO: 10, a polynucleotide having a nucleotide sequence encoding a light chain of an anti-CD37 antibody comprising a light chain variable region consisting of an amino acid sequence at amino acid positions 1 to 105 of SEQ ID NO: 12, and a polynucleotide having a nucleotide sequence encoding an anti-CD3 scFV region comprising a heavy chain variable region of an anti-CD3 antibody consisting of an amino acid sequence at amino acid positions 1 to 125 of SEQ ID NO: 16 and a light chain variable region of an anti-CD3 antibody consisting of an amino acid sequence at amino acid positions 146 to 254 of SEQ ID NO: 16; and,
(c) a polynucleotide having a nucleotide sequence encoding a heavy chain fragment of an anti-CD37 antibody comprising a heavy chain variable region consisting of an amino acid sequence at amino acid positions 1 to 115 of SEQ ID NO: 10, a polynucleotide having a nucleotide sequence encoding a light chain of an anti-CD37 antibody comprising a light chain variable region consisting of an amino acid sequence at amino acid positions 1 to 105 of SEQ ID NO: 12, and a polynucleotide having a nucleotide sequence encoding an anti-CD3 scFV region comprising a heavy chain variable region of an anti-CD3 antibody consisting of an amino acid sequence at amino acid positions 1 to 125 of SEQ ID NO: 18 and a light chain variable region of an anti-CD3 antibody consisting of an amino acid sequence at amino acid positions 146 to 254 of SEQ ID NO: 18.

In one embodiment, polynucleotides of the anti-CD37/anti-CD3 bispecific antibody of the present invention are selected from the group consisting of (a) to (c):
(a) a polynucleotide having a nucleotide sequence encoding a heavy chain of an anti-CD37 antibody in which a first Fc polypeptide is linked to a heavy chain fragment of an anti-CD37 antibody comprising a heavy chain variable region consisting of an amino acid sequence at amino acid positions 1 to 115 of SEQ ID NO: 10, a polynucleotide having a nucleotide sequence encoding a light chain of an anti-CD37 antibody comprising a light chain variable region consisting of an amino acid sequence at amino acid positions 1 to 105 of SEQ ID NO: 12, and a polynucleotide having a nucleotide sequence encoding a polypeptide in which a second Fc polypeptide is linked to an anti-CD3 scFV region comprising a heavy chain variable region of an anti-CD3 antibody consisting of an amino acid sequence at amino acid positions 1 to 125 of SEQ ID NO: 14 and a light chain variable region of an anti-CD3 antibody consisting of an amino acid sequence at amino acid positions 146 to 254 of SEQ ID NO: 14;
(b) a polynucleotide having a nucleotide sequence encoding a heavy chain of an anti-CD37 antibody in which a first Fc polypeptide is linked to a heavy chain fragment of an anti-CD37 antibody comprising a heavy chain variable region consisting of an amino acid sequence at amino acid positions 1 to 115 of SEQ ID NO: 10, a polynucleotide having a nucleotide sequence encoding a light chain of an anti-CD37 antibody comprising a light chain variable region consisting of an amino acid sequence at amino acid positions 1 to 105 of SEQ ID NO: 12, and a polynucleotide having a nucleotide sequence encoding a polypeptide in which a second Fc polypeptide is linked to an anti-CD3 scFV region comprising a heavy chain variable region of an anti-CD3 antibody consisting of an amino acid sequence at amino acid positions 1 to 125 of SEQ ID NO: 16 and a light chain variable region of an anti-CD3 antibody consisting of an amino acid sequence at amino acid positions 146 to 254 of SEQ ID NO: 16; and
(c) a polynucleotide having a nucleotide sequence encoding a heavy chain of an anti-CD37 antibody in which a first Fc polypeptide is linked to a heavy chain fragment of an anti-CD37 antibody comprising a heavy chain variable region consisting of an amino acid sequence at amino acid positions 1 to 115 of SEQ ID NO: 10, a polynucleotide having a nucleotide sequence encoding a light chain of an anti-CD37 antibody comprising a light chain variable region consisting of an amino acid sequence at amino acid positions 1 to 105 of SEQ ID NO: 12, and a polynucleotide having a nucleotide sequence encoding a polypeptide in which a second Fc polypeptide is linked to an anti-CD3 scFV region comprising a heavy chain variable region of an anti-CD3 antibody consisting of an amino acid sequence at amino acid positions 1 to 125 of SEQ ID NO: 18 and a light chain variable region of an anti-CD3 antibody consisting of an amino acid sequence at amino acid positions 146 to 254 of SEQ ID NO: 18.

In one embodiment, polynucleotides of the anti-CD37/anti-CD3 bispecific antibody of the present invention are selected from the group consisting of (a) to (c):
(a) a polynucleotide having a nucleotide sequence encoding a heavy chain of an anti-CD37 antibody consisting of an amino acid sequence of SEQ ID NO: 10, a polynucleotide having a nucleotide sequence encoding a light chain of an anti-CD37 antibody consisting of an amino acid sequence of SEQ ID NO: 12, and a polynucleotide comprising a nucleotide sequence encoding a polypeptide consisting of an amino acid sequence of SEQ ID NO: 14 in which the anti-CD3 scFv region and the second Fc polypeptide are linked;
(b) a polynucleotide having a nucleotide sequence encoding a heavy chain of an anti-CD37 antibody consisting of an amino acid sequence of SEQ ID NO: 10, a polynucleotide having a nucleotide sequence encoding a light chain of an anti-CD37 antibody consisting of an amino acid sequence of SEQ ID NO: 12, and a polynucleotide comprising a nucleotide sequence encoding a polypeptide consisting of an amino acid sequence of SEQ ID NO: 16 in which the anti-CD3 scFv region and the second Fc polypeptide are linked; and
(c) a polynucleotide having a nucleotide sequence encoding a heavy chain of an anti-CD37 antibody consisting of an amino acid sequence of SEQ ID NO: 10, a polynucleotide having a nucleotide sequence encoding a light chain of an anti-CD37 antibody consisting of an amino acid sequence of SEQ ID NO: 12, and a polynucleotide comprising a nucleotide sequence encoding a polypeptide consisting of an amino acid sequence of SEQ ID NO: 18 in which the anti-CD3 scFv region and the second Fc polypeptide are linked.

The polynucleotide of the anti-CD37/anti-CD3 bispecific antibody of the present invention can be produced by those skilled in the art based on the nucleotide sequence thereof by employing a method known in this technical field. For example, the polynucleotide of the anti-CD37/anti-CD3 bispecific antibody of the present invention can be synthesized by employing a gene synthesis method known in this technical field. As the gene synthesis method, any of various methods known to those skilled in the art such as a synthesis method for an antibody gene described in International Publication No. WO 90/07861 can be employed.

### <Expression Vector for Anti-CD37/Anti-CD3 Bispecific Antibody of Invention>

The present invention also provides an expression vector comprising polynucleotides of the anti-CD37/anti-CD3 bispecific antibody of the present invention (also referred to as the "expression vector for the anti-CD37/anti-CD3 bispecific antibody of the present invention") described in (a) to (j). These polynucleotides may be each contained in different vectors, or a plurality of the polynucleotides may be contained in one vector:
(a) a polynucleotide having a nucleotide sequence encoding a heavy chain fragment of an anti-CD37 antibody comprising a heavy chain variable region consisting of an amino acid sequence at amino acid positions 1 to 115 of SEQ ID NO: 10;
(b) a polynucleotide having a nucleotide sequence encoding a light chain of an anti-CD37 antibody comprising a light chain variable region consisting of an amino acid sequence at amino acid positions 1 to 105 of SEQ ID NO: 12;
(c) a polynucleotide having a nucleotide sequence encoding an anti-CD3 scFv region consisting of an amino acid sequence at amino acid positions 1 to 254 of SEQ ID NO: 14;
(d) a polynucleotide having a nucleotide sequence encoding an anti-CD3 scFv region consisting of an amino acid sequence at amino acid positions 1 to 254 of SEQ ID NO: 16;
(e) a polynucleotide having a nucleotide sequence encoding an anti-CD3 scFv region consisting of an amino acid sequence at amino acid positions 1 to 254 of SEQ ID NO: 18;
(f) a polynucleotide having a nucleotide sequence encoding a heavy chain of an anti-CD37 antibody consisting of an amino acid sequence of SEQ ID NO: 10;
(g) a polynucleotide having a nucleotide sequence encoding a light chain of an anti-CD37 antibody consisting of an amino acid sequence of SEQ ID NO: 12;
(h) a polynucleotide having a nucleotide sequence encoding a polypeptide consisting of an amino acid sequence of SEQ ID NO: 14 in which the anti-CD3 scFv region and the second Fc polypeptide are linked;
(i) a polynucleotide having a nucleotide sequence encoding a polypeptide consisting of an amino acid sequence of SEQ ID NO: 16 in which the anti-CD3 scFv region and the second Fc polypeptide are linked; and
(j) a polynucleotide having a nucleotide sequence encoding a polypeptide consisting of an amino acid sequence of SEQ ID NO: 18 in which the anti-CD3 scFv region and the second Fc polypeptide are linked.

In one embodiment, the expression vector for the anti-CD37/anti-CD3 bispecific antibody of the present invention comprises a polynucleotide having a nucleotide sequence encoding a heavy chain fragment of an anti-CD37 antibody comprising a heavy chain variable region consisting of amino acid sequence at amino acid positions 1 to 115 of SEQ ID NO: 10 and a polynucleotide having a nucleotide sequence encoding a light chain of an anti-CD37 antibody comprising a light chain variable region consisting of an amino acid sequence at amino acid positions 1 to 105 of SEQ ID NO: 12.

In one embodiment, the expression vector for the anti-CD37/anti-CD3 bispecific antibody of the present invention comprises a polynucleotide described in any one of (k) to (p):
(k) a polynucleotide having a nucleotide sequence encoding an anti-CD3 scFV region comprising a heavy chain variable region of an anti-CD3 antibody consisting of an amino acid sequence at amino acid positions 1 to 125 of SEQ ID NO: 14 and a light chain variable region of an anti-CD3 antibody consisting of an amino acid sequence at amino acid positions 146 to 254 of SEQ ID NO: 14;
(l) a polynucleotide having a nucleotide sequence encoding an anti-CD3 scFV region comprising a heavy chain variable region of an anti-CD3 antibody consisting of an amino acid sequence at amino acid positions 1 to 125 of SEQ ID NO: 16 and a light chain variable region of an anti-CD3 antibody consisting of an amino acid sequence at amino acid positions 146 to 254 of SEQ ID NO: 16;
(m) a polynucleotide having a nucleotide sequence encoding an anti-CD3 scFV region comprising a heavy chain variable region of an anti-CD3 antibody consisting of an amino acid sequence at amino acid positions 1 to 125 of SEQ ID NO: 18 and a light chain variable region of an anti-CD3 antibody consisting of an amino acid sequence at amino acid positions 146 to 254 of SEQ ID NO: 18;
(n) a polynucleotide having a nucleotide sequence encoding a polypeptide in which a second Fc polypeptide is linked to an anti-CD3 scFV region comprising a heavy chain variable region of an anti-CD3 antibody consisting of an amino acid sequence at amino acid positions 1 to 125 of SEQ ID NO: 14 and a light chain variable region of an anti-CD3 antibody consisting of an amino acid sequence at amino acid positions 146 to 254 of SEQ ID NO: 14;
(o) a polynucleotide having a nucleotide sequence encoding a polypeptide in which a second Fc polypeptide is linked to an anti-CD3 scFV region comprising a heavy chain variable region of an anti-CD3 antibody consisting of an amino acid sequence at amino acid positions 1 to 125 of SEQ ID NO: 16 and a light chain variable region of an anti-CD3 antibody consisting of an amino acid sequence at amino acid positions 146 to 254 of SEQ ID NO: 16; and
(p) a polynucleotide having a nucleotide sequence encoding a polypeptide in which a second Fc polypeptide is linked to an anti-CD3 scFV region comprising a heavy chain variable region of an anti-CD3 antibody consisting of an amino acid sequence at amino acid positions 1 to 125 of SEQ ID NO: 18 and a light chain variable region of an anti-CD3 antibody consisting of an amino acid sequence at amino acid positions 146 to 254 of SEQ ID NO: 18.

In one embodiment, the expression vector for the anti-CD37/anti-CD3 bispecific antibody of the present invention comprises a polynucleotide described in any one of (a) to (c):
(a) a polynucleotide having a nucleotide sequence encoding a heavy chain fragment of an anti-CD37 antibody comprising a heavy chain variable region consisting of an amino acid sequence at amino acid positions 1 to 115 of SEQ ID NO: 10, a polynucleotide having a nucleotide sequence encoding a light chain of an anti-CD37 antibody comprising a light chain variable region consisting of an amino acid sequence at amino acid positions 1 to 105 of SEQ ID NO: 12, and a polynucleotide having a nucleotide sequence encoding an anti-CD3 scFV region comprising a heavy chain variable region of an anti-CD3 antibody consisting of an amino acid sequence at amino acid positions 1 to 125 of SEQ ID NO: 14 and a light chain variable region of an anti-CD3 antibody consisting of an amino acid sequence at amino acid positions 146 to 254 of SEQ ID NO: 14;
(b) a polynucleotide having a nucleotide sequence encoding a heavy chain fragment of an anti-CD37 antibody comprising a heavy chain variable region consisting of an amino acid sequence at amino acid positions 1 to 115 of SEQ ID NO: 10, a polynucleotide having a nucleotide sequence encoding a light chain of an anti-CD37 antibody comprising a light chain variable region consisting of an amino acid sequence at amino acid positions 1 to 105 of SEQ ID NO: 12, and a polynucleotide having a nucleotide sequence encoding an anti-CD3 scFV region comprising a heavy chain variable region of an anti-CD3 antibody consisting of an amino acid sequence at amino acid positions 1 to 125 of SEQ ID NO: 16 and a light chain variable region of an anti-CD3 antibody consisting of an amino acid sequence at amino acid positions 146 to 254 of SEQ ID NO: 16; and
(c) a polynucleotide having a nucleotide sequence encoding a heavy chain fragment of an anti-CD37 antibody comprising a heavy chain variable region consisting of an amino acid sequence at amino acid positions 1 to 115 of SEQ ID NO: 10, a polynucleotide having a nucleotide sequence encoding a light chain of an anti-CD37 antibody comprising a light chain variable region consisting of an amino acid sequence at amino acid positions 1 to 105 of SEQ ID NO: 12, and a polynucleotide having a nucleotide sequence encoding an anti-CD3 scFV region comprising a heavy chain variable region of an anti-CD3 antibody consisting of an amino acid sequence at amino acid positions 1 to 125 of SEQ ID NO: 18 and a light chain variable region of an anti-CD3 antibody consisting of an amino acid sequence at amino acid positions 146 to 254 of SEQ ID NO: 18.

In one embodiment, the expression vector for the anti-CD37/anti-CD3 bispecific antibody of the present invention comprises a polynucleotide described in any one of (a) to (c):
(a) a polynucleotide having a nucleotide sequence encoding a heavy chain of an anti-CD37 antibody in which a first Fc polypeptide is linked to a heavy chain fragment of an anti-CD37 antibody comprising a heavy chain variable region consisting of an amino acid sequence at amino acid positions 1 to 115 of SEQ ID NO: 10, a polynucleotide having a nucleotide sequence encoding a light chain of an anti-CD37 antibody comprising a light chain variable region consisting of an amino acid sequence at amino acid positions 1 to 105 of SEQ ID NO: 12, and a polynucleotide having a nucleotide sequence encoding a polypeptide in which a second Fc polypeptide is linked to an anti-CD3 scFV region comprising a heavy chain variable region of an anti-CD3 antibody consisting of an amino acid sequence at amino acid positions 1 to 125 of SEQ ID NO: 14 and a light chain variable region of an anti-CD3 antibody consisting of an amino acid sequence at amino acid positions 146 to 254 of SEQ ID NO: 14;
(b) a polynucleotide having a nucleotide sequence encoding a heavy chain of an anti-CD37 antibody in which a first Fc polypeptide is linked to a heavy chain fragment of an anti-CD37 antibody comprising a heavy chain variable region consisting of an amino acid sequence at amino acid positions 1 to 115 of SEQ ID NO: 10, a polynucleotide having a nucleotide sequence encoding a light chain of an anti-CD37 antibody comprising a light chain variable region consisting of an amino acid sequence at amino acid positions 1 to 105 of SEQ ID NO: 12, and a polynucleotide having a nucleotide sequence encoding a polypeptide in which a second Fc polypeptide is linked to an anti-CD3 scFV region comprising a heavy chain variable region of an anti-CD3 antibody consisting of an amino acid sequence at amino acid positions 1 to 125 of SEQ ID NO: 16 and a light chain variable region of an anti-CD3 antibody consisting of an amino acid sequence at amino acid positions 146 to 254 of SEQ ID NO: 16; and
(c) a polynucleotide having a nucleotide sequence encoding a heavy chain of an anti-CD37 antibody in which a first Fc polypeptide is linked to a heavy chain fragment of an anti-CD37 antibody comprising a heavy chain variable region consisting of an amino acid sequence at amino acid positions 1 to 115 of SEQ ID NO: 10, a polynucleotide having a nucleotide sequence encoding a light chain of an anti-CD37 antibody comprising a light chain variable region consisting of an amino acid sequence at amino acid positions 1 to 105 of SEQ ID NO: 12, and a polynucleotide having a nucleotide sequence encoding a polypeptide in which a second Fc polypeptide is linked to an anti-CD3 scFV region comprising a heavy chain variable region of an anti-CD3 antibody consisting of an amino acid sequence at amino acid positions 1 to 125 of SEQ ID NO: 18 and a light chain variable region of an anti-CD3 antibody consisting of an amino acid sequence at amino acid positions 146 to 254 of SEQ ID NO: 18.

In one embodiment, the expression vector for the anti-CD37/anti-CD3 bispecific antibody of the present invention comprises a polynucleotide described in any one of (a) to (c):
(a) a polynucleotide having a nucleotide sequence encoding a heavy chain of an anti-CD37 antibody consisting of an amino acid sequence of SEQ ID NO: 10, a polynucleotide having a nucleotide sequence encoding a light chain of an anti-CD37 antibody consisting of an amino acid sequence of SEQ ID NO: 12, and a polynucleotide comprising a nucleotide sequence encoding a polypeptide consisting of an amino acid sequence of SEQ ID NO: 14 in which the anti-CD3 scFv region and the second Fc polypeptide are linked;
(b) a polynucleotide having a nucleotide sequence encoding a heavy chain of an anti-CD37 antibody consisting of an amino acid sequence of SEQ ID NO: 10, a polynucleotide having a nucleotide sequence encoding a light chain of an anti-CD37 antibody consisting of an amino acid sequence of SEQ ID NO: 12, and a polynucleotide comprising a nucleotide sequence encoding a polypeptide consisting of an amino acid sequence of SEQ ID NO: 16 in which the anti-CD3 scFv region and the second Fc polypeptide are linked; and
(c) a polynucleotide having a nucleotide sequence encoding a heavy chain of an anti-CD37 antibody consisting of an amino acid sequence of SEQ ID NO: 10, a polynucleotide having a nucleotide sequence encoding a light chain of an anti-CD37 antibody consisting of an amino acid sequence of SEQ ID NO: 12, and a polynucleotide comprising a nucleotide sequence encoding a polypeptide consisting of an amino acid sequence of SEQ ID NO: 18 in which the anti-CD3 scFv region and the second Fc polypeptide are linked.

The expression vector for the anti-CD37/anti-CD3 bispecific antibody of the present invention is not particularly limited as long as it can produce a polynucleotide in various host cells such as a eukaryotic cell (e.g., an animal cell, an insect cell, a plant cell, or a yeast) and/or a prokaryotic cell (such as *E. coli*). Examples of the expression vector include a plasmid vector and a virus vector. As the plasmid vector, for example, pcDNA series (Thermo Fisher Scientific), pALTER(R)-MAX (Promega Corporation), pHEK293 Ultra Expression Vector (Takara Bio Inc.), or pEE 6.4 or pEE 12.4 (Lonza Biologics) can be used. As the virus vector, for example, a lentivirus, an adenovirus, a retrovirus, or an adeno-associated virus can be used. For example, when a lentivirus is used for introducing the polynucleotide into a cell, pLVSIN-CMV/EF1α vector (Takara Bio Inc.) or pLenti vector (Thermo Fisher Scientific) can be used as the lentivirus. In one embodiment, a vector used in the expression vector for the bispecific antibody of the present invention is pcDNA^{™} 3.4-TOPO(R) (Thermo Fisher Scientific) or pcDNA^{™} 3.1 (Thermo Fisher Scientific).

The expression vector for the anti-CD37/anti-CD3 bispecific antibody of the present invention may contain a promoter operably linked to the polynucleotide. Examples of the promoter for expressing the polynucleotide in an animal cell include a virus-derived promoter such as CMV, RSV and SV40, an actin promoter, an EF (elongation factor) 1α promoter, and a heat shock promoter. Examples of the promoter for expressing the polynucleotide in a bacterium (such as *Escherichia*) include a trp promoter, a lac promoter, a λPL promoter, and a tac promoter. Examples of the promoter for expressing the polynucleotide in a yeast include a GAL1 promoter, a GAL10 promoter, a PHO5 promoter, a PGK promoter, a GAP promoter, and an ADH promoter.

When an animal cell, an insect cell, or a yeast is used as the host cell for preparation of an expression vector, the expression vector for the bispecific antibody of the present invention can include a start codon and a stop codon. In this case, an enhancer sequence, the 5'- and 3'-untranslated regions of a gene encoding the antibody of the present invention, or the heavy chain or light chain thereof, a secretory signal sequence, a splice joint, a polyadenylation site, or a replicable unit may be included. When *E. coli* is used as the host cell, the expression vector of the present invention can include a start codon, a stop codon, a terminator region, and a replicable unit. The expression vector of the present invention may contain a drug selection marker gene usually used depending on purpose (such as a tetracycline resistance gene, an ampicillin resistance gene, a kanamycin resistance gene, a neomycin resistance gene, and a dihydrofolate reductase gene).

### <Host Cell Comprising Polynucleotide of Anti-CD37/Anti-CD3 Bispecific Antibody of Invention>

The present invention also provides a host cell that has been transformed by the expression vector of the anti-CD37/anti-CD3 bispecific antibody of the present invention or comprises the polynucleotide of the anti-CD37/anti-CD3 bispecific antibody in the cell (collectively also referred to as the "host cell comprising the polynucleotide of the anti-CD37/anti-CD3 bispecific antibody of the present invention"). The host cell comprises not only a cell to be cultured *in vitro* but also a cell that constitutes a tissue *in vivo.*

In one embodiment, the host cells comprising the polynucleotide of the anti-CD37/anti-CD3 bispecific antibody of the present invention comprises a polynucleotide of the anti-CD37/anti-CD3 bispecific antibody of the present invention described in (a) to (j) or an expression vector of the anti-CD37/anti-CD3 bispecific antibody of the present invention comprising the polynucleotide:
(a) a polynucleotide having a nucleotide sequence encoding a heavy chain fragment of an anti-CD37 antibody comprising a heavy chain variable region consisting of an amino acid sequence at amino acid positions 1 to 115 of SEQ ID NO: 10;
(b) a polynucleotide having a nucleotide sequence encoding a light chain of an anti-CD37 antibody comprising a light chain variable region consisting of an amino acid sequence at amino acid positions 1 to 105 of SEQ ID NO: 12;
(c) a polynucleotide having a nucleotide sequence encoding an anti-CD3 scFv region consisting of an amino acid sequence at amino acid positions 1 to 254 of SEQ ID NO: 14;
(d) a polynucleotide having a nucleotide sequence encoding an anti-CD3 scFv region consisting of an amino acid sequence at amino acid positions 1 to 254 of SEQ ID NO: 16;
(e) a polynucleotide having a nucleotide sequence encoding an anti-CD3 scFv region consisting of an amino acid sequence at amino acid positions 1 to 254 of SEQ ID NO: 18;
(f) a polynucleotide having a nucleotide sequence encoding a heavy chain of an anti-CD37 antibody consisting of an amino acid sequence of SEQ ID NO: 10;
(g) a polynucleotide having a nucleotide sequence encoding a light chain of an anti-CD37 antibody consisting of an amino acid sequence of SEQ ID NO: 12;
(h) a polynucleotide having a nucleotide sequence encoding a polypeptide consisting of an amino acid sequence of SEQ ID NO: 14 in which the anti-CD3 scFv region and the second Fc polypeptide are linked;
(i) a polynucleotide having a nucleotide sequence encoding a polypeptide consisting of an amino acid sequence of SEQ ID NO: 16 in which the anti-CD3 scFv region and the second Fc polypeptide are linked; or
(j) a polynucleotide having a nucleotide sequence encoding a polypeptide consisting of an amino acid sequence of SEQ ID NO: 18 in which the anti-CD3 scFv region and the second Fc polypeptide are linked.

In one embodiment, the host cell comprising the polynucleotide of the anti-CD37/anti-CD3 bispecific antibody of the present invention comprises a polynucleotide having a nucleotide sequence encoding a heavy chain fragment of an anti-CD37 antibody comprising a heavy chain variable region consisting of an amino acid sequence at amino acid positions 1 to 115 of SEQ ID NO: 10 and a polynucleotide having a nucleotide sequence encoding a light chain of an anti-CD37 antibody comprising a light chain variable region consisting of an amino acid sequence at amino acid positions 1 to 105 of SEQ ID NO: 12.

In one embodiment, the host cell comprising the polynucleotide of the anti-CD37/anti-CD3 bispecific antibody of the present invention comprises a polynucleotide of the anti-CD37/anti-CD3 bispecific antibody of the present invention described in any one of (k) to (p):
(k) a polynucleotide having a nucleotide sequence encoding an anti-CD3 scFV region comprising a heavy chain variable region of an anti-CD3 antibody consisting of an amino acid sequence at amino acid positions 1 to 125 of SEQ ID NO: 14 and a light chain variable region of an anti-CD3 antibody consisting of an amino acid sequence at amino acid positions 146 to 254 of SEQ ID NO: 14;
(l) a polynucleotide having a nucleotide sequence encoding an anti-CD3 scFV region comprising a heavy chain variable region of an anti-CD3 antibody consisting of an amino acid sequence at amino acid positions 1 to 125 of SEQ ID NO: 16 and a light chain variable region of an anti-CD3 antibody consisting of an amino acid sequence at amino acid positions 146 to 254 of SEQ ID NO: 16;
(m) a polynucleotide having a nucleotide sequence encoding an anti-CD3 scFV region comprising a heavy chain variable region of an anti-CD3 antibody consisting of an amino acid sequence at amino acid positions 1 to 125 of SEQ ID NO: 18 and a light chain variable region of an anti-CD3 antibody consisting of an amino acid sequence at amino acid positions 146 to 254 of SEQ ID NO: 18;
(n) a polynucleotide having a nucleotide sequence encoding a polypeptide in which a second Fc polypeptide is linked to an anti-CD3 scFV region comprising a heavy chain variable region of an anti-CD3 antibody consisting of an amino acid sequence at amino acid positions 1 to 125 of SEQ ID NO: 14 and a light chain variable region of an anti-CD3 antibody consisting of an amino acid sequence at amino acid positions 146 to 254 of SEQ ID NO: 14;
(o) a polynucleotide having a nucleotide sequence encoding a polypeptide in which a second Fc polypeptide is linked to an anti-CD3 scFV region comprising a heavy chain variable region of an anti-CD3 antibody consisting of an amino acid sequence at amino acid positions 1 to 125 of SEQ ID NO: 16 and a light chain variable region of an anti-CD3 antibody consisting of an amino acid sequence at amino acid positions 146 to 254 of SEQ ID NO: 16; and
(p) a polynucleotide having a nucleotide sequence encoding a polypeptide in which a second Fc polypeptide is linked to an anti-CD3 scFV region comprising a heavy chain variable region of an anti-CD3 antibody consisting of an amino acid sequence at amino acid positions 1 to 125 of SEQ ID NO: 18 and a light chain variable region of an anti-CD3 antibody consisting of an amino acid sequence at amino acid positions 146 to 254 of SEQ ID NO: 18.

In one embodiment, the host cell comprising the polynucleotide of the anti-CD37/anti-CD3 bispecific antibody of the present invention comprises polynucleotides described in any one of (a) to (c):
(a) a polynucleotide having a nucleotide sequence encoding a heavy chain fragment of an anti-CD37 antibody comprising a heavy chain variable region consisting of an amino acid sequence at amino acid positions 1 to 115 of SEQ ID NO: 10, a polynucleotide having a nucleotide sequence encoding a light chain of an anti-CD37 antibody comprising a light chain variable region consisting of an amino acid sequence at amino acid positions 1 to 105 of SEQ ID NO: 12, and a polynucleotide having a nucleotide sequence encoding an anti-CD3 scFV region comprising a heavy chain variable region of an anti-CD3 antibody consisting of an amino acid sequence at amino acid positions 1 to 125 of SEQ ID NO: 14 and a light chain variable region of an anti-CD3 antibody consisting of an amino acid sequence at amino acid positions 146 to 254 of SEQ ID NO: 14;
(b) a polynucleotide having a nucleotide sequence encoding a heavy chain fragment of an anti-CD37 antibody comprising a heavy chain variable region consisting of an amino acid sequence at amino acid positions 1 to 115 of SEQ ID NO: 10, a polynucleotide having a nucleotide sequence encoding a light chain of an anti-CD37 antibody comprising a light chain variable region consisting of an amino acid sequence at amino acid positions 1 to 105 of SEQ ID NO: 12, and a polynucleotide having a nucleotide sequence encoding an anti-CD3 scFV region comprising a heavy chain variable region of an anti-CD3 antibody consisting of an amino acid sequence at amino acid positions 1 to 125 of SEQ ID NO: 16 and a light chain variable region of an anti-CD3 antibody consisting of an amino acid sequence at amino acid positions 146 to 254 of SEQ ID NO: 16; and
(c) a polynucleotide having a nucleotide sequence encoding a heavy chain fragment of an anti-CD37 antibody comprising a heavy chain variable region consisting of an amino acid sequence at amino acid positions 1 to 115 of SEQ ID NO: 10, a polynucleotide having a nucleotide sequence encoding a light chain of an anti-CD37 antibody comprising a light chain variable region consisting of an amino acid sequence at amino acid positions 1 to 105 of SEQ ID NO: 12, and a polynucleotide having a nucleotide sequence encoding an anti-CD3 scFV region comprising a heavy chain variable region of an anti-CD3 antibody consisting of an amino acid sequence at amino acid positions 1 to 125 of SEQ ID NO: 18 and a light chain variable region of an anti-CD3 antibody consisting of an amino acid sequence at amino acid positions 146 to 254 of SEQ ID NO: 18.

In one embodiment, the host cell comprising the polynucleotide of an anti-CD37/anti-CD3 bispecific antibody of the present invention comprises polynucleotides described in any one of (a) to (c):
(a) a polynucleotide having a nucleotide sequence encoding a heavy chain of an anti-CD37 antibody in which a first Fc polypeptide is linked to a heavy chain fragment of an anti-CD37 antibody comprising a heavy chain variable region consisting of an amino acid sequence at amino acid positions 1 to 115 of SEQ ID NO: 10, a polynucleotide having a nucleotide sequence encoding a light chain of an anti-CD37 antibody comprising a light chain variable region consisting of an amino acid sequence at amino acid positions 1 to 105 of SEQ ID NO: 12, and a polynucleotide having a nucleotide sequence encoding a polypeptide in which a second Fc polypeptide is linked to an anti-CD3 scFV region comprising a heavy chain variable region of an anti-CD3 antibody consisting of an amino acid sequence at amino acid positions 1 to 125 of SEQ ID NO: 14 and a light chain variable region of an anti-CD3 antibody consisting of an amino acid sequence at amino acid positions 146 to 254 of SEQ ID NO: 14;
(b) a polynucleotide having a nucleotide sequence encoding a heavy chain of an anti-CD37 antibody in which a first Fc polypeptide is linked to a heavy chain fragment of an anti-CD37 antibody comprising a heavy chain variable region consisting of an amino acid sequence at amino acid positions 1 to 115 of SEQ ID NO: 10, a polynucleotide having a nucleotide sequence encoding a light chain of an anti-CD37 antibody comprising a light chain variable region consisting of an amino acid sequence at amino acid positions 1 to 105 of SEQ ID NO: 12, and a polynucleotide having a nucleotide sequence encoding a polypeptide in which a second Fc polypeptide is linked to an anti-CD3 scFV region comprising a heavy chain variable region of an anti-CD3 antibody consisting of an amino acid sequence at amino acid positions 1 to 125 of SEQ ID NO: 16 and a light chain variable region of an anti-CD3 antibody consisting of an amino acid sequence at amino acid positions 146 to 254 of SEQ ID NO: 16; and
(c) a polynucleotide having a nucleotide sequence encoding a heavy chain of an anti-CD37 antibody in which a first Fc polypeptide is linked to a heavy chain fragment of an anti-CD37 antibody comprising a heavy chain variable region consisting of an amino acid sequence at amino acid positions 1 to 115 of SEQ ID NO: 10, a polynucleotide having a nucleotide sequence encoding a light chain of an anti-CD37 antibody comprising a light chain variable region consisting of an amino acid sequence at amino acid positions 1 to 105 of SEQ ID NO: 12, and a polynucleotide having a nucleotide sequence encoding a polypeptide in which a second Fc polypeptide is linked to an anti-CD3 scFV region comprising a heavy chain variable region of an anti-CD3 antibody consisting of an amino acid sequence at amino acid positions 1 to 125 of SEQ ID NO: 18 and a light chain variable region of an anti-CD3 antibody consisting of an amino acid sequence at amino acid positions 146 to 254 of SEQ ID NO: 18.

In one embodiment, the host cell comprising the polynucleotide of the anti-CD37/anti-CD3 bispecific antibody of the present invention comprises polynucleotides described in any one of (a) to (c):
(a) a polynucleotide having a nucleotide sequence encoding a heavy chain of an anti-CD37 antibody consisting of an amino acid sequence of SEQ ID NO: 10, a polynucleotide having a nucleotide sequence encoding a light chain of an anti-CD37 antibody consisting of an amino acid sequence of SEQ ID NO: 12, and a polynucleotide having a nucleotide sequence encoding a polypeptide consisting of an amino acid sequence of SEQ ID NO: 14 in which the anti-CD3 scFv region and the second Fc polypeptide are linked;
(b) a polynucleotide having a nucleotide sequence encoding a heavy chain of an anti-CD37 antibody consisting of an amino acid sequence of SEQ ID NO: 10, a polynucleotide having a nucleotide sequence encoding a light chain of an anti-CD37 antibody consisting of an amino acid sequence of SEQ ID NO: 12, and a polynucleotide having a nucleotide sequence encoding a polypeptide consisting of an amino acid sequence of SEQ ID NO: 16 in which the anti-CD3 scFv region and the second Fc polypeptide are linked; and
(c) a polynucleotide having a nucleotide sequence encoding a heavy chain of an anti-CD37 antibody consisting of an amino acid sequence of SEQ ID NO: 10, a polynucleotide having a nucleotide sequence encoding a light chain of an anti-CD37 antibody consisting of an amino acid sequence of SEQ ID NO: 12, and a polynucleotide having a nucleotide sequence encoding a polypeptide consisting of an amino acid sequence of SEQ ID NO: 18 in which the anti-CD3 scFv region and the second Fc polypeptide are linked.

The host cell comprising the polynucleotide of the anti-CD37/anti-CD3 bispecific antibody of the present invention is not particularly limited as long as it is compatible with the expression vector or gene transfer method used and can express the polypeptide encoded by the polynucleotide. Examples of the host cell include various cells such as conventional cells usually used in this technical field and artificially established cells (e.g., animal cells (such as a CHO-K1 cell, an ExpiCHO-S (R) cell, a CHOK1SV cell, a CHO-DG44 cell, a HEK293 cell, and a NS0 cell), insect cells (such as Sf9), bacteria (such as *Escherichia*), and yeasts (such as *Saccharomyces* and *Pichia*)*.* In one embodiment, the host cell of the present invention is a CHO-K1 cell or an ExpiCHO-S (R) cell.

The method of introducing the polynucleotide of the anti-CD37/anti-CD3 bispecific antibody of the present invention or the expression vector of the anti-CD37/anti-CD3 bispecific antibody of the present invention into the host cell is not particularly limited, and for example, a method generally used by those skilled in the art, such as the calcium phosphate method, the electroporation method, and the lipofection method, can be used. Generally known delivery systems for polynucleotides such as liposomes, lipid nanoparticles, and exosomes can be used as lipofection methods.

Selection of the host cell comprising polynucleotide of the anti-CD37/anti-CD3 bispecific antibody of the present invention can be performed by a method usually used by those skilled in the art. Examples of the selection method available include a drug selection method using a drug selection marker gene and a drug such as tetracycline, ampicillin, neomycin and hygromycin; and a cell isolation method such as a limiting dilution method, a single cell sorting method, and a colony pick-up method.

### <Production Method of Anti-CD37/Anti-CD3 Bispecific Antibody of Invention>

The present invention also provides a method for producing the anti-CD37/anti-CD3 bispecific antibody of the present invention (also referred to as the "production method of the anti-CD37/anti-CD3 bispecific antibody of the present invention"). The production method of the anti-CD37/anti-CD3 bispecific antibody of the present invention can include culturing the host cell comprising the polynucleotide of the anti-CD37/anti-CD3 bispecific antibody described in the section of <Host Cell Comprising Polynucleotide of Anti-CD37/Anti-CD3 Bispecific Antibody of Invention> to express the antibody in the cell or a culture supernatant, and a method for collecting, isolating and purifying the antibody, for example, but is not limited to such a method as long as the anti-CD37/anti-CD3 bispecific antibody of the present invention can be produced.

The host cell comprising the polynucleotide of the anti-CD37/anti-CD3 bispecific antibody of the present invention can be cultured by a known method. Culture conditions such as a temperature, pH of a medium and a culture time can be appropriately selected by those skilled in the art. When the host cell is an animal cell, for example, MEM medium (Science, 1959, Vol. 130, p. 432-437), DMEM medium (Virol., 1959, Vol. 8, p. 396), RPMI-1640 medium (J. Am. Med. Assoc., 1967, Vol. 199, p. 519), or 199 medium (Exp. Biol. Med., 1950, Vol. 73, p. 1-8) comprising about 5 to 20% of fetal bovine serum can be used as the medium. The pH of the medium is, for example, about 6 to 8, and the culture is performed usually at about 30 to 40°C for about 15 to 336 hours with aeration or stirring, if necessary. When the host cell is an insect cell, for example, Grace's medium (Proc. Natl. Acad. Sci. USA., 1985, Vol. 82, p. 8404) comprising fetal bovine serum or the like can be used as the medium. The pH of the medium is, for example, about 5 to 8, and the culture is performed usually at about 20 to 40°C for about 15 to 100 hours with aeration or stirring, if necessary. When the host cell is *E. coli* or a yeast, for example, a liquid medium comprising a nutrition source is suitably used as the medium. The nutrient medium comprises, for example, a carbon source, an inorganic nitrogen source, or an organic nitrogen source necessary for growth of the transformed host cell. Examples of the carbon source include glucose, dextran, soluble starch, and sucrose, examples of the inorganic nitrogen source or organic nitrogen source include ammonium salts, nitric acid salts, amino acids, a corn steep liquor, peptone, casein, a meat extract, a soybean cake, and a potato extract. Other nutrients (e.g., inorganic salts (such as calcium chloride, sodium dihydrogen phosphate, and magnesium chloride) or vitamins), an antibiotic (such as tetracycline, neomycin, ampicillin, or kanamycin), or the like may be contained, if desired. The pH of the medium is, for example, about 5 to 8. When the host cell is *E. coli,* for example, LB medium, M9 medium (Molecular Cloning, Cold Spring Harbor Laboratory, Vol. 3, A2.2) or the like can be used as the medium. The culture is performed usually at about 14 to 43°C for about 3 to 24 hours with aeration or stirring, if necessary. When the host cell is a yeast, for example, Burkholder minimum medium (Proce. Natl. Acad. Sci. USA., 1980, Vol. 77, p. 4505) or the like can be used as the medium. The culture is performed usually at about 20 to 35°C for about 14 to 144 hours with aeration or stirring, if necessary. Through such culture, the anti-CD37/anti-CD3 bispecific antibody of the present invention can be expressed.

The production method of the anti-CD37/anti-CD3 bispecific antibody of the present invention can include, in addition to culturing the host cell comprising a polynucleotide of the anti-CD37/anti-CD3 bispecific antibody of the present invention to express the anti-CD37/anti-CD3 bispecific antibody, collecting, isolating or purifying the anti-CD37/anti-CD3 bispecific antibody from the transformed host cell. Examples of an isolation or purification method include a method utilizing solubility such as salting-out and a solvent precipitation method; a method utilizing a difference in a molecular weight such as dialysis, ultrafiltration, and gel filtration; a method utilizing charge such as ion exchange chromatography and hydroxyapatite chromatography; a method utilizing specific affinity such as affinity chromatography; a method utilizing a difference in hydrophobicity such as reverse phase high performance liquid chromatography; and a method utilizing a difference in an isoelectric point such as isoelectric focusing. In one embodiment, the antibody secreted into a culture supernatant can be purified by various chromatography such as column chromatography using a protein A column or a protein G column.

The anti-CD37/anti-CD3 bispecific antibody of the present invention also comprises an anti-CD37/anti-CD3 bispecific antibody produced by the production method of the anti-CD37/anti-CD3 bispecific antibody of the present invention.

### <Pharmaceutical Composition of Anti-CD37/Anti-CD3 Bispecific Antibody of Invention>

The present invention also provides a pharmaceutical composition of the anti-CD37/anti-CD3 bispecific antibody (also referred to as the "pharmaceutical composition of the anti-CD37/anti-CD3 bispecific antibody of the present invention"). The pharmaceutical composition of the anti-CD37/anti-CD3 bispecific antibody of the present invention comprises a pharmaceutical composition comprising the anti-CD37/anti-CD3 bispecific antibody of the present invention and a pharmaceutically acceptable excipient. The pharmaceutical composition of the present invention can be prepared by a method usually used with an excipient usually used in this technical field, i.e., a pharmaceutical excipient, a pharmaceutical carrier, or the like. Examples of a dosage form of such a pharmaceutical composition include parenteral agents, such as an injection and a drop, which can be administered by intravenous administration, subcutaneous administration, intraperitoneal administration, or the like. In formulation, an excipient, a carrier, an additive, or the like suitable to the dosage form can be used in a pharmaceutically acceptable range.

The pharmaceutical composition of the present invention can contain a post-translationally modified product of the anti-CD37/anti-CD3 bispecific antibody of the present invention. For example, a pharmaceutical composition comprising an antibody or the like which has undergone both of or one of lysine deletion at the C-terminal and pyroglutamylation at the N-terminal can be embraced in the present invention.

In one embodiment, the pharmaceutical composition of the anti-CD37/anti-CD3 bispecific antibody of the present invention is a pharmaceutical composition comprising an anti-CD37/anti-CD3 bispecific antibody of the present invention selected from any one of (a) to (c) and/or a post-translationally modified product of the antibody:
(a) a bispecific antibody comprising a heavy chain of an anti-CD37 antibody in which a first Fc polypeptide is linked to a heavy chain fragment of the anti-CD37 antibody comprising a heavy chain variable region consisting of an amino acid sequence at amino acid positions 1 to 115 of SEQ ID NO: 10; a light chain of an anti-CD37 antibody comprising a light chain variable region consisting of an amino acid sequence at amino acid positions 1 to 105 of SEQ ID NO: 12; and a polypeptide in which a second Fc polypeptide is linked to an anti-CD3 scFV region comprising a heavy chain variable region of an anti-CD3 antibody consisting of an amino acid sequence at amino acid positions 1 to 125 of SEQ ID NO: 14 and a light chain variable region of an anti-CD3 antibody consisting of an amino acid sequence at amino acid positions 146 to 254 of SEQ ID NO: 14;
(b) a bispecific antibody comprising a heavy chain of an anti-CD37 antibody in which a first Fc polypeptide is linked to a heavy chain fragment of the anti-CD37 antibody comprising a heavy chain variable region consisting of an amino acid sequence at amino acid positions 1 to 115 of SEQ ID NO: 10; a light chain of an anti-CD37 antibody comprising a light chain variable region consisting of an amino acid sequence at amino acid positions 1 to 105 of SEQ ID NO: 12; and a polypeptide in which a second Fc polypeptide is linked to an anti-CD3 scFV region comprising a heavy chain variable region of an anti-CD3 antibody consisting of an amino acid sequence at amino acid positions 1 to 125 of SEQ ID NO: 16 and a light chain variable region of an anti-CD3 antibody consisting of an amino acid sequence at amino acid positions 146 to 254 of SEQ ID NO: 16; and
(c) a bispecific antibody comprising a heavy chain of an anti-CD37 antibody in which a first Fc polypeptide is linked to a heavy chain fragment of the anti-CD37 antibody comprising a heavy chain variable region consisting of an amino acid sequence at amino acid positions 1 to 115 of SEQ ID NO: 10; a light chain of an anti-CD37 antibody comprising a light chain variable region consisting of an amino acid sequence at amino acid positions 1 to 105 of SEQ ID NO: 12; and a polypeptide in which a second Fc polypeptide is linked to an anti-CD3 scFV region comprising a heavy chain variable region of an anti-CD3 antibody consisting of an amino acid sequence at amino acid positions 1 to 125 of SEQ ID NO: 18 and a light chain variable region of an anti-CD3 antibody consisting of an amino acid sequence at amino acid positions 146 to 254 of SEQ ID NO: 18.

In one embodiment, the pharmaceutical composition of the present invention is a pharmaceutical composition comprising an anti-CD37/anti-CD3 bispecific antibody of the present invention selected from any one of (a) to (c) and/or a post-translationally modified product of the antibody:
(a) a bispecific antibody comprising a heavy chain of an anti-CD37 antibody consisting of an amino acid sequence of SEQ ID NO: 10, a light chain of an anti-CD37 antibody consisting of an amino acid sequence of SEQ ID NO: 12, and a polypeptide consisting of an amino acid sequence of SEQ ID NO: 14 in which the anti-CD3 scFv region and the second Fc polypeptide are linked;
(b) a bispecific antibody comprising a heavy chain of an anti-CD37 antibody consisting of an amino acid sequence of SEQ ID NO: 10, a light chain of an anti-CD37 antibody consisting of an amino acid sequence of SEQ ID NO: 12, and a polypeptide consisting of an amino acid sequence of SEQ ID NO: 16 in which the anti-CD3 scFv region and the second Fc polypeptide are linked; and
(c) a bispecific antibody comprising a heavy chain of an anti-CD37 antibody consisting of an amino acid sequence of SEQ ID NO: 10, a light chain of an anti-CD37 antibody consisting of an amino acid sequence of SEQ ID NO: 12, and a polypeptide consisting of an amino acid sequence of SEQ ID NO: 18 in which the anti-CD3 scFv region and the second Fc polypeptide are linked.

In one embodiment, the pharmaceutical composition of the present invention is a pharmaceutical composition comprising a bispecific antibody comprising a heavy chain of an anti-CD37 antibody consisting of an amino acid sequence of SEQ ID NO: 10, a light chain of an anti-CD37 antibody consisting of an amino acid sequence of SEQ ID NO: 12, and a polypeptide consisting of an amino acid sequence of SEQ ID NO: 14 in which the anti-CD3 scFv region and the second Fc polypeptide are linked, and/or a post-translationally modified product of the antibody.

In one embodiment, the pharmaceutical composition of the present invention is a pharmaceutical composition comprising a bispecific antibody comprising a heavy chain of an anti-CD37 antibody consisting of an amino acid sequence of SEQ ID NO: 10, a light chain of an anti-CD37 antibody consisting of an amino acid sequence of SEQ ID NO: 12, and a polypeptide consisting of an amino acid sequence of SEQ ID NO: 16 in which the anti-CD3 scFv region and the second Fc polypeptide are linked, and/or a post-translationally modified product of the antibody.

In one embodiment, the pharmaceutical composition of the present invention is a pharmaceutical composition comprising a bispecific antibody comprising a heavy chain of an anti-CD37 antibody consisting of an amino acid sequence of SEQ ID NO: 10, a light chain of an anti-CD37 antibody consisting of an amino acid sequence of SEQ ID NO: 12, and a polypeptide consisting of an amino acid sequence of SEQ ID NO: 18 in which the anti-CD3 scFv region and the second Fc polypeptide are linked, and/or a post-translationally modified product of the antibody.

The amounts of the anti- CD37/anti-CD3 bispecific antibody of the present invention added in formulation are varied depending on the degree of symptom and the age of a patient, a dosage form of the formulation to be used, a binding titer of the antibody, or the like, and for example, can be approximately 0.001 mg/kg to 100 mg/kg.

### <Pharmaceutical Use of Anti-CD37/Anti-CD3 Bispecific Antibody of Invention>

The anti-CD37/anti-CD3 bispecific antibody of the present invention and a pharmaceutical composition comprising the same can be used for treating cancer. Furthermore, the present invention comprises a method for treating cancer, the method comprising administering, to a subject, a therapeutically effective amount of the anti-CD37/anti-CD3 bispecific antibody of the present invention. Moreover, the present invention comprises the anti-CD37/anti-CD3 bispecific antibody for treating cancer. In addition, the present invention comprises use of the anti-CD37/anti-CD3 bispecific antibody of the present invention in production of a pharmaceutical composition for treating cancer. Examples of the cancer to be treated by the present invention include, but are not particularly limited to, peritoneal dissemination of various cancer cells; gastric cancer; lung cancer; blood cancers such as acute lymphoblastic leukemia (ALL), acute myelogenous leukemia (AML), chronic myelogenous leukemia (CML), chronic lymphocytic leukemia (CLL), Hodgkin's lymphoma, non-Hodgkin lymphoma, B cell lymphoma, multiple myeloma, T cell lymphoma, and myelodysplastic syndromes; solid cancers such as adenocarcinoma, squamous cell carcinoma, adenosquamous carcinoma, undifferentiated carcinoma, large cell carcinoma, non-small cell lung cancer, small cell lung cancer, mesothelioma, skin cancer, skin T cell lymphoma, breast cancer, prostate cancer, bladder cancer, vaginal cancer, cervix cancer, head and neck cancer, uterine cancer, cervical cancer, liver cancer, gallbladder cancer, bile duct cancer, kidney cancer, pancreatic cancer, colon cancer, colorectal cancer, rectal cancer, small intestine cancer, gastric cancer, esophageal cancer, testicular cancer, ovarian cancer and brain tumor; cancers of bone tissues, cartilage tissues, adipose tissues, muscle tissues, vascular tissues, and blood-forming tissues; sarcomas such as chondrosarcoma, Ewing's sarcoma, malignant hemangioendothelioma, malignant schwannoma, osteosarcoma, and soft tissue sarcoma; and blastomas such as glioblastoma, glioblastoma multiforme, hepatoblastoma, medulloblastoma, nephroblastoma, neuroblastoma, pancreatoblastoma, pleuropulmonary blastoma, and retinoblastoma.

In one embodiment, the cancer to be treated by the present invention is non-Hodgkin lymphoma, B cell lymphoma, CLL, AML, or T cell lymphoma. In one embodiment, the cancer to be treated by the present invention is preferably non-Hodgkin lymphoma, B cell lymphoma, or CLL.

### <Anti-TAA/Anti-CD3 Multispecific Antibody of Invention>

The present invention also provides an anti-tumor associated antigen (TAA)/anti-CD3 multispecific antibody (also referred to as the "anti-TAA/anti-CD3 multispecific antibody of the present invention") comprising a heavy chain variable region and a light chain variable region of an anti-TAA antibody that binds to TAA; and an anti-CD3 scFv region comprising a heavy chain variable region and a light chain variable region of an anti-CD3 antibody that binds to CD3.

In one embodiment, the anti-CD3 scFv region of the anti-TAA/anti-CD3 multispecific antibody of the present invention is any one of (1) to (16):
(1) an anti-CD3 scFv region comprising a heavy chain variable region of an anti-CD3 antibody consisting of an amino acid sequence at amino acid positions 1 to 125 of SEQ ID NO: 14 and a light chain variable region of an anti-CD3 antibody consisting of an amino acid sequence at amino acid positions 146 to 254 of SEQ ID NO: 14;
(2) an anti-CD3 scFv region comprising a heavy chain variable region of an anti-CD3 antibody consisting of an amino acid sequence at amino acid positions 1 to 125 of SEQ ID NO: 16 and a light chain variable region of an anti-CD3 antibody consisting of an amino acid sequence at amino acid positions 146 to 254 of SEQ ID NO: 16;
(3) an anti-CD3 scFv region comprising a heavy chain variable region of an anti-CD3 antibody consisting of an amino acid sequence at amino acid positions 1 to 125 of SEQ ID NO: 18 and a light chain variable region of an anti-CD3 antibody consisting of an amino acid sequence at amino acid positions 146 to 254 of SEQ ID NO: 18;
(4) an anti-CD3 scFv region comprising a heavy chain variable region of an anti-CD3 antibody consisting of an amino acid sequence at amino acid positions 1 to 125 of SEQ ID NO: 20 and a light chain variable region of an anti-CD3 antibody consisting of an amino acid sequence at amino acid positions 146 to 254 of SEQ ID NO: 20;
(5) an anti-CD3 scFv region comprising a heavy chain variable region of an anti-CD3 antibody consisting of an amino acid sequence at amino acid positions 1 to 125 of SEQ ID NO: 22 and a light chain variable region of an anti-CD3 antibody consisting of an amino acid sequence at amino acid positions 146 to 254 of SEQ ID NO: 22;
(6) an anti-CD3 scFv region comprising a heavy chain variable region of an anti-CD3 antibody consisting of an amino acid sequence at amino acid positions 1 to 125 of SEQ ID NO: 24 and a light chain variable region of an anti-CD3 antibody consisting of an amino acid sequence at amino acid positions 146 to 254 of SEQ ID NO: 24;
(7) an anti-CD3 scFv region comprising a heavy chain variable region of an anti-CD3 antibody consisting of an amino acid sequence at amino acid positions 1 to 125 of SEQ ID NO: 26 and a light chain variable region of an anti-CD3 antibody consisting of an amino acid sequence at amino acid positions 146 to 254 of SEQ ID NO: 26;
(8) an anti-CD3 scFv region comprising a heavy chain variable region of an anti-CD3 antibody consisting of an amino acid sequence at amino acid positions 1 to 125 of SEQ ID NO: 28 and a light chain variable region of an anti-CD3 antibody consisting of an amino acid sequence at amino acid positions 146 to 254 of SEQ ID NO: 28;
(9) an anti-CD3 scFv region comprising a heavy chain variable region of an anti-CD3 antibody consisting of an amino acid sequence at amino acid positions 1 to 125 of SEQ ID NO: 30 and a light chain variable region of an anti-CD3 antibody consisting of an amino acid sequence at amino acid positions 146 to 254 of SEQ ID NO: 30;
(10) an anti-CD3 scFv region comprising a heavy chain variable region of an anti-CD3 antibody consisting of an amino acid sequence at amino acid positions 1 to 125 of SEQ ID NO: 32 and a light chain variable region of an anti-CD3 antibody consisting of an amino acid sequence at amino acid positions 146 to 254 of SEQ ID NO: 32;
(11) an anti-CD3 scFv region comprising a heavy chain variable region of an anti-CD3 antibody consisting of an amino acid sequence at amino acid positions 1 to 125 of SEQ ID NO: 34 and a light chain variable region of an anti-CD3 antibody consisting of an amino acid sequence at amino acid positions 146 to 254 of SEQ ID NO: 34;
(12) an anti-CD3 scFv region comprising a heavy chain variable region of an anti-CD3 antibody consisting of an amino acid sequence at amino acid positions 1 to 125 of SEQ ID NO: 36 and a light chain variable region of an anti-CD3 antibody consisting of an amino acid sequence at amino acid positions 146 to 254 of SEQ ID NO: 36;
(13) an anti-CD3 scFv region comprising a heavy chain variable region of an anti-CD3 antibody consisting of an amino acid sequence at amino acid positions 1 to 125 of SEQ ID NO: 38 and a light chain variable region of an anti-CD3 antibody consisting of an amino acid sequence at amino acid positions 146 to 254 of SEQ ID NO: 38;
(14) an anti-CD3 scFv region comprising a heavy chain variable region of an anti-CD3 antibody consisting of an amino acid sequence at amino acid positions 1 to 125 of SEQ ID NO: 40 and a light chain variable region of an anti-CD3 antibody consisting of an amino acid sequence at amino acid positions 146 to 254 of SEQ ID NO: 40;
(15) an anti-CD3 scFv region comprising a heavy chain variable region of an anti-CD3 antibody consisting of an amino acid sequence at amino acid positions 1 to 125 of SEQ ID NO: 42 and a light chain variable region of an anti-CD3 antibody consisting of an amino acid sequence at amino acid positions 146 to 254 of SEQ ID NO: 42; and
(16) an anti-CD3 scFv region comprising a heavy chain variable region of an anti-CD3 antibody consisting of an amino acid sequence at amino acid positions 1 to 125 of SEQ ID NO: 44 and a light chain variable region of an anti-CD3 antibody consisting of an amino acid sequence at amino acid positions 146 to 254 of SEQ ID NO: 44.

In one embodiment, the anti-CD3 scFv region of the anti-TAA/anti-CD3 multispecific antibody of the present invention is any one of (1) to (16):
(1) an anti-CD3 scFv region consisting of an amino acid sequence at amino acid positions 1 to 254 of SEQ ID NO: 14;
(2) an anti-CD3 scFv region consisting of an amino acid sequence at amino acid positions 1 to 254 of SEQ ID NO: 16;
(3) an anti-CD3 scFv region consisting of an amino acid sequence at amino acid positions 1 to 254 of SEQ ID NO: 18;
(4) an anti-CD3 scFv region consisting of an amino acid sequence at amino acid positions 1 to 254 of SEQ ID NO: 20;
(5) an anti-CD3 scFv region consisting of an amino acid sequence at amino acid positions 1 to 254 of SEQ ID NO: 22;
(6) an anti-CD3 scFv region consisting of an amino acid sequence at amino acid positions 1 to 254 of SEQ ID NO: 24;
(7) an anti-CD3 scFv region consisting of an amino acid sequence at amino acid positions 1 to 254 of SEQ ID NO: 26;
(8) an anti-CD3 scFv region consisting of an amino acid sequence at amino acid positions 1 to 254 of SEQ ID NO: 28;
(9) an anti-CD3 scFv region consisting of an amino acid sequence at amino acid positions 1 to 254 of SEQ ID NO: 30;
(10) an anti-CD3 scFv region consisting of an amino acid sequence at amino acid positions 1 to 254 of SEQ ID NO: 32;
(11) an anti-CD3 scFv region consisting of an amino acid sequence at amino acid positions 1 to 254 of SEQ ID NO: 34;
(12) an anti-CD3 scFv region consisting of an amino acid sequence at amino acid positions 1 to 254 of SEQ ID NO: 36;
(13) an anti-CD3 scFv region consisting of an amino acid sequence at amino acid positions 1 to 254 of SEQ ID NO: 38;
(14) an anti-CD3 scFv region consisting of an amino acid sequence at amino acid positions 1 to 254 of SEQ ID NO: 40;
(15) an anti-CD3 scFv region consisting of an amino acid sequence at amino acid positions 1 to 254 of SEQ ID NO: 42; and
(16) an anti-CD3 scFv region consisting of an amino acid sequence at amino acid positions 1 to 254 of SEQ ID NO: 44.

In one embodiment, the anti-TAA/anti-CD3 multispecific antibody of the present invention provides an anti-TAA/anti-CD3 bispecific antibody. Various formats of a bispecific antibody comprising a scFv region are known in this technical field, and any of these formats can be used for a bispecific antibody, the anti-TAA/anti-CD3 multispecific antibody of the present invention (mAbs, 2017, Vol. 9, p. 182-212).

In one embodiment, the anti-TAA/anti-CD3 multispecific antibody of the present invention is a bispecific antibody (also referred to as the "anti-TAA/anti-CD3 bispecific antibody of the present invention") having structures according to (a) to (c):
(a) a Fab region of an anti-TAA antibody consisting of a heavy chain fragment comprising a heavy chain variable region of the anti-TAA antibody and a light chain comprising a light chain variable region of the anti-TAA antibody;
(b) an anti-CD3 scFV region comprising a heavy chain variable region and a light chain variable region of an anti-CD3 antibody; and
(c) an Fc region consisting of a first Fc polypeptide linked to the heavy chain fragment of the Fab region (a) and a second Fc polypeptide linked to the anti-CD3 scFv region (b).

In the anti-TAA/anti-CD3 bispecific antibody of the present invention, any one of the constant regions Igy, Igµ , Igα, Igδ, and Igε can be selected as the Fc region. The Igy can be selected from, for example, Igγ1, Igγ2, Igγ3, and Igγ4. In one embodiment, the first Fc polypeptide and the second Fc polypeptide are Fc polypeptides derived from the human Igγ1 constant region. The Fc region may have, for example, a knobs-into holes-mutation. Examples of the knobs-into-holes mutation to be used include, but are not limited to, T366W mutation in one Fc polypeptide forming the Fc region, and T366S, L368A, and Y407V mutations in another Fc polypeptide forming the Fc region (see International Publication No. WO 1998/050431). In one embodiment, the knobs-into-holes mutation contained in the anti-TAA/anti-CD3 multispecific antibody of the present invention is T366W mutation in one Fc polypeptide forming the Fc region, and T366S, L368A, and Y407V mutations in another Fc polypeptide forming the Fc region.

The Fc region in the anti-TAA/anti-CD3 bispecific antibody of the present invention may contain mutation that deteriorates ACCC or CDC described in the section of <Anti-CD37/Anti-CD3 Bispecific Antibody of Invention>. Examples of the mutation that deteriorates ADCC or CDC include, but are not limited to, LALA mutation.

The Fc region in the anti-TAA/anti-CD3 bispecific antibody of the present invention may further contain mutation based on another known technique. For example, the Fc region may contain P329A mutation, P331G mutation, or P331S mutation. The P329A mutation is a replacement of proline with alanine at amino acid position 329 in human Igγ1 constant region, and the P331G mutation and P331S mutation are each a replacement of proline with glycine or serine at amino acid position 331 in human Igγ1 constant region. The P329A mutation is known to deteriorate the ADCC activity of the antibodies, and the P329A mutation, P331G mutation, and P331S mutation are known to deteriorate the CDC of the antibodies (J Immunol., 2000, Vol. 164 (8). p. 4049-4184). Here the positions 329 and 331 are amino acid positions in human Igyl constant region according to EU index.

In one embodiment, the anti-TAA/anti-CD3 bispecific antibody of the present invention comprises an Fc region comprising knobs-into-holes mutation. In one embodiment, the anti-TAA/anti-CD3 multispecific antibody of the present invention comprises an Fc region comprising amino acid mutations of L234A and L235A (LALA mutation). In one embodiment, the anti-TAA/anti-CD3 bispecific antibody of the present invention comprises an Fc region comprising knobs-into-holes mutation or LALA mutation. In one embodiment, the anti-TAA/anti-CD3 bispecific antibody of the present invention comprises an Fc region comprising knobs-into-holes mutation and LALA mutation. In one embodiment, the anti-TAA/anti-CD3 bispecific antibody of the present invention comprises an Fc region comprising P329A mutation. In one embodiment, the anti-TAA/anti-CD3 bispecific antibody of the present invention comprises an Fc region comprising P331G mutation or P331S mutation. In one embodiment, the anti-TAA/anti-CD3 bispecific antibody of the present invention comprises an Fc region comprising one or more mutations of the knobs-into-holes mutation, LALA mutation, P329A mutation, P331G mutation, and P331S mutation. In one embodiment, the anti-TAA/anti-CD3 bispecific antibody of the present invention comprises an Fc region comprising a knobs-Into-holes mutation and/or LALA mutation, and P329A mutation and/or P331G mutation or P331S mutation. In one embodiment, the anti-TAA/anti-CD3 bispecific antibody of the present invention comprises an Fc region comprising knobs-into-holes mutation and/or LALA mutation, and P329A mutation and/or P331G mutation.

In the anti-TAA/anti-CD3 bispecific antibody of the present invention, the heavy chain fragment of the Fab region of the anti-TAA antibody and the first Fc polypeptide may be linked via a hinge region. Alternatively, in the anti-TAA/anti-CD3 bispecific antibody of the present invention, the anti-CD3 scFv region and the second Fc polypeptide may be linked via a hinge region. In one embodiment, the anti-TAA/anti-CD3 bispecific antibody of the present invention comprises a heavy chain fragment of the Fab region of the anti-TAA antibody and the first Fc polypeptide which are linked via a hinge region, and the anti-CD3 scFv region and the second Fc polypeptide which are linked via a hinge region.

In the case of comprising a hinge region, the anti-TAA/anti-CD3 bispecific antibody of the present invention may contain a mutation in the hinge region based on a known technique. For example, the hinge region may contain C220S mutation (Methods, 2019, Vol. 154, p. 38-50). The C220S mutation aims to remove the cysteine at position 220, which has become free as a result of loss of the paired cysteine due to loss of the cysteine present in the light chain constant region. In one embodiment, the anti-TAA/anti-CD3 bispecific antibody of the present invention comprises C220S mutation in the hinge region linking the anti-CD3 scFv region to the second Fc polypeptide. In one embodiment, the anti-TAA/anti-CD3 bispecific antibody of the present invention comprises knobs-into-holes mutation, LALA mutation, and P329A mutation and/or P331G mutation in the Fc region, and C220S mutation in the hinge region linking the anti-CD3 scFv region to the second Fc polypeptide.

In one embodiment, the anti-TAA/anti-CD3 multispecific antibody of the present invention is
a multispecific antibody which is an anti-TAA/anti-CD3 bispecific antibody, comprising: a heavy chain of an anti-TAA antibody in which the first Fc polypeptide is linked to a heavy chain fragment comprising a heavy chain variable region of the anti-TAA antibody; a light chain comprising a light chain variable region of the anti-TAA antibody; and a polypeptide in which the anti-CD3 scFv region comprising a heavy chain variable region and a light chain variable region of an anti-CD3 antibody that binds to CD3 and a second Fc polypeptide are linked, wherein the polypeptide, in which the anti-CD3 scFv region and the second Fc polypeptide are linked, is any one of (1) to (16):
(1) a polypeptide consisting of an amino acid sequence of SEQ ID NO: 14 in which the anti-CD3 scFv region and the second Fc polypeptide are linked;
(2) a polypeptide consisting of an amino acid sequence of SEQ ID NO: 16 in which the anti-CD3 scFv region and the second Fc polypeptide are linked;
(3) a polypeptide consisting of an amino acid sequence of SEQ ID NO: 18 in which the anti-CD3 scFv region and the second Fc polypeptide are linked;
(4) a polypeptide consisting of an amino acid sequence of SEQ ID NO: 20 in which the anti-CD3 scFv region and the second Fc polypeptide are linked;
(5) a polypeptide consisting of an amino acid sequence of SEQ ID NO: 22 in which the anti-CD3 scFv region and the second Fc polypeptide are linked;
(6) a polypeptide consisting of an amino acid sequence of SEQ ID NO: 24 in which the anti-CD3 scFv region and the second Fc polypeptide are linked;
(7) a polypeptide consisting of an amino acid sequence of SEQ ID NO: 26 in which the anti-CD3 scFv region and the second Fc polypeptide are linked;
(8) a polypeptide consisting of an amino acid sequence of SEQ ID NO: 28 in which the anti-CD3 scFv region and the second Fc polypeptide are linked;
(9) a polypeptide consisting of an amino acid sequence of SEQ ID NO: 30 in which the anti-CD3 scFv region and the second Fc polypeptide are linked;
(10) a polypeptide consisting of an amino acid sequence of SEQ ID NO: 32 in which the anti-CD3 scFv region and the second Fc polypeptide are linked;
(11) a polypeptide consisting of an amino acid sequence of SEQ ID NO: 34 in which the anti-CD3 scFv region and the second Fc polypeptide are linked;
(12) a polypeptide consisting of an amino acid sequence of SEQ ID NO: 36 in which the anti-CD3 scFv region and the second Fc polypeptide are linked;
(13) a polypeptide consisting of an amino acid sequence of SEQ ID NO: 38 in which the anti-CD3 scFv region and the second Fc polypeptide are linked;
(14) a polypeptide consisting of an amino acid sequence of SEQ ID NO: 40 in which the anti-CD3 scFv region and the second Fc polypeptide are linked;
(15) a polypeptide consisting of an amino acid sequence of SEQ ID NO: 42 in which the anti-CD3 scFv region and the second Fc polypeptide are linked; and
(16) a polypeptide consisting of an amino acid sequence of SEQ ID NO: 44 in which the anti-CD3 scFv region and the second Fc polypeptide are linked.

In general, it is known that under conditions in which a bispecific antibody that binds to two antigen molecules is present at a higher concentration than the antigen, a large number of dimers in which the bispecific antibody binds to only one of the antigen molecules are formed, inhibiting the formation of trimers that binds to both antigens. As a result, it is known, for example, that the effect of T-cell cytotoxic activity is attenuated in a high concentration range of a T-cell recruiting antibody that binds to CD3 (AAPS J. 2019, Vol. 21(4), p. 66). In the bispecific T cell recruiting antibody, a decrease in the amount of cytokines produced (e.g., IFN-y and TNF-α) and attenuation of cytotoxic activity in a high antibody concentration range are considered the result of inhibition of the formation of the trimers described above. Such results cannot be obtained when anti-TAA or anti-CD3 antibodies are each independently assayed, and the properties become apparent only after preparation of bispecific T-cell recruiting antibodies. The above properties apply not only to bispecific antibodies, but also to trispecific antibodies and other multispecific antibodies. When an antibody to be developed as a pharmaceutical is selected based on these properties, results obtained by the Redirected T Cell Cytotoxicity (RTCC) assay or cytokine production evaluation described in Example 4 can be used, for example.

The anti-TAA/anti-CD3 multispecific antibody of the present invention may be an anti-TAA/anti-CD3/anti-CD137 trispecific antibody (also referred to as the "anti-TAA/anti-CD3/anti-CD137 trispecific antibody of the present invention") in which an anti-CD137 scFv region comprising the heavy chain variable region and the light chain variable region of the anti-CD137 antibody that binds to CD137 is further linked to the anti-TAA/anti-CD3 bispecific antibody of the present invention. In one embodiment, the anti-TAA/anti-CD3 multispecific antibody of the present invention is an anti-TAA/anti-CD3/anti-CD137 trispecific antibody in which an anti-CD137 scFv region that binds to CD137 is further added to the anti-TAA/anti-CD3 bispecific antibody of the present invention.

In one embodiment, the anti-TAA/anti-CD3/anti-CD137 trispecific antibody of the present invention comprises an anti-CD137 scFV region according to any one of (a) to (e):
(a) an anti-CD137 scFv region comprising a heavy chain variable region comprising a CDR1 consisting of an amino acid sequence at amino acid positions 31 to 35 of SEQ ID NO: 46, a CDR2 consisting of an amino acid sequence at amino acid positions 50 to 66 of SEQ ID NO: 46, and a CDR3 consisting of an amino acid sequence at amino acid positions 99 to 107 of SEQ ID NO: 46; and a light chain variable region comprising a CDR1 consisting of an amino acid sequence at amino acid positions 157 to 167 of SEQ ID NO: 46, a CDR2 consisting of an amino acid sequence at amino acid positions 183 to 189 of SEQ ID NO: 46, and a CDR3 consisting of an amino acid sequence at amino acid positions 222 to 231 of SEQ ID NO: 46;
(b) an anti-CD137 scFv region comprising a heavy chain variable region comprising a CDR1 consisting of an amino acid sequence at amino acid positions 31 to 35 of SEQ ID NO: 48, a CDR2 consisting of an amino acid sequence at amino acid positions 50 to 65 of SEQ ID NO: 48, and a CDR3 consisting of an amino acid sequence at amino acid positions 98 to 107 of SEQ ID NO: 48; and a light chain variable region comprising a CDR1 consisting of an amino acid sequence at amino acid positions 156 to 168 of SEQ ID NO: 48, a CDR2 consisting of an amino acid sequence at amino acid positions 184 to 190 of SEQ ID NO: 48, and a CDR3 consisting of an amino acid sequence at amino acid positions 223 to 233 of SEQ ID NO: 48;
(c) an anti-CD137 scFv region comprising a light chain variable region comprising a CDR1 consisting of an amino acid sequence at amino acid positions 23 to 35 of SEQ ID NO: 50, a CDR2 consisting of an amino acid sequence at amino acid positions 51 to 57 of SEQ ID NO: 50, and a CDR3 consisting of an amino acid sequence at amino acid positions 90 to 100 of SEQ ID NO: 50; and a heavy chain variable region comprising a CDR1 consisting of an amino acid sequence at amino acid positions 162 to 166 of SEQ ID NO: 50, a CDR2 consisting of an amino acid sequence at amino acid positions 181 to 196 of SEQ ID NO: 50, and a CDR3 consisting of an amino acid sequence at amino acid positions 229 to 238 of SEQ ID NO: 50;
(d) an anti-CD137 scFv region comprising a heavy chain variable region comprising a CDR1 consisting of an amino acid sequence at amino acid positions 31 to 35 of SEQ ID NO: 52, a CDR2 consisting of an amino acid sequence at amino acid positions 50 to 65 of SEQ ID NO: 52, and a CDR3 consisting of an amino acid sequence at amino acid positions 98 to 107 of SEQ ID NO: 52; and a light chain variable region comprising a CDR1 consisting of an amino acid sequence at amino acid positions 156 to 168 of SEQ ID NO: 52, a CDR2 consisting of an amino acid sequence at amino acid positions 184 to 190 of SEQ ID NO: 52, and a CDR3 consisting of an amino acid sequence at amino acid positions 223 to 233 of SEQ ID NO: 52; and
(e) an anti-CD137 scFv region comprising a heavy chain variable region comprising a CDR1 consisting of an amino acid sequence at amino acid positions 31 to 35 of SEQ ID NO: 54, a CDR2 consisting of an amino acid sequence at amino acid positions 50 to 65 of SEQ ID NO: 54, and a CDR3 consisting of an amino acid sequence at amino acid positions 98 to 110 of SEQ ID NO: 54; and a light chain variable region comprising a CDR1 consisting of an amino acid sequence at amino acid positions 159 to 171 of SEQ ID NO: 54, a CDR2 consisting of an amino acid sequence at amino acid positions 187 to 193 of SEQ ID NO: 54, and a CDR3 consisting of an amino acid sequence at amino acid positions 226 to 236 of SEQ ID NO: 54.

In one embodiment, the anti-TAA/anti-CD3/anti-CD137 trispecific antibody of the present invention comprises an anti-CD137 scFV region according to any one of (a) to (e):
(a) an anti-CD137 scFv region comprising a heavy chain variable region consisting of an amino acid sequence at amino acid positions 1 to 118 of SEQ ID NO: 46 and a light chain variable region consisting of an amino acid sequence at amino acid positions 134 to 242 of SEQ ID NO: 46;
(b) an anti-CD137 scFv region comprising a heavy chain variable region consisting of an amino acid sequence at amino acid positions 1 to 118 of SEQ ID NO: 48 and a light chain variable region consisting of an amino acid sequence at amino acid positions 134 to 244 of SEQ ID NO: 48;
(c) an anti-CD137 scFv region comprising a light chain variable region consisting of an amino acid sequence at amino acid positions 1 to 111 of SEQ ID NO: 50 and a heavy chain variable region consisting of an amino acid sequence at amino acid positions 132 to 249 of SEQ ID NO: 50;
(d) an anti-CD137 scFv region comprising a heavy chain variable region consisting of an amino acid sequence at amino acid positions 1 to 118 of SEQ ID NO: 52 and a light chain variable region consisting of an amino acid sequence at amino acid positions 134 to 244 of SEQ ID NO: 52; and
(e) an anti-CD137 scFv region comprising a heavy chain variable region consisting of an amino acid sequence at amino acid positions 1 to 121 of SEQ ID NO: 54 and a light chain variable region consisting of an amino acid sequence at amino acid positions 137 to 247 of SEQ ID NO: 54.

In one embodiment, the anti-TAA/anti-CD3/anti-CD137 trispecific antibody of the present invention comprises an anti-CD137 scFV region according to any one of (a) to (e):
(a) an anti-CD137 scFv region consisting of an amino acid sequence at amino acid positions 1 to 242 of SEQ ID NO: 46;
(b) an anti-CD137 scFv region consisting of an amino acid sequence at amino acid positions 1 to 244 of SEQ ID NO: 48;
(c) an anti-CD137 scFv region consisting of an amino acid sequence at amino acid positions 1 to 249 of SEQ ID NO: 50;
(d) an anti-CD137 scFv region consisting of an amino acid sequence at amino acid positions 1 to 244 of SEQ ID NO: 52; and
(e) an anti-CD137 scFv region consisting of an amino acid sequence at amino acid positions 1 to 247 of SEQ ID NO: 54.

In the anti-TAA/anti-CD3/anti-CD137 trispecific antibody, the number of anti-CD137 scFv regions and fusion sites are not particularly limited, and, for example, the anti-CD137 scFv region is linked to the carboxy terminal of the first Fc polypeptide or second Fc polypeptide, or to the amino terminal or carboxy terminal of the light chain of the anti-TAA/anti-CD3 bispecific antibody. In one embodiment, the anti-TAA/anti-CD3/anti-CD137 trispecific antibody of the present invention comprises an anti-CD137 scFv region that is linked to the carboxy terminal of the first Fc polypeptide or the second Fc polypeptide of the anti-TAA/anti-CD3 bispecific antibody. In one embodiment, the anti-TAA/anti-CD3/anti-CD137 trispecific antibody of the present invention comprises an anti-CD 137 scFv region that is linked to the carboxy terminals of the first Fc polypeptide and the second Fc polypeptide of the anti-TAA/anti-CD3 bispecific antibody.

In one embodiment, the anti-TAA/anti-CD3/anti-CD137 multispecific antibody of the present invention is a trispecific antibody having structures according to (a) to (d):
(a) a Fab region of an anti-TAA antibody consisting of a heavy chain fragment comprising a heavy chain variable region of the anti-TAA antibody and a light chain comprising a light chain variable region of the anti-TAA antibody;
(b) an anti-CD3 scFV region comprising a heavy chain variable region and a light chain variable region of an anti-CD3 antibody;
(c) an Fc region consisting of a first Fc polypeptide linked to the heavy chain fragment of the Fab region (a) and a second Fc polypeptide linked to the anti-CD3 scFv region (b); and
(d) an anti-CD 137 scFv region linked to one or both of carboxy terminals of the first Fc polypeptide and the second Fc polypeptide.

In one embodiment, the anti-TAA/anti-CD3/anti-CD137 trispecific antibody of the present invention is an anti-TAA/anti-CD3/anti-CD 137 trispecific antibody comprising a heavy chain of an anti-TAA antibody in which a first Fc polypeptide is linked to a heavy chain fragment comprising a heavy chain variable region of the anti-TAA antibody; a light chain comprising a light chain variable region of the anti-TAA antibody; and a polypeptide in which an anti-CD3 scFv region comprising a heavy chain variable region and a light chain variable region of an anti-CD3 antibody that binds to CD3 and a second Fc polypeptide are linked, wherein an anti-CD137 scFv region comprising a heavy chain variable region and a light chain variable region of an anti-CD137 antibody is also linked to one or both of carboxy terminals of the first Fc polypeptide and the second Fc polypeptide.

In one embodiment, the anti-TAA/anti-CD3/anti-CD137 trispecific antibody of the present invention is an anti-TAA/anti-CD3/anti-CD 137 trispecific antibody comprising a heavy chain in which a first Fc polypeptide is linked to a heavy chain fragment comprising a heavy chain variable region of the anti-TAA antibody; a light chain comprising a light chain variable region of the anti-TAA antibody; and a polypeptide in which an anti-CD3 scFv region comprising a heavy chain variable region and a light chain variable region of an anti-CD3 antibody that binds to CD3 and a second Fc polypeptide are linked, wherein an anti-CD137 scFv region comprising a heavy chain variable region and a light chain variable region of an anti-CD137 antibody is also linked to one or both of carboxy terminals of the first Fc polypeptide and the second Fc polypeptide,
wherein the anti-CD3 scFv region is any one of (1) to (16):
   (1) an anti-CD3 scFv region consisting of an amino acid sequence at amino acid positions 1 to 254 of SEQ ID NO: 14;
   (2) an anti-CD3 scFv region consisting of an amino acid sequence at amino acid positions 1 to 254 of SEQ ID NO: 16;
   (3) an anti-CD3 scFv region consisting of an amino acid sequence at amino acid positions 1 to 254 of SEQ ID NO: 18;
   (4) an anti-CD3 scFv region consisting of an amino acid sequence at amino acid positions 1 to 254 of SEQ ID NO: 20;
   (5) an anti-CD3 scFv region consisting of an amino acid sequence at amino acid positions 1 to 254 of SEQ ID NO: 22;
   (6) an anti-CD3 scFv region consisting of an amino acid sequence at amino acid positions 1 to 254 of SEQ ID NO: 24;
   (7) an anti-CD3 scFv region consisting of an amino acid sequence at amino acid positions 1 to 254 of SEQ ID NO: 26;
   (8) an anti-CD3 scFv region consisting of an amino acid sequence at amino acid positions 1 to 254 of SEQ ID NO: 28;
   (9) an anti-CD3 scFv region consisting of an amino acid sequence at amino acid positions 1 to 254 of SEQ ID NO: 30;
   (10) an anti-CD3 scFv region consisting of an amino acid sequence at amino acid positions 1 to 254 of SEQ ID NO: 32;
   (11) an anti-CD3 scFv region consisting of an amino acid sequence at amino acid positions 1 to 254 of SEQ ID NO: 34;
   (12) an anti-CD3 scFv region consisting of an amino acid sequence at amino acid positions 1 to 254 of SEQ ID NO: 36;
   (13) an anti-CD3 scFv region consisting of an amino acid sequence at amino acid positions 1 to 254 of SEQ ID NO: 38;
   (14) an anti-CD3 scFv region consisting of an amino acid sequence at amino acid positions 1 to 254 of SEQ ID NO: 40;
   (15) an anti-CD3 scFv region consisting of an amino acid sequence at amino acid positions 1 to 254 of SEQ ID NO: 42; and
   (16) an anti-CD3 scFv region consisting of an amino acid sequence at amino acid positions 1 to 254 of SEQ ID NO: 44, and
wherein the anti-CD137 scFv region is any one of (a) to (e):
   (a) an anti-CD137 scFv region consisting of an amino acid sequence at amino acid positions 1 to 242 of SEQ ID NO: 46;
   (b) an anti-CD137 scFv region consisting of an amino acid sequence at amino acid positions 1 to 244 of SEQ ID NO: 48;
   (c) an anti-CD137 scFv region consisting of an amino acid sequence at amino acid positions 1 to 249 of SEQ ID NO: 50;
   (d) an anti-CD137 scFv region consisting of an amino acid sequence at amino acid positions 1 to 244 of SEQ ID NO: 52; and
   (e) an anti-CD137 scFv region consisting of an amino acid sequence at amino acid positions 1 to 247 of SEQ ID NO: 54.

The anti-TAA antibody to be used in the anti-TAA/anti-CD3 multispecific antibody of the present invention is not particularly limited as long as it comprises a heavy chain variable region and a light chain variable region that bind to TAA expressed on the surface of human cancer cells. The TAA is desirably an antigen that is expressed only in cancer cells or is overexpressed in cancer cells.

In one embodiment, the anti-TAA antibody to be used in the anti-TAA/anti-CD3 multispecific antibody of the present invention is an anti-TAA antibody that binds to any one of TAAs:
HER2, HER3, HER4, CD19, CD20, CD22, CD33, CD38, CD40, CD44, CD70, CD123, CD138, CXCR3, CXCR5, CCR3, CCR4, CCR9, CRTH2, PMCH, CD4, CD25, CD200, endoplasmin, BCMA, CD276, TSPAN8, CLDN4, CLDN6, CLDN18.2, ENPP3, SLC34A2, TROP2, MerTK, Nectin-4, ASGR1, mesothelin, PSMA, LIV-1, MUC1, MUC2, MUC4, MUC16, CDH6, CEACAM1, CEACAM3, CEACAM4, CEACAM5, CEACAM6, CEACAM7, CEACAM16, CEACAM18, CEACAM19, CEACAM20, CEACAM21, URLC10, NY-ESO-1, GAA, OFA, cyclinB1, WT-1, CEF, VEGRR1, VEGFR2, TTK, HPV16, HPV16E7, HPV18, CEAIMA910, KOC1, SL-701, MART-1, gp100, tyrosinase, survivin, MAGE-3.1, MAGE-10.A2, OVA BiP, gp209-2M, melan-A, NA17.A2, KOC1, CO16, DEPDC1, MPHOSPH1, MAGE12, ONT-10, GD2L, GD3L, URLC10, CDCA1, TF, PSA, TERT, STF-II, G17DT, ICT-107, Dex2, hTERT, PAP, TRP2, LRRC15, c-Met, PD-L1, FAP, EGFR, B3-H3, GPC2, GPC3, 5T4

In one embodiment, the anti-TAA/anti-CD3 multispecific antibody of the present invention may be post-translationally modified. In one embodiment, the post-translational modification is pyroglutamylation at the N-terminal of the heavy chain variable region and/or lysine deletion at the heavy chain C-terminal. It is known in technical field that the post-translational modification by pyroglutamylation at the N-terminal or lysine deletion at the C-terminal does not affect the activity of the antibody (Analytical Biochemistry, 2006, Vol. 348, p. 24-39).

### <Polynucleotide of Anti-TAA/Anti-CD3 Multispecific Antibody of Invention>

The present invention provides a polynucleotide for use in the production of the anti-TAA/anti-CD3 multispecific antibody of the present invention (also referred to as the "polynucleotide of the anti-TAA/anti-CD3 multispecific antibody of the present invention"). In one embodiment, the polynucleotide for use in the production of the anti-TAA/anti-CD3 multispecific antibody of the present invention is a polynucleotide selected from the group consisting of (1) to (16):
(1) a polynucleotide having a nucleotide sequence encoding an anti-CD3 scFv region consisting of an amino acid sequence at amino acid positions 1 to 254 of SEQ ID NO: 14;
(2) a polynucleotide having a nucleotide sequence encoding an anti-CD3 scFv region consisting of an amino acid sequence at amino acid positions 1 to 254 of SEQ ID NO: 16;
(3) a polynucleotide having a nucleotide sequence encoding an anti-CD3 scFv region consisting of an amino acid sequence at amino acid positions 1 to 254 of SEQ ID NO: 18;
(4) a polynucleotide having a nucleotide sequence encoding an anti-CD3 scFv region consisting of an amino acid sequence at amino acid positions 1 to 254 of SEQ ID NO: 20;
(5) a polynucleotide having a nucleotide sequence encoding an anti-CD3 scFv region consisting of an amino acid sequence at amino acid positions 1 to 254 of SEQ ID NO: 22;
(6) a polynucleotide having a nucleotide sequence encoding an anti-CD3 scFv region consisting of an amino acid sequence at amino acid positions 1 to 254 of SEQ ID NO: 24;
(7) a polynucleotide having a nucleotide sequence encoding an anti-CD3 scFv region consisting of an amino acid sequence at amino acid positions 1 to 254 of SEQ ID NO: 26;
(8) a polynucleotide having a nucleotide sequence encoding an anti-CD3 scFv region consisting of an amino acid sequence at amino acid positions 1 to 254 of SEQ ID NO: 28;
(9) a polynucleotide having a nucleotide sequence encoding an anti-CD3 scFv region consisting of an amino acid sequence at amino acid positions 1 to 254 of SEQ ID NO: 30;
(10) a polynucleotide having a nucleotide sequence encoding an anti-CD3 scFv region consisting of an amino acid sequence at amino acid positions 1 to 254 of SEQ ID NO: 32;
(11) a polynucleotide having a nucleotide sequence encoding an anti-CD3 scFv region consisting of an amino acid sequence at amino acid positions 1 to 254 of SEQ ID NO: 34;
(12) a polynucleotide having a nucleotide sequence encoding an anti-CD3 scFv region consisting of an amino acid sequence at amino acid positions 1 to 254 of SEQ ID NO: 36;
(13) a polynucleotide having a nucleotide sequence encoding an anti-CD3 scFv region consisting of an amino acid sequence at amino acid positions 1 to 254 of SEQ ID NO: 38;
(14) a polynucleotide having a nucleotide sequence encoding an anti-CD3 scFv region consisting of an amino acid sequence at amino acid positions 1 to 254 of SEQ ID NO: 40;
(15) a polynucleotide having a nucleotide sequence encoding an anti-CD3 scFv region consisting of an amino acid sequence at amino acid positions 1 to 254 of SEQ ID NO: 42; and
(16) a polynucleotide having a nucleotide sequence encoding an anti-CD3 scFv region consisting of an amino acid sequence at amino acid positions 1 to 254 of SEQ ID NO: 44.

In one embodiment, the polynucleotide for use in the production of the anti-TAA/anti-CD3/anti-CD137 trispecific antibody of the present invention is a polynucleotide encoding an anti-CD137 scFv region selected from the group consisting of (a) to (e):
(a) a polynucleotide having a nucleotide sequence encoding an anti-CD 137 scFv region consisting of an amino acid sequence at amino acid positions 1 to 242 of SEQ ID NO: 46;
(b) a polynucleotide having a nucleotide sequence encoding an anti-CD 137 scFv region consisting of an amino acid sequence at amino acid positions 1 to 244 of SEQ ID NO: 48;
(c) a polynucleotide having a nucleotide sequence encoding an anti-CD137 scFv region consisting of an amino acid sequence at amino acid positions 1 to 249 of SEQ ID NO: 50;
(d) a polynucleotide having a nucleotide sequence encoding an anti-CD137 scFv region consisting of an amino acid sequence at amino acid positions 1 to 244 of SEQ ID NO: 52; and
(e) a polynucleotide having a nucleotide sequence encoding an anti-CD 137 scFv region consisting of an amino acid sequence at amino acid positions 1 to 247 of SEQ ID NO: 54.

The polynucleotide of the anti-TAA/anti-CD3 multispecific antibody of the present invention can be easily produced by those skilled in the art with reference to the section of <Polynucleotide of Anti-CD37/Anti-CD3 Bispecific Antibody of Invention>.

### <Expression Vector for Anti-TAA/Anti-CD3 Multispecific Antibody of Invention>

The present invention also provides an expression vector comprising polynucleotides encoding the anti-TAA/anti-CD3 multispecific antibody of the present invention (also referred to as the "expression vector of the anti-TAA/anti-CD3 multispecific antibody of the present invention"). The expression vector can be easily produced by those skilled in the art with reference to the section of <Expression Vector of Anti-CD37/Anti-CD3 Bispecific Antibody of Invention>. The polynucleotides of the anti-TAA/anti-CD3 multispecific antibody of the present invention may be each contained in different vectors, or a plurality of the polynucleotides may be contained in one vector.

### <Host Cell of Anti-TAA/Anti-CD3 Multispecific Antibody of Invention>

The present invention also provides a host cell comprising a polynucleotide encoding the anti-TAA/anti-CD3 multispecific antibody of the present invention (also referred to as the "host cell of the anti-TAA/anti-CD3 multispecific antibody of the present invention"). The host cell comprising a polynucleotide encoding the anti-TAA/anti-CD3 multispecific antibody of the present invention can be easily produced by those skilled in the art with reference to the section of <Host Cell of Anti-CD37/Anti-CD3 Bispecific Antibody of Invention>.

### <Production Method of Anti-TAA/Anti-CD3 Multispecific Antibody of Invention>

The present invention also provides a method for producing the anti-TAA/anti-CD3 multispecific antibody of the present invention (also referred to as the "production method of the anti-TAA/anti-CD3 multispecific antibody of the present invention"). The anti-TAA/anti-CD3 multispecific antibody of the present invention can be easily produced by those skilled in the art with reference to the section of <Production Method of Anti-CD37/Anti-CD3 Bispecific Antibody of Invention>.

### <Pharmaceutical Composition of Anti-TAA/Anti-CD3 Multispecific Antibody of Invention>

The present invention also provides a pharmaceutical composition comprising the anti-TAA/anti-CD3 multispecific antibody of the present invention and a pharmaceutically acceptable excipient (also referred to as the "pharmaceutical composition of the anti-TAA/anti-CD3 multispecific antibody of the present invention"). Examples of the dosage form, excipient, carrier, additive, and the like in the pharmaceutical composition of the anti-TAA/anti-CD3 multispecific antibody of the present invention include those described in the section of <Pharmaceutical Composition or the like of Anti-CD37/Anti-CD3 Bispecific Antibody of Invention>. The amounts of the anti-TAA/anti-CD3 multispecific antibody of the present invention added in formulation are varied depending on the degree of symptom and the age of a patient, a dosage form of the formulation to be used, a binding titer of the antibody, or the like, and for example, can be approximately 0.001 mg/kg to 100 mg/kg. The pharmaceutical composition of the anti-TAA/anti-CD3 multispecific antibody of the present invention can be used for treating cancer of a subject. In one embodiment, the pharmaceutical composition of the anti-TAA/anti-CD3 multispecific antibody of the present invention is used for treating cancer of a subject. The pharmaceutical composition of the anti-TAA/anti-CD3 multispecific antibody of the present invention can be easily prepared by those skilled in the art with reference to the section of <Anti-CD37/Anti-CD3 Bispecific Antibody of Invention>. For example, a pharmaceutical composition comprising an antibody or the like which has undergone both of or one of lysine deletion at the C-terminal and pyroglutamylation at the N-terminal can be embraced in the present invention.

### <Pharmaceutical Use of Anti-TAA/Anti-CD3 Multispecific Antibody of Invention>

The anti-TAA/anti-CD3 multispecific antibody of the present invention and a pharmaceutical composition comprising the same can be used for treating cancer. Furthermore, the present invention comprises a method for treating cancer, the method comprising administering, to a subject, a therapeutically effective amount of the anti-TAA/anti-CD3 bispecific antibody of the present invention. Moreover, the present invention comprises the anti-TAA/anti-CD3 multispecific antibody for treating cancer. In addition, the present invention comprises use of the anti-TAA/anti-CD3 multispecific antibody of the present invention in production of a pharmaceutical composition for treating cancer. The present invention further provides a method for treating cancer, the method comprising administering, to a subject, a therapeutically effective amount of the anti-TAA/anti-CD3 multispecific antibody of the present invention or the pharmaceutical composition of the anti-TAA/anti-CD3 multispecific antibody of the present invention; an anti-TAA/anti-CD3 multispecific antibody for treating cancer; and use of the anti-TAA/anti-CD3 multispecific antibody of the present invention in production of a pharmaceutical composition for treating cancer (all of which are together referred to as the "pharmaceutical use of the anti-TAA/anti-CD3 multispecific antibody of the present invention"). Examples of the cancer to be treated in the pharmaceutical use of the anti-TAA/anti-CD37 antibody and the like of the present invention include cancers described in the section of <Pharmaceutical Composition of Anti-CD37/Anti-CD3 Bispecific Antibody of Invention>. The pharmaceutical use of the anti-TAA/anti-CD3 multispecific antibody of the present invention can be performed by those skilled in the art with reference to the section of <Pharmaceutical Use of Anti-CD37/Anti-CD3 Bispecific Antibody of Invention>.

### <Anti-CD3 scFv of Invention>

The present invention further provides anti-CD3 scFv according to any one of (1) to (16):
(1) anti-CD3 scFv comprising a heavy chain variable region of an anti-CD3 antibody consisting of an amino acid sequence at amino acid positions 1 to 125 of SEQ ID NO: 14 and a light chain variable region of an anti-CD3 antibody consisting of an amino acid sequence at amino acid positions 146 to 254 of SEQ ID NO: 14;
(2) anti-CD3 scFv comprising a heavy chain variable region of an anti-CD3 antibody consisting of an amino acid sequence at amino acid positions 1 to 125 of SEQ ID NO: 16 and a light chain variable region of an anti-CD3 antibody consisting of an amino acid sequence at amino acid positions 146 to 254 of SEQ ID NO: 16;
(3) anti-CD3 scFv comprising a heavy chain variable region of an anti-CD3 antibody consisting of an amino acid sequence at amino acid positions 1 to 125 of SEQ ID NO: 18 and a light chain variable region of an anti-CD3 antibody consisting of an amino acid sequence at amino acid positions 146 to 254 of SEQ ID NO: 18;
(4) anti-CD3 scFv comprising a heavy chain variable region of an anti-CD3 antibody consisting of an amino acid sequence at amino acid positions 1 to 125 of SEQ ID NO: 20 and a light chain variable region of an anti-CD3 antibody consisting of an amino acid sequence at amino acid positions 146 to 254 of SEQ ID NO: 20;
(5) anti-CD3 scFv comprising a heavy chain variable region of an anti-CD3 antibody consisting of an amino acid sequence at amino acid positions 1 to 125 of SEQ ID NO: 22 and a light chain variable region of an anti-CD3 antibody consisting of an amino acid sequence at amino acid positions 146 to 254 of SEQ ID NO: 22;
(6) anti-CD3 scFv comprising a heavy chain variable region of an anti-CD3 antibody consisting of an amino acid sequence at amino acid positions 1 to 125 of SEQ ID NO: 24 and a light chain variable region of an anti-CD3 antibody consisting of an amino acid sequence at amino acid positions 146 to 254 of SEQ ID NO: 24;
(7) anti-CD3 scFv comprising a heavy chain variable region of an anti-CD3 antibody consisting of an amino acid sequence at amino acid positions 1 to 125 of SEQ ID NO: 26 and a light chain variable region of an anti-CD3 antibody consisting of an amino acid sequence at amino acid positions 146 to 254 of SEQ ID NO: 26;
(8) anti-CD3 scFv comprising a heavy chain variable region of an anti-CD3 antibody consisting of an amino acid sequence at amino acid positions 1 to 125 of SEQ ID NO: 28 and a light chain variable region of an anti-CD3 antibody consisting of an amino acid sequence at amino acid positions 146 to 254 of SEQ ID NO: 28;
(9) anti-CD3 scFv comprising a heavy chain variable region of an anti-CD3 antibody consisting of an amino acid sequence at amino acid positions 1 to 125 of SEQ ID NO: 30 and a light chain variable region of an anti-CD3 antibody consisting of an amino acid sequence at amino acid positions 146 to 254 of SEQ ID NO: 30;
(10) anti-CD3 scFv comprising a heavy chain variable region of an anti-CD3 antibody consisting of an amino acid sequence at amino acid positions 1 to 125 of SEQ ID NO: 32 and a light chain variable region of an anti-CD3 antibody consisting of an amino acid sequence at amino acid positions 146 to 254 of SEQ ID NO: 32;
(11) anti-CD3 scFv comprising a heavy chain variable region of an anti-CD3 antibody consisting of an amino acid sequence at amino acid positions 1 to 125 of SEQ ID NO: 34 and a light chain variable region of an anti-CD3 antibody consisting of an amino acid sequence at amino acid positions 146 to 254 of SEQ ID NO: 34;
(12) anti-CD3 scFv comprising a heavy chain variable region of an anti-CD3 antibody consisting of an amino acid sequence at amino acid positions 1 to 125 of SEQ ID NO: 36 and a light chain variable region of an anti-CD3 antibody consisting of an amino acid sequence at amino acid positions 146 to 254 of SEQ ID NO: 36;
(13) anti-CD3 scFv comprising a heavy chain variable region of an anti-CD3 antibody consisting of an amino acid sequence at amino acid positions 1 to 125 of SEQ ID NO: 38 and a light chain variable region of an anti-CD3 antibody consisting of an amino acid sequence at amino acid positions 146 to 254 of SEQ ID NO: 38;
(14) anti-CD3 scFv comprising a heavy chain variable region of an anti-CD3 antibody consisting of an amino acid sequence at amino acid positions 1 to 125 of SEQ ID NO: 40 and a light chain variable region of an anti-CD3 antibody consisting of an amino acid sequence at amino acid positions 146 to 254 of SEQ ID NO: 40;
(15) anti-CD3 scFv comprising a heavy chain variable region of an anti-CD3 antibody consisting of an amino acid sequence at amino acid positions 1 to 125 of SEQ ID NO: 42 and a light chain variable region of an anti-CD3 antibody consisting of an amino acid sequence at amino acid positions 146 to 254 of SEQ ID NO: 42; and
(16) anti-CD3 scFv comprising a heavy chain variable region of an anti-CD3 antibody consisting of an amino acid sequence at amino acid positions 1 to 125 of SEQ ID NO: 44 and a light chain variable region of an anti-CD3 antibody consisting of an amino acid sequence at amino acid positions 146 to 254 of SEQ ID NO: 44.

In one embodiment, the anti-CD3 scFv of the present invention is any one of (1) to (16):
(1) anti-CD3 scFv consisting of an amino acid sequence at amino acid positions 1 to 254 of SEQ ID NO: 14;
(2) anti-CD3 scFv consisting of an amino acid sequence at amino acid positions 1 to 254 of SEQ ID NO: 16;
(3) anti-CD3 scFv consisting of an amino acid sequence at amino acid positions 1 to 254 of SEQ ID NO: 18;
(4) anti-CD3 scFv consisting of an amino acid sequence at amino acid positions 1 to 254 of SEQ ID NO: 20;
(5) anti-CD3 scFv consisting of an amino acid sequence at amino acid positions 1 to 254 of SEQ ID NO: 22;
(6) anti-CD3 scFv consisting of an amino acid sequence at amino acid positions 1 to 254 of SEQ ID NO: 24;
(7) anti-CD3 scFv consisting of an amino acid sequence at amino acid positions 1 to 254 of SEQ ID NO: 26;
(8) anti-CD3 scFv consisting of an amino acid sequence at amino acid positions 1 to 254 of SEQ ID NO: 28;
(9) anti-CD3 scFv consisting of an amino acid sequence at amino acid positions 1 to 254 of SEQ ID NO: 30;
(10) anti-CD3 scFv consisting of an amino acid sequence at amino acid positions 1 to 254 of SEQ ID NO: 32;
(11) anti-CD3 scFv consisting of an amino acid sequence at amino acid positions 1 to 254 of SEQ ID NO: 34;
(12) anti-CD3 scFv consisting of an amino acid sequence at amino acid positions 1 to 254 of SEQ ID NO: 36;
(13) anti-CD3 scFv consisting of an amino acid sequence at amino acid positions 1 to 254 of SEQ ID NO: 38;
(14) anti-CD3 scFv consisting of an amino acid sequence at amino acid positions 1 to 254 of SEQ ID NO: 40;
(15) anti-CD3 scFv consisting of an amino acid sequence at amino acid positions 1 to 254 of SEQ ID NO: 42; and
(16) anti-CD3 scFv consisting of an amino acid sequence at amino acid positions 1 to 254 of SEQ ID NO: 44.

The present invention also provides a polynucleotide encoding the anti-CD3 scFv described above, an expression vector comprising the polynucleotide, and a host cell comprising the polynucleotide or the expression vector.

The anti-CD3 scFv of the present invention is also available as an anti-TAA/anti-CD3 multispecific antibody comprising the anti-TAA antibody that binds to TAA and the anti-CD3 scFv region of the present invention. Furthermore, the anti-CD3 scFv of the present invention provides cells, viral vectors, or plasmid vectors in which the anti-CD3 scFv is expressed on the cell surface. Such cells, viral vectors, or plasmid vectors can be produced by those skilled in the art using the anti-CD3 scFv of the present invention or a polynucleotide encoding the anti-CD3 scFv.

Specific examples, which are referred to for gaining further understanding of the present invention, are provided herein for illustrative purposes only, and these examples are not intended to limit the present invention.

### EXAMPLES

Currently, various bispecific T cell recruiting antibodies targeting TAA are being developed as therapeutic agents for cancer. The present applicant has succeeded in obtaining an anti-TSPAN8/anti-CD3 bispecific antibody using an anti-TSPAN8 antibody that selectively recognizes TSPAN8 expressed in a cancer cell (WO 2022/102768) as well and has been studying the clinical application of the bispecific antibody. Furthermore, for the purpose of producing a drug discovery platform for the development of an anti-TAA/anti-CD3 bispecific antibody, which is a bispecific antibody that binds to any TAA and CD3, the present applicant has decided to conduct an improvement study of the anti-CD3 scFv obtained in the examination for preparation of the anti-TSPAN8-anti-CD3 bispecific antibody.

The bioactivity of bispecific T-cell recruiting antibodies is known to be affected by various factors, such as formats of the antibodies, expression levels of targeted TAAs, epitopes recognized by the antibodies, affinities to TAA and CD3, and *in vivo* kinetics. From the viewpoint of efficacy and safety, the present inventors considered that the optimal affinity of anti-CD3 scFv for the CD3 would vary depending on the expression level of the TAA to be combined and the affinity of an anti-TAA antibody for the TAA, and therefore conducted the following studies under the assumption that a bispecific T-cell recruiting antibody with an optimal balance of affinity could be obtained by preparing multiple anti-CD3 scFvs having diverse affinity and searching for an optimal combination of any anti-TAA antibody and anti-CD3 scFv.

### [Example 1: Obtaining Anti-TSPAN8/Anti-CD3 Bispecific Antibody Having Diverse CD3 Binding Affinity]

Based on the information about an anti-TSPAN8/anti-CD3 bispecific antibody composed of polypeptides consisting of the amino acid sequences set forth in SEQ ID NOs: 2, 4, and 6, the following studies were conducted to prepare an anti-CD3 scFv group having diverse affinity for CD3.

The heavy chain of the anti-TSPAN8 antibody consisting of SEQ ID NO: 2 and the polypeptide in which the anti-CD3 scFv region consisting of SEQ ID NO: 6 and the second Fc polypeptide are linked. This construct contains LALA mutation, N297G mutation, and knobs-into-holes mutation. The LALA mutation refers to the amino acid mutation of L234A and L235A of human Igyl constant region. The N297G mutation is replacement of asparagine with glycine at amino acid position 297 in human Igyl constant region according to EU index (Protein cell, 2018, Vol. 9, p. 63-73). The knobs-into-holes mutation is T366W mutation in one Fc polypeptide (corresponding to the amino acid sequence at amino acid positions 255 to 486 of SEQ ID NO: 6) forming the Fc region, and T366S, L368A, and Y407V mutations in another Fc polypeptide (corresponding to the amino acid sequence at amino acid positions 220 to 451 of SEQ ID NO: 2) forming the Fc region. The positions of the mutations are amino acid positions in human Igγ1 constant region according to EU index.

### [Example 1-1: Sequence Design of Anti-CD3 scFv Having Diverse CD3 Binding Affinity]

To estimate amino acid residues that may affect CD3 binding affinity in the anti-CD3 scFv of SEQ ID NO: 6, analysis was conducted using (1) public information, (2) structural information on the antibody, (3) amino acid frequency information at each amino acid residue position, and (4) human Germline sequence information. Methods are described in detail below. As a result, 66 variants listed in Table 1 were designed.
(1) As public information, examples of modification of the amino acid sequence of anti-CD3 scFv described in PTLs 1 to 4 were used as reference. PTL 1 describes anti-CD3 scFv derived from mice. In PTLs 2 to 4, the PTL 1-derived anti-CD3 scFv is humanized and the CD3 binding affinity is modified.
(2) The structural information on the antibodies was taken from 5FCS (DOI: 10.2210/pdb5FCS/pdb), registered in PDB (Protein Data Bank; https://www.rcsb.org) as reference. The 5FCS contained 3D structural information on a diabody having a high amino acid identity (95% identity for heavy chain variable region and 93% identity for light chain variable region) with the amino acid sequence of the anti-CD3 scFv contained in SEQ ID NO: 6, which was utilized as information for adjusting the affinity. MOE, an integrated computational chemistry system provided by Chemical Computing Group, was used to observe the structural information.
(3) Amino acid frequency information at each amino acid residue position was obtained with reference to abYsis (http://www.abysis.org/abysis/). It has been known that the amino acid sequence of an antibody variable region has a tendency for amino acids to appear depending on the position of the residue. For example, in human antibodies, glutamine or glutamic acid is found to overwhelmingly occur as the first amino acid in the heavy chain variable region. In other words, an antibody having an amino acid that deviates from this tendency of occurrence frequency is expected to be physically unstable because the sequence of the antibody is unnatural. Therefore, this information was determined to be useful as information for designing a sable antibody.
(4) Human Germline sequence information was obtained with reference to IMGT (http://www.IMGT.org/). Historically, humanized antibodies have been successful in reducing antigenic risk in clinical practice. To keep antigenic risk low in the clinical practice, variants were designed to be as close as possible to the human Germline sequence.

Based on the information of (1) to (4) above, the following analysis and discussion were conducted. Specifically, for example, PTL 4 discloses that replacement of the 58th (H58) amino acid in the Kabat Numbering of the heavy chain variable region, tyrosine (Tyr), with alanine (Ala) causes a decrease in the binding activity of anti-CD3 scFv to CD3. Since the amino acid of H58 in SEQ ID NO: 6 is also Tyr, replacement with another amino acid was assumed to change the binding activity. However, since the mechanism of the decrease in binding activity was unclear, the structural information on the 5FCS registered in the PDB was used for further discussion. When the structural information on 5FCS was observed using MOE, it was inferred that Tyr of H58 was not directly involved in binding to an antigen, but played a major role in maintaining the structure of CDR-H2 (according to the definition of Kabat Numbering) through hydrophobic interaction. Since CDRs have been generally known to play a very important role in binding to an antigen, the Tyr of H58 was considered to maintain the binding ability to CD3 by supporting the structure of CDR-H2. In other words, since it is inferred that the H58 is not directly involved in antigen binding, replacement with another amino acid would be less likely to result in complete loss of activity. Candidate amino acids to be substituted were selected with reference to the amino acid frequency information in H58. The results revealed that aspartic acid (Asp), isoleucine (Ile), lysine (Lys), asparagine (Asn), arginine (Arg), serine (Ser), threonine (Thr), and the like could be candidates for the amino acid to be substituted. Thus, stable modified antibodies could be created by replacing H58 with these amino acids. Finally, confirmation of the human Germline sequence closely related to SEQ ID NO: 6 revealed that H58 of IGHV3-72*01 (IMGT Accession No. X92206) was glutamic acid (Glu), and H58 of IGHV3-73*01 (IMGT Accession No. X70197) was Ala. Therefore, the replacement of H58 with Glu or Ala would reduce the risk of high antigenicity in clinical practice. In comprehensive consideration of the above, it was decided that the candidate amino acid for substitution in the H58 would be any one of Asp, Ile, Lys, Asn, Arg, Ser, Thr, Glu, and Ala.

In the same manner as described above, amino acids to be substituted were determined for each of the amino acids H30 to 31, H30 to 32, H31, H35, H52C, H52C&54, H58, H73, H100A, H101, H49, H100E, L89, L89&91, L91, L96, and L36. Eventually, sequences of the 66 variants were designed (Table 1).

### [Example 1-2: Obtaining Anti-TSPAN8/Anti-CD3 scFv Bispecific Antibody Group Having Diverse CD3 Binding Affinity] The 66 variants designed in Example 1-1 were obtained.

DNA encoding a polypeptide in which the anti-CD3 scFv region set forth in SEQ ID NO: 5 and the second Fc polypeptide were linked was synthesized according to a conventional method and inserted into a pcDNA3.4 TOPO vector (A14697, available from Thermo Fisher Scientific) (hereinafter, the polypeptide in which the anti-CD3 scFv region and the second Fc polypeptide were linked is referred to as "anti-CD3 scFv-Fc"). The thus produced vector is designated as ApiD00000. DNA encoding the amino acid sequences of 66 variants was synthesized by a conventional method and inserted into a pcDNA 3.4 TOPO vector. The thus produced vectors are designated as ApiD00001 to ApiD00066.
Similarly, DNA was synthesized based on the nucleotide sequences (SEQ ID NO: 1 and SEQ ID NO: 3) encoding the anti-TSPAN8 antibody to be inserted into a pcDNA3.4 TOPO vector. The thus prepared vectors are designated as ApiD00067 and ApiD00068, respectively.

To obtain anti-TSPAN8/anti-CD3 bispecific antibodies containing anti-CD3 scFv before modification, ExpiCHO-S (R) cells (A29127, available from Thermo Fisher Scientific) were also transfected with ApiD00067, ApiD00068, and ApiD00000 encoding anti-CD3 scFv. According to a conventional method, culture supernatants were purified using MabSelect SuRe (17-5438-02, available from GE Healthcare) to obtain purified antibodies. The resulting anti-TSPAN8/anti-CD3 bispecific antibody is designated as BTE-0001. To obtain anti-TSPAN8/anti-CD3 bispecific antibodies containing 66 anti-CD3 scFv variants, ExpiCHO-S (R) cells were also transfected with ApiD00067, ApiD00068, and any one of ApiD00001 to ApiD00066 encoding anti-CD3 scFv. According to a conventional method, culture supernatants were purified using MabSelect SuRe to obtain 66 purified antibodies. The resulting anti-TSPAN8/anti-CD3 bispecific antibodies are designated as BTE-0002 to BTE-0067. The names of the vectors used for the preparation of the anti-TSPAN8/anti-CD3 bispecific antibodies and information on the modification sites and substituted amino acids of the anti-CD3 scFv are shown in Table 1.

**[Table 1-1]**

| Antibody name | Heavy chain (anti-TSPAN8) | Light chain (anti-TSPAN8) | Anti-CD3 scFv-Fc (anti-CD3) | Mutation of anti-CD3 variable region (amino acid before mutation_Kabat number_amino acid after mutation) |
|---|---|---|---|---|
| BTE-0001 | ApiD00067 | ApiD00068 | ApiD00000 | |
| BTE-0002 | ApiD00067 | ApiD00068 | ApiD00001 | T_H31_I |
| BTE-0003 | ApiD00067 | ApiD00068 | ApiD00002 | T_H31_G |
| BTE-0004 | ApiD00067 | ApiD00068 | ApiD00003 | T_H31_S |
| BTE-0005 | ApiD00067 | ApiD00068 | ApiD00004 | N_H30_S, T_H31_G, Y_H32_S |
| BTE-0006 | ApiD00067 | ApiD00068 | ApiD00005 | N_H30_S, T_H31_D, Y_H32_H |
| BTE-0007 | ApiD00067 | ApiD00068 | ApiD00006 | N_H30_S, T_H31_S |
| BTE-0008 | ApiD00067 | ApiD00068 | ApiD00007 | N_H35_S |
| BTE-0009 | ApiD00067 | ApiD00068 | ApiD00008 | N_H35_H |
| BTE-0010 | ApiD00067 | ApiD00068 | ApiD00009 | N_H35_D |
| BTE-0011 | ApiD00067 | ApiD00068 | ApiD00010 | N_H35_A |
| BTE-0012 | ApiD00067 | ApiD00068 | ApiD00011 | N_H35_G |
| BTE-0013 | ApiD00067 | ApiD00068 | ApiD00012 | N_H35_W |
| BTE-0014 | ApiD00067 | ApiD00068 | ApiD00013 | N_H35_Y |
| BTE-0015 | ApiD00067 | ApiD00068 | ApiD00014 | Y_H52C_A |
| BTE-0016 | ApiD00067 | ApiD00068 | ApiD00015 | Y_H52C_F |
| BTE-0017 | ApiD00067 | ApiD00068 | ApiD00016 | Y_H52C_A, N_H54_S |
| BTE-0018 | ApiD00067 | ApiD00068 | ApiD00017 | Y_H58_A |
| BTE-0019 | ApiD00067 | ApiD00068 | ApiD00018 | Y_H58_E |
| BTE-0020 | ApiD00067 | ApiD00068 | ApiD00019 | Y_H58_D |
| BTE-0021 | ApiD00067 | ApiD00068 | ApiD00020 | Y_H58_I |
| BTE-0022 | ApiD00067 | ApiD00068 | ApiD00021 | Y_H58_K |
| BTE-0023 | ApiD00067 | ApiD00068 | ApiD00022 | Y_H58_N |
| BTE-0024 | ApiD00067 | ApiD00068 | ApiD00023 | Y_H58_R |
| BTE-0025 | ApiD00067 | ApiD00068 | ApiD00024 | Y_H58_S |
| BTE-0026 | ApiD00067 | ApiD00068 | ApiD00025 | Y_H58_T |
| BTE-0027 | ApiD00067 | ApiD00068 | ApiD00026 | D_H73_T |
| BTE-0028 | ApiD00067 | ApiD00068 | ApiD00027 | D_H73_N |
| BTE-0029 | ApiD00067 | ApiD00068 | ApiD00028 | D_H73_E |
| BTE-0030 | ApiD00067 | ApiD00068 | ApiD00029 | D_H73_K |
| BTE-0031 | ApiD00067 | ApiD00068 | ApiD00030 | S_H100A_E |
| BTE-0032 | ApiD00067 | ApiD00068 | ApiD00031 | S_H100A_P |

**[Table 1-2]**

| | | | | |
|---|---|---|---|---|
| BTE-0033 | ApiD00067 | ApiD00068 | ApiD00032 | S_H100A_A |
| BTE-0034 | ApiD00067 | ApiD00068 | ApiD00033 | S_H100A_D |
| BTE-0035 | ApiD00067 | ApiD00068 | ApiD00034 | S_H100A_F |
| BTE-0036 | ApiD00067 | ApiD00068 | ApiD00035 | S_H100A_G |
| BTE-0037 | ApiD00067 | ApiD00068 | ApiD00036 | S_H100A_H |
| BTE-0038 | ApiD00067 | ApiD00068 | ApiD00037 | S_H100A_I |
| BTE-0039 | ApiD00067 | ApiD00068 | ApiD00038 | S_H100A_K |
| BTE-0040 | ApiD00067 | ApiD00068 | ApiD00039 | S_H100A_L |
| BTE-0041 | ApiD00067 | ApiD00068 | ApiD00040 | S_H100A_M |
| BTE-0042 | ApiD00067 | ApiD00068 | ApiD00041 | S_H100A_N |
| BTE-0043 | ApiD00067 | ApiD00068 | ApiD00042 | S_H100A_Q |
| BTE-0044 | ApiD00067 | ApiD00068 | ApiD00043 | S_H100A_R |
| BTE-0045 | ApiD00067 | ApiD00068 | ApiD00044 | S_H100A_T |
| BTE-0046 | ApiD00067 | ApiD00068 | ApiD00045 | S_H100A_V |
| BTE-0047 | ApiD00067 | ApiD00068 | ApiD00046 | S_H100A_Y |
| BTE-0048 | ApiD00067 | ApiD00068 | ApiD00047 | A_H101_D |
| BTE-0049 | ApiD00067 | ApiD00068 | ApiD00048 | A_H49_G |
| BTE-0050 | ApiD00067 | ApiD00068 | ApiD00049 | A_H49_S |
| BTE-0051 | ApiD00067 | ApiD00068 | ApiD00050 | W_H100E_Y |
| BTE-0052 | ApiD00067 | ApiD00068 | ApiD00051 | A_L89_L |
| BTE-0053 | ApiD00067 | ApiD00068 | ApiD00052 | A_L89_L, W_L91_Y |
| BTE-0054 | ApiD00067 | ApiD00068 | ApiD00053 | A_L89_L, W_L91_S |
| BTE-0055 | ApiD00067 | ApiD00068 | ApiD00054 | A_L89_V, W_L91_Y |
| BTE-0056 | ApiD00067 | ApiD00068 | ApiD00055 | W_L91_Y |
| BTE-0057 | ApiD00067 | ApiD00068 | ApiD00056 | W_L96_Y |
| BTE-0058 | ApiD00067 | ApiD00068 | ApiD00057 | W_L96_F |
| BTE-0059 | ApiD00067 | ApiD00068 | ApiD00058 | W_L96_G |
| BTE-0060 | ApiD00067 | ApiD00068 | ApiD00059 | W_L96_L |
| BTE-0061 | ApiD00067 | ApiD00068 | ApiD00060 | W_L96_P |
| BTE-0062 | ApiD00067 | ApiD00068 | ApiD00061 | W_L96_R |
| BTE-0063 | ApiD00067 | ApiD00068 | ApiD00062 | W_L96_V |
| BTE-0064 | ApiD00067 | ApiD00068 | ApiD00063 | V_L36_F |
| BTE-0065 | ApiD00067 | ApiD00068 | ApiD00064 | V_L36_Y |
| BTE-0066 | ApiD00067 | ApiD00068 | ApiD00065 | V_L36_H |
| BTE-0067 | ApiD00067 | ApiD00068 | ApiD00066 | V_L36_L |

### [Example 1-3: Preparation of CD3εδ Complex Protein]

To evaluate the binding activity of the anti-TSPAN8/anti-CD3 bispecific antibody to CD3, CD3εδ complex protein was obtained. The CD3εδ complex protein was obtained by introducing two expression vectors containing CD3ε or CD3δ into cells. pcDNA3.4 TOPO vector was used as the expression vector. DNA encoding an amino acid sequence in which alpaca Fc (corresponding to amino acid positions 102 to 328 in Genbank Accession No. CAO79574.1) was linked to the signal sequence and extracellular domain of CD3ε (corresponding to amino acid positions 1 to 126 in UNIPROT Accession No. P07766) in this order was inserted into the first expression vector. DNA encoding an amino acid sequence in which alpaca Fc (corresponding to amino acid positions 102 to 327 in Genbank Accession No. CAO79574.1) was linked to the signal sequence and extracellular domain of CD3δ (corresponding to amino acid positions 2 to 105 in UNIPROT Accession No. P04234) in this order was inserted into the second vector. DNA was synthesized according to a conventional method. The two vectors thus prepared were transfected into ExpiCHO-S (R) cells. Culture supernatants were purified by affinity purification using MabSelect SuRe and anion-exchange method using HiTrap(R) Q High Performance (17-1154-01, available from GE Healthcare) to obtain CD3εδ complex protein.

### [Example 1-4: Evaluation of CD3 Binding Activity of Anti-TSPAN8/Anti-CD3 Bispecific Antibody Group Having Diverse CD3 Binding Affinity]

Binding activities of the anti-TSPAN8/anti-CD3 bispecific antibodies BTE-0001 to BTE-0067 to CD3 were evaluated by the ELISA. ELISA was performed according to a conventional method. The CD3εδ complex protein obtained in Example 1-3 was immobilized on a plate. BTE-0001 to BTE-0067 were used as the test antibodies. As a secondary antibody, Goat anti-Human IgG Fc, Multi-Species SP ads-HRP (2014-05, available from SouthernBiotech) was used. TMB+Substrate-Chromogen (S1599, available from Dako) was used as the detection reagent. Infinite M200pro (available from Tecan) was used to measure the absorbance. The EC50 values calculated from the measured absorbance are shown in Table 2. The EC50 values were calculated using variable slope model (four parameters) in GraphPad Prism with the maximum value in the same plate fixed at the top and the minimum value in the blank in the same plate fixed at the bottom. Since BTE-0001 was always measured in each plate as a control, only the EC50 value of BTE-0001 was expressed as the average value of a plurality of plates.

### [Example 1-5: Evaluation of Stability of Anti-TSPAN8/Anti-CD3 scFv Bispecific Antibody Group Having Diverse CD3 Binding Affinity]

A thermal shift assay (hereinafter referred to as "TSA") was performed to evaluate the stability of BTE-0001 to BTE-0067. The midpoint denaturation temperature (hereinafter referred to as "Tm") at which 50% of the molecules were unfolded was measured by TSA. It is generally known that the higher the Tm, the more thermostable the protein. Protein Thermal Shift Dye Kit (4461146, available from Applied biosystems, hereinafter referred to as "Kit") was used for the TSA. Each antibody was mixed with a fluorescent dye attached to the Kit according to the protocol of the Kit, dispensed into a 96 well PCR plate (4346907, available from Applied Biosystems), and measured using StepOnePlus Real-time PCR System (available from Applied Biosystems). The measurement of TSA was taken by raising the temperature from 25°C to 99°C. Protein Thermal Shift Software (4466038, available from Applied biosystems) was used for the analysis.

The lowest Tm was calculated from the spectrum obtained in the measurement according to a conventional method. The difference from Tm of BTE-0001 was defined as dTm. The results are shown in Table 2.

As a result, most of BTE-0002 to BTE-0067 obtained by the modification had the same or lower Tm than BTE-0001 before modification. Only BTE-0006 to BTE-0008, BTE-0010, BTE-0034, BTE-0048, and BTE0062 had higher Tm than BTE-0001. Although Tm was expected to be about 63°C or higher, most of the resulting antibodies had a Tm around 60°C, suggesting that the thermal stability was not sufficient.

**[Table 2-1]**

| Antibody name | ELISA EC50 (nq/mL) | TSA Tm (°C) | TSA dTm (°C) |
|---|---|---|---|
| BTE-0001 | 6.52 | 60.0 | 0 |
| BTE-0002 | 10.4 | 59.7 | -0.3 |
| BTE-0003 | 7.67 | 58.9 | -1.1 |
| BTE-0004 | 5.37 | 60.0 | 0 |
| BTE-0005 | 38.7 | 59.3 | -0.7 |
| BTE-0006 | 224 | 61.1 | 1.1 |
| BTE-0007 | 3.79 | 61.1 | 1.1 |
| BTE-0008 | 431 | 63.3 | 3.3 |
| BTE-0009 | 286 | 60.0 | 0 |
| BTE-0010 | 2040 | 61.9 | 1.9 |
| BTE-0011 | 378 | 59.7 | -0.3 |
| BTE-0012 | 993 | 60.0 | 0 |
| BTE-0013 | >7500 | N.T. | N.T. |
| BTE-0014 | >7500 | N.T. | N.T. |
| BTE-0015 | 17.6 | 60.0 | 0 |
| BTE-0016 | 4.20 | 59.3 | -0.7 |
| BTE-0017 | 30.0 | 59.3 | -0.7 |
| BTE-0018 | 61.3 | 59.7 | -0.3 |
| BTE-0019 | 577 | 60.0 | 0 |
| BTE-0020 | 1260 | 60.0 | 0 |
| BTE-0021 | 38.7 | 58.6 | -1.4 |
| BTE-0022 | 46.2 | 57.1 | -2.9 |
| BTE-0023 | 208 | 58.9 | -1.1 |
| BTE-0024 | 41.2 | 57.1 | -2.9 |
| BTE-0025 | 49.4 | 60.0 | 0 |
| BTE-0026 | 45.9 | 59.3 | -0.7 |
| BTE-0027 | 2950 | N.D. | N.D. |
| BTE-0028 | 380 | N.D. | N.D. |
| BTE-0029 | 80.2 | N.D. | N.D. |
| BTE-0030 | >7500 | N.T. | N.T. |
| BTE-0031 | 17.4 | 60.0 | 0 |
| BTE-0032 | 255 | 58.9 | -1.1 |
| BTE-0033 | 10.4 | 58.6 | -1.4 |

**[Table 2-2]**

| | | | |
|---|---|---|---|
| BTE-0034 | 47.1 | 60.8 | 0.8 |
| BTE-0035 | 87.5 | 57.1 | -2.9 |
| BTE-0036 | 4.31 | 59.7 | -0.3 |
| BTE-0037 | 31.0 | 58.6 | -1.4 |
| BTE-0038 | 438 | 56.7 | -3.3 |
| BTE-0039 | 8.35 | 58.2 | -1.8 |
| BTE-0040 | 38.9 | 57.5 | -2.5 |
| BTE-0041 | 40.7 | 58.2 | -1.8 |
| BTE-0042 | 57.5 | 59.3 | -0.7 |
| BTE-0043 | 19.8 | 58.9 | -1.1 |
| BTE-0044 | 8.44 | 57.8 | -2.2 |
| BTE-0045 | 8.25 | 58.9 | -1.1 |
| BTE-0046 | 320 | 57.1 | -2.9 |
| BTE-0047 | 135 | 56.7 | -3.3 |
| BTE-0048 | 142 | 61.5 | 1.5 |
| BTE-0049 | 4.98 | 58.9 | -1.1 |
| BTE-0050 | 9.99 | 56.4 | -3.6 |
| BTE-0051 | 7.35 | 59.7 | -0.3 |
| BTE-0052 | 1230 | 54.9 | -5.1 |
| BTE-0053 | >7500 | 56.4 | -3.6 |
| BTE-0054 | >7500 | 56.4 | -3.6 |
| BTE-0055 | 35.5 | 58.2 | -1.8 |
| BTE-0056 | 98.9 | 59.7 | -0.3 |
| BTE-0057 | 1010 | 58.2 | -1.8 |
| BTE-0058 | 847 | 58.2 | -1.8 |
| BTE-0059 | >7500 | 55.3 | -4.7 |
| BTE-0060 | >7500 | 58.2 | -1.8 |
| BTE-0061 | >7500 | 58.9 | -1.1 |
| BTE-0062 | >7500 | 61.1 | 1.1 |
| BTE-0063 | >7500 | 58.2 | -1.8 |
| BTE-0064 | 1870 | 58.6 | -1.4 |
| BTE-0065 | >7500 | 57.5 | -2.5 |
| BTE-0066 | >7500 | 60.0 | 0 |
| BTE-0067 | 3.34 | 58.6 | -1.4 |

In the table, N.D. indicates not determined, and N.T. Indicates not tested.

### [Example 2: Obtaining Anti-TSPAN8/Anti-CD3 Bispecific Antibody Having Diverse CD3 Binding Affinity and Improved Stability]

In Example 1, the present inventors succeeded in obtaining anti-TSPAN8/anti-CD3 bispecific antibodies having diverse CD3 binding affinity. At the same time, however, it was also suggested that the thermal stability of the antibodies was not sufficient. Therefore, 11 antibodies were selected from the antibodies obtained in Example 1 using the CD3 binding affinity as an indicator to conduct studies with the aim of improving stability. BTE-0015, BTE-0025, BTE-0031, and BTE-0034 were selected as antibodies with high binding activity, BTE-0006, BTE-0009, and BTE-0048 were selected as antibodies with medium binding activity, and BTE-0008, BTE-0011, BTE-0012, and BTE-0019 were selected as antibodies with low binding activity. Hereinafter, these antibodies are also collectively referred to as "11 anti-TSPAN8/anti-CD3 bispecific antibodies".

### [Example 2-1: Sequence Design of Anti-TSPAN8/anti-CD3 Bispecific Antibody with Mutation Introduced to Improve Stability]

Variants were designed to improve the stability of the 11 anti-TSPAN8/anti-CD3 bispecific antibodies. US Patent No. 9701759 and Japanese Patent No. 5686953 were referred to in designing the variants. Specifically, correlations between the sequences and the property values were analyzed with reference to the property values of Tm (°C) and yield (mg/mL) described in Fig. 27 of US Patent No. 9701759. For example, in 53 of the 55 antibodies listed in Fig. 27 of U.S. Patent No. 9701759, the 75th amino acid (L75) in the light chain variable region is leucine (Leu), whereas XENP11838 and XENP12149 are each isoleucine (Ile). It was found that these antibodies tended to have higher Tm and yield than the other 53 antibodies. Therefore, in the 11 anti-TSPAN8/anti-CD3 bispecific antibodies, a mutation altering the L75 amino acid from Leu to Ile was introduced. As a result of the same correlation analysis for all amino acid residues, it was found that the Tm and the yield of the modified antibodies tended to be high when the 100th amino acid (H100) in the heavy chain variable region was aspartic acid (Asp), when the 58th amino acid (L58) in the light chain variable region was valine (Val), when the 85th amino acid (L85) in the light chain variable region was aspartic acid (Asp), and when the 95th amino acid (L95) in the light chain variable region was histidine (His). Note that the above L75, H100, L58, L85, and L95 are all based on the Kabat numbering.

Based on this result, 55 antibodies were designed by introducing mutations of Ile at L75, Asp at H100, Val at L58, Asp at L85, and His at L95 into the 11 anti-TSPAN8/anti-CD3 bispecific antibodies.

In addition, in the 55 anti-TSPAN8/anti-CD3 bispecific antibodies, P329A mutation, in which the 329th proline (Pro) in the EU index was substituted with Ala, was introduced instead of N297G used in Example 1. To confirm the effect of the change from N297G to P329A on stability, bispecific antibodies (BTE-0125 to BTE-0135) were also designed by changing N297G in the 11 anti-TSPAN8/anti-CD3 bispecific antibodies to P329A. The antibodies prepared in Example 2-1 are also referred to as "66 anti-TSPAN8/anti-CD3 bispecific antibodies". The names of the vectors used for the production of the 66 anti-TSPAN8/anti-CD3 bispecific antibodies and information on the modification sites and substituted amino acids of the anti-CD3 scFv are shown in Table 3.

**[Table 3-1]**

| Antibody name | Heavy chain (anti-TSPAN8) | Light chain (anti-TSPAN8) | Anti-CD3 scFv-Fc (anti-CD3) | Mutation of anti-CD3 variable region (amino acid before mutation_Kabat number_amino acid after mutation) |
|---|---|---|---|---|
| BTE-0070 | ApiD00133 | ApiD00068 | ApiD00078 | N_H30_S, T_H31_D, Y_H32_H, N_H100_D |
| BTE-0071 | ApiD00133 | ApiD00068 | ApiD00079 | N_H35_S, N_^{.}H100_D |
| BTE-0072 | ApiD00133 | ApiD00068 | ApiD00080 | N_H35_H, N_^{.}H100_D |
| BTE-0073 | ApiD00133 | ApiD00068 | ApiD00081 | N_H35_A, N_H100_D |
| BTE-0074 | ApiD00133 | ApiD00068 | ApiD00082 | N_H35_G, N_^{.}H100_D |
| BTE-0075 | ApiD00133 | ApiD00068 | ApiD00083 | Y_H52C_A, N_H100_D |
| BTE-0076 | ApiD00133 | ApiD00068 | ApiD00084 | Y_H58_E, N_H100_D |
| BTE-0077 | ApiD00133 | ApiD00068 | ApiD00085 | Y_H58_S, N_H100_D |
| BTE-0078 | ApiD00133 | ApiD00068 | ApiD00086 | N_H100_D, S_H100A_E |
| BTE-0079 | ApiD00133 | ApiD00068 | ApiD00087 | N_H100_D, S_H100A_D |
| BTE-0080 | ApiD00133 | ApiD00068 | ApiD00088 | N_H100_D, A_H101_D |
| BTE-0081 | ApiD00133 | ApiD00068 | ApiD00136 | N_H30_S, T_H31_D, Y_H32_H, T_L58_V |
| BTE-0082 | ApiD00133 | ApiD00068 | ApiD00137 | N_H35_S,T_L58_V |
| BTE-0083 | ApiD00133 | ApiD00068 | ApiD00138 | N_H35_H, T_L58_V |
| BTE-0084 | ApiD00133 | ApiD00068 | ApiD00139 | N_H35_A, T_L58_V |
| BTE-0085 | ApiD00133 | ApiD00068 | ApiD00140 | N_H35_G, T_L58_V |
| BTE-0086 | ApiD00133 | ApiD00068 | ApiD00141 | Y_H52C_A, T_L58_V |
| BTE-0087 | ApiD00133 | ApiD00068 | ApiD00142 | Y_H58_E, T_L58_V |
| BTE-0088 | ApiD00133 | ApiD00068 | ApiD00143 | Y_H58_S, T_L58_V |
| BTE-0089 | ApiD00133 | ApiD00068 | ApiD00144 | S_H100A_E, T_L58_V |
| BTE-0090 | ApiD00133 | ApiD00068 | ApiD00145 | S_H100A_D, T_L58_V |
| BTE-0091 | ApiD00133 | ApiD00068 | ApiD00146 | A_H101_D, T_L58_V |
| BTE-0092 | ApiD00133 | ApiD00068 | ApiD00100 | N_H30_S, T_H31_D, Y_H32_H, L_L75_I |
| BTE-0093 | ApiD00133 | ApiD00068 | ApiD00101 | N_H35_S, L_L75_I |
| BTE-0094 | ApiD00133 | ApiD00068 | ApiD00102 | N_H35_H, L_L75_I |
| BTE-0095 | ApiD00133 | ApiD00068 | ApiD00103 | N_H35_A, L_L75_I |
| BTE-0096 | ApiD00133 | ApiD00068 | ApiD00104 | N_H35_G, L_L75_I |
| BTE-0097 | ApiD00133 | ApiD00068 | ApiD00105 | Y_H52C_A, L_L75_I |
| BTE-0098 | ApiD00133 | ApiD00068 | ApiD00106 | Y_H58_E, L_L75_I |
| BTE-0099 | ApiD00133 | ApiD00068 | ApiD00107 | Y_H58_S, L_L75_I |
| BTE-0100 | ApiD00133 | ApiD00068 | ApiD00108 | S_H100A_E, L_L75_I |
| BTE-0101 | ApiD00133 | ApiD00068 | ApiD00109 | S_H100A_D, L_L75_I |
| BTE-0102 | ApiD00133 | ApiD00068 | ApiD00110 | A_H101_D, L_L75_I |

**[Table 3-2]**

| | | | | |
|---|---|---|---|---|
| BTE-0103 | ApiD00133 | ApiD00068 | ApiD00111 | N_H30_S, T_H31_D, Y_H32_H, E_L85_D |
| BTE-0104 | ApiD00133 | ApiD00068 | ApiD00112 | N_H35_S, E_L85_D |
| BTE-0105 | ApiD00133 | ApiD00068 | ApiD00113 | N_H35_H, E_L85_D |
| BTE-0106 | ApiD00133 | ApiD00068 | ApiD00114 | N_H35_A, E_L85_D |
| BTE-0107 | ApiD00133 | ApiD00068 | ApiD00115 | N_H35_G, E_L85_D |
| BTE-0108 | ApiD00133 | ApiD00068 | ApiD00116 | Y_H52C_A, E_L85_D |
| BTE-0109 | ApiD00133 | ApiD00068 | ApiD00117 | Y_H58_E, E_L85_D |
| BTE-0110 | ApiD00133 | ApiD00068 | ApiD00118 | Y_H58_S, E_L85_D |
| BTE-0111 | ApiD00133 | ApiD00068 | ApiD00119 | S_H100A_E, E_L85_D |
| BTE-0112 | ApiD00133 | ApiD00068 | ApiD00120 | S_H100A_D, E_L85_D |
| BTE-0113 | ApiD00133 | ApiD00068 | ApiD00121 | A_H101_D, E_L85_D |
| BTE-0114 | ApiD00133 | ApiD00068 | ApiD00122 | N_H30_S, T_H31_D, Y_H32_H, L_L95_H |
| BTE-0115 | ApiD00133 | ApiD00068 | ApiD00123 | N_H35_S, L_L95_H |
| BTE-0116 | ApiD00133 | ApiD00068 | ApiD00124 | N_H35_H, L_L95_H |
| BTE-0117 | ApiD00133 | ApiD00068 | ApiD00125 | N_H35_A, L_L95_H |
| BTE-0118 | ApiD00133 | ApiD00068 | ApiD00126 | N_H35_G, L_L95_H |
| BTE-0119 | ApiD00133 | ApiD00068 | ApiD00127 | Y_H52C_A, L_L95_H |
| BTE-0120 | ApiD00133 | ApiD00068 | ApiD00128 | Y_H58_E, L_L95_H |
| BTE-0121 | ApiD00133 | ApiD00068 | ApiD00129 | Y_H58_S, L_L95_H |
| BTE-0122 | ApiD00133 | ApiD00068 | ApiD00130 | S_H100A_E, L_L95_H |
| BTE-0123 | ApiD00133 | ApiD00068 | ApiD00131 | S_H100A_D, L_L95_H |
| BTE-0124 | ApiD00133 | ApiD00068 | ApiD00132 | A_H101_D, L_L95_H |
| BTE-0125 | ApiD00133 | ApiD00068 | ApiD00147 | N_H30_S, T_H31_D, Y_H32_H |
| BTE-0126 | ApiD00133 | ApiD00068 | ApiD00148 | N_H35_S |
| BTE-0127 | ApiD00133 | ApiD00068 | ApiD00149 | N_H35_H |
| BTE-0128 | ApiD00133 | ApiD00068 | ApiD00150 | N_H35_A |
| BTE-0129 | ApiD00133 | ApiD00068 | ApiD00151 | N_H35_G |
| BTE-0130 | ApiD00133 | ApiD00068 | ApiD00152 | Y_H52C_A |
| BTE-0131 | ApiD00133 | ApiD00068 | ApiD00153 | Y_H58_E, |
| BTE-0132 | ApiD00133 | ApiD00068 | ApiD00154 | Y_H58_S |
| BTE-0133 | ApiD00133 | ApiD00068 | ApiD00155 | S_H100A_E |
| BTE-0134 | ApiD00133 | ApiD00068 | ApiD00156 | S_H100A_D |
| BTE-0135 | ApiD00133 | ApiD00068 | ApiD00157 | A_H101_D |

### [Example 2-2: Obtaining Anti-TSPAN8/Anti-CD3 Bispecific Antibody with Mutation Introduced to Improve Stability]

DNA encoding the amino acid sequences of anti-CD3 scFv-Fc of the 66 anti-TSPAN8/anti-CD3 bispecific antibodies was synthesized according to a conventional method and inserted into a pcDNA3.4 TOPO vector. The thus prepared vectors are designated as ApiD00078 to ApiD00088, ApiD00100 to ApiD00132, and ApiD00136 to ApiD00157 (hereinafter also referred to as "66 anti-CD3 scFv vectors") (Table 3). As the N297G mutation contained in the Fc region of these vectors was changed to P329A mutation, DNA in which the N297G mutation in the heavy chain amino acid sequence (SEQ ID NO: 2) of the anti-TSPAN8 antibody was changed to the P329A mutation was synthesized and inserted into the pcDNA3.4 TOPO vector. The thus produced vector is designated as ApiD00133.

To obtain modified anti-TSPAN8/anti-CD3 bispecific antibodies, ExpiCHO-S (R) cells were transfected with any one of the 66 anti-CD3 scFv vectors, ApiD00067, and ApiD00068. According to a conventional method, culture supernatants were purified using MabSelect SuRe to obtain 66 anti-TSPAN8/anti-CD3 antibodies of BTE-0070 to BTE-135. The names of the vectors used for the production of the anti-TSPAN8/anti-CD3 bispecific antibodies and information on the modification sites and substituted amino acids of the anti-CD3 scFv are shown in Table 3.

### [Example 2-3: Evaluation of CD3 Binding Activity of Anti-TSPAN8/Anti-CD3 Bispecific Antibody Group with Introduced Mutation to Improve Stability]

Binding activities of the 66 anti-TSPAN8/anti-CD3 bispecific antibodies to CD3 were evaluated by the ELISA described in Example 1-4. As a result, even when various modifications were made to the anti-CD3 scFv to improve the stability, various binding activities to CD3 were not significantly impaired, and the original binding activities were mostly maintained. The ratios of the EC50 values for binding to CD3 and the EC50 values for BTE-0001 are shown in Table 4.

### [Example 2-4: Evaluation of Stability of 66 Anti-TSPAN8/Anti-CD3 Bispecific Antibodies with Introduced Mutation to Improve Stability]

Stabilities of the 66 anti-TSPAN8/anti-CD3 bispecific antibodies were evaluated by the TSA described in Example 1-5. The resulting Tm and the difference dTm from Tm of BTE-0001 are shown in Table 4. An antibody having ELISA EC50 of 1250 ng/mL or less and Tm of 63°C or higher in TSA was defined as an antibody with improved stability. Furthermore, an antibody was selected with which the amount of antibody produced (yield) relative to the medium volume during protein preparation was 1 mg/mL or more. In addition, selected 34 anti-TSPAN8/anti-CD3 scFv bispecific antibodies allowed to stand at 40°C for a week to evaluate their stabilities. The stabilities of the antibodies were evaluated by measuring CD3 binding activities of the antibodies before and after static incubation using surface plasmon resonance (SPR). Biacore T-200 (Cytiva) was used for the measurements. A chip used for the Biacore measurements was prepared by immobilizing an anti-His antibody on the Series S Sensor Chip CM5 (29104988, available from Cytiva) using the His Capture Kit ( 28995056, available from Cytiva) and Amine Coupling Kit (BR100050, available from Cytiva), followed by binding of Human CD3 epsilon & CD3 delta Heterodimer Protein, His Tag & Tag Free (MALS verifies) (CDD-H52W1, available from AcroBiosystems). The CD3 binding activity after static incubation (Biacore40C1W) was calculated with the CD3 binding activity before static incubation as 100% (Table 4). The lower the relative values in the Biacore40C1W, the poorer the static storage stability. As a result, antibodies BTE-0079, 0080, 0090, 0091, 0092, 0097 to 0102, 0111 to 0113, 0134, 0135 with a Tm of 63°C or higher and a residual activity of 78% or more retained were found. The anti-CD3 scFvs used in the 16 antibodies are also referred to as "16 anti-CD3 scFvs".

**[Table 4-1]**

| Antibody name | ELISA EC50 (nq/mL) | Tm TSA (°C) | dTm TSA (°C) | Biacore 40C1W(%) |
|---|---|---|---|---|
| BTE-0070 | 632 | 63.5 | 3.5 | N.T. |
| BTE-0071 | >7500 | 66.3 | 6.3 | N.T. |
| BTE-0072 | 1640 | 62.4 | 2.4 | N.T. |
| BTE-0073 | 1330 | 63.0 | 3.0 | N.T. |
| BTE-0074 | >7500 | 64.0 | 4.0 | N.T. |
| BTE-0075 | 113 | 62.4 | 2.4 | N.T. |
| BTE-0076 | 438 | 62.7 | 2.7 | N.T. |
| BTE-0077 | 274 | 62.2 | 2.2 | N.T. |
| BTE-0078 | 634 | 63.7 | 3.7 | N.T. |
| BTE-0079 | 220 | 64.0 | 4.0 | 78.9 |
| BTE-0080 | 1180 | 64.3 | 4.3 | 93.9 |
| BTE-0081 | 152 | 63.8 | 3.8 | 67.2 |
| BTE-0082 | 322 | 66.0 | 6.0 | 57.2 |
| BTE-0083 | 165 | 62.1 | 2.1 | N.T. |
| BTE-0084 | 361 | 61.2 | 1.2 | N.T. |
| BTE-0085 | 729 | 61.6 | 1.6 | N.T. |
| BTE-0086 | 37.9 | 62.1 | 2.1 | N.T. |
| BTE-0087 | 1020 | 62.2 | 2.2 | N.T. |
| BTE-0088 | 55.6 | 61.9 | 1.9 | N.T. |
| BTE-0089 | 97.5 | 63.0 | 3.0 | N.T. |
| BTE-0090 | 120 | 63.7 | 3.7 | 103.4 |
| BTE-0091 | 623 | 64.1 | 4.1 | 103.3 |
| BTE-0092 | 187 | 64.6 | 4.6 | 89.3 |
| BTE-0093 | 90.3 | 66.9 | 6.9 | N.D. |
| BTE-0094 | 297 | 63.0 | 3.0 | N.T. |
| BTE-0095 | 410 | 63.5 | 3.5 | N.T. |
| BTE-0096 | 802 | 64.1 | 4.1 | 59.5 |
| BTE-0097 | 30.8 | 63.1 | 3.1 | 83.2 |
| BTE-0098 | 438 | 63.5 | 3.5 | 79.2 |
| BTE-0099 | 68.2 | 63.1 | 3.1 | 82.8 |
| BTE-0100 | 247 | 64.1 | 4.1 | 83.6 |

**[Table 4-2]**

| | | | | |
|---|---|---|---|---|
| BTE-0101 | 113 | 64.3 | 4.3 | 83.3 |
| BTE-0102 | 770 | 64.4 | 4.4 | 101.2 |
| BTE-0103 | 180 | 64.1 | 4.1 | 42.1 |
| BTE-0104 | 499 | 66.6 | 6.6 | N.D. |
| BTE-0105 | 201 | 63.0 | 3.0 | N.T. |
| BTE-0106 | 327 | 62.7 | 2.7 | N.T. |
| BTE-0107 | 594 | 63.0 | 3.0 | N.T. |
| BTE-0108 | 8.31 | 62.9 | 2.9 | 72.2 |
| BTE-0109 | 217 | 63.2 | 3.2 | N.T. |
| BTE-0110 | 47.1 | 62.8 | 2.8 | N.T. |
| BTE-0111 | 66.0 | 63.7 | 3.7 | 78.7 |
| BTE-0112 | 43.3 | 64.1 | 4.1 | 84.8 |
| BTE-0113 | 363 | 64.6 | 4.6 | 101.4 |
| BTE-0114 | 86.0 | 63.5 | 3.5 | N.T. |
| BTE-0115 | 358 | 65.3 | 5.3 | 57.5 |
| BTE-0116 | 187 | 61.8 | 1.8 | N.T. |
| BTE-0117 | 90.0 | 61.2 | 1.2 | N.T. |
| BTE-0118 | 709 | 61.5 | 1.5 | N.T. |
| BTE-0119 | 23.2 | 62.7 | 2.7 | N.T. |
| BTE-0120 | 240 | 62.7 | 2.7 | N.T. |
| BTE-0121 | 45.8 | 62.2 | 2.2 | N.T. |
| BTE-0122 | 242 | 63.5 | 3.5 | N.T. |
| BTE-0123 | 192 | 64.3 | 4.3 | N.T. |
| BTE-0124 | 378 | 63.7 | 3.7 | 69.7 |
| BTE-0125 | 163 | 63.1 | 3.1 | 51.8 |
| BTE-0126 | 330 | 65.4 | 5.4 | 60.0 |
| BTE-0127 | 122 | 61.5 | 1.5 | 41.6 |
| BTE-0128 | 174 | 61.3 | 1.3 | 64.9 |
| BTE-0129 | 469 | 61.9 | 1.9 | 52.8 |
| BTE-0130 | 10.9 | 61.8 | 1.8 | 77.7 |
| BTE-0131 | 269 | 62.1 | 2.1 | 79.2 |
| BTE-0132 | 31.5 | 61.8 | 1.8 | 72.9 |
| BTE-0133 | 69.3 | 62.6 | 2.6 | 69.0 |
| BTE-0134 | 46.0 | 63.1 | 3.1 | 86.8 |
| BTE-0135 | 204 | 63.5 | 3.5 | 84.8 |

In the table, N.D. indicates not determined, and N.T. Indicates not tested.

### [Example 3: Obtaining Anti-CD37/Anti-CD3 Bispecific Antibody]

To confirm whether the 16 anti-CD3 scFvs obtained in Example 2-4 could also be used for bispecific antibodies with any anti-TAA antibody, an attempt was made to obtain an anti-CD37/anti-CD3 bispecific antibody using a CD37 antibody.

As the anti-CD37 antibodies, anti-CD37 antibodies set forth in SEQ ID NOs: 10 and 12 derived from International Publication No. WO 2015017552 were used. DNA was synthesized based on the DNA sequence encoding the anti-CD37 antibody described in SEQ ID NO: 9 and SEQ ID NO: 11 of International Publication No. WO 2015017552 and inserted into the pcDNA3.4 TOPO vector. The thus prepared vectors are designated as ApiD00158 and ApiD00159. ExpiCHO-S (R) cells were transfected with ApiD00087, ApiD00088, ApiD00145, ApiD00146, ApiD00100, ApiD00105 to ApiD00110, ApiD00119 to ApiD00121, ApiD00156, and ApiD00157 encoding anti-CD3 scFv. According to a conventional method, culture supernatants were purified using MabSelect SuRe. The resulting 16 anti-CD37/anti-CD3 bispecific antibodies are referred to as BTE-0136 to 0149, 0151, and 0152 (hereinafter, also referred to as the "16 anti-CD37/anti-CD3 bispecific antibodies"). The names of the vectors used for the production of the anti-CD37/anti-CD3 bispecific antibodies, the modified amino acid number of the anti-CD3 scFv, and mutation information are shown in Table 5.

**[Table 5]**

| Antibody name | Heavy chain (anti-CD37) | Light chain (anti-CD37) | Anti-CD3 scFv-Fc (anti-CD3) | Mutation of anti-CD3 variable region (amino acid before mutation_Kabat number_amino acid after mutation) |
|---|---|---|---|---|
| BTE-0136 | ApiD00158 | ApiD00159 | ApiD00087 (SEQ ID NO: 14) | N_H100_D, S_H100A_D |
| BTE-0137 | ApiD00158 | ApiD00159 | ApiD00088 (SEQ ID NO: 20) | N_H100_D, A_H101_D |
| BTE-0138 | ApiD00158 | ApiD00159 | ApiD00145 (SEQ ID NO: 22) | S_H100A_D, T_L58_V |
| BTE-0139 | ApiD00158 | ApiD00159 | ApiD00146 (SEQ ID NO: 24) | A_H101_D, T_L58_V 24) |
| BTE-0140 | ApiD00158 | ApiD00159 | ApiD00100 (SEQ ID NO: 26) | N_H30_S, T_H31_D, Y_H32_H, L_L75_I |
| BTE-0141 | ApiD00158 | ApiD00159 | ApiD00105 (SEQ ID NO: 28) | Y_H52C_A, L_L75_I |
| BTE-0142 | ApiD00158 | ApiD00159 | ApiD00106 (SEQ ID NO: 16) | Y_H58_E, L_L75_I |
| BTE-0143 | ApiD00158 | ApiD00159 | ApiD00107 (SEQ ID NO: 30) | Y_H58_S, L_L75_I |
| BTE-0144 | ApiD00158 | ApiD00159 | ApiD00108 (SEQ ID NO: 32) | S_H100A_E, L_L75_I |
| BTE-0145 | ApiD00158 | ApiD00159 | ApiD00109 (SEQ ID NO: 34) | S_H100A_D, L_L75_I |
| BTE-0146 | ApiD00158 | ApiD00159 | ApiD00110 (SEQ ID NO: 36) | A_H101_D, L_L75_I |
| BTE-0147 | ApiD00158 | ApiD00159 | ApiD00119 (SEQ ID NO: 38) | S_H100A_E, E_L85_D |
| BTE-0148 | ApiD00158 | ApiD00159 | ApiD00120 (SEQ ID NO: 18) | S_H100A_D, E_L85_D |
| BTE-0149 | ApiD00158 | ApiD00159 | ApiD00121 (SEQ ID NO: 40) | A_H101_D, E_L85_D |
| BTE-0151 | ApiD00158 | ApiD00159 | ApiD00156 (SEQ ID NO: 42) | S_H100A_D |
| BTE-0152 | ApiD00158 | ApiD00159 | ApiD00157 (SEQ ID NO: 44) | A_H101_D |

### [Example 4: Evaluation of In Vitro Activity of Anti-CD37/Anti-CD3 Bispecific Antibody]

To select an antibody with excellent stability and activity from the 16 anti-CD37/anti-CD3 bispecific antibodies prepared in Example 3, the binding activity to human CD3, stability, cytotoxicity in a co-culture system of CD37 expressing cells and human T cells (Redirected T Cell Cytotoxicity: RTCC), and cytokine production were evaluated as follows.

### [Example 4-1: Evaluation of Binding Activity of 16 Anti-CD37/Anti-CD3 Bispecific Antibodies]

Binding activities of the 16 anti-CD37/anti-CD3 bispecific antibodies to CD3 were evaluated by the ELISA described in Example 1-4. As a result, it was confirmed that the 16 anti-CD3 bispecific antibodies substantially maintained their various binding activities to CD3 even in the bispecific antibodies with anti-CD37 antibodies. EC50 values for the binding activities of the 16 anti-CD37/anti-CD3 bispecific antibodies to CD3 are shown in Table 6.

### [Example 4-2: Evaluation of Stability of 16 Anti-CD37/Anti-CD3 Bispecific Antibodies]

Stabilities of the 16 anti-CD37/anti-CD3 bispecific antibodies were evaluated by the TSA described in Example 1-5. Tm and the difference dTm from Tm of BTE-0001 are shown in Table 6. After the antibodies were allowed to stand at 40°C for a week, the residual rates of anti-CD3 binding activities were measured (Table 6). All of the 16 anti-CD37/anti-CD3 bispecific antibodies exhibited approximately 70% or more stability in Biacore40C1W. In particular, BTE-0136, 0138, 0141, 0142, 0144, 0145, 0148, and 0151 showed a residual rate of 80% or more, suggesting that these antibodies were highly stable.

**[Table 6]**

| Antibody name | ELISA EC50 (ng/mL) | Tm TSA (°C) | dTm TSA (°C) | Biacore 40C1W(%) |
|---|---|---|---|---|
| BTE-0136 | 66.9 | 64.3 | 4.3 | 98.4 |
| BTE-0137 | 1600 | 64.6 | 4.6 | 79.7 |
| BTE-0138 | 71.0 | 63.7 | 3.7 | 98.9 |
| BTE-0139 | 444 | 64.3 | 4.3 | 71.4 |
| BTE-0140 | 205 | 64.6 | 4.6 | 69.6 |
| BTE-0141 | 29.0 | 63.4 | 3.4 | 111.7 |
| BTE-0142 | 292 | 63.7 | 3.7 | 84.0 |
| BTE-0143 | 64.4 | 63.3 | 3.3 | 77.7 |
| BTE-0144 | 138 | 64.1 | 4.1 | 85.2 |
| BTE-0145 | 85.4 | 64.4 | 4.4 | 101.2 |
| BTE-0146 | 488 | 65.3 | 5.3 | 70.1 |
| BTE-0147 | 31.7 | 63.8 | 3.8 | 73.9 |
| BTE-0148 | 36.4 | 64.4 | 4.4 | 88.8 |
| BTE-0149 | 209 | 64.9 | 4.9 | 72.2 |
| BTE-0151 | 74.7 | 63.1 | 3.1 | 90.2 |
| BTE-0152 | 360 | 63.7 | 3.7 | 70.2 |

### [Example 4-3: Evaluation of RTCC Activity of 16 Anti-CD37/Anti-CD3 Bispecific Antibodies]

In vitro RTCC activities induced by the action of anti-CD37/anti-CD3 bispecific antibodies on a co-culture system of CHO-K1 cells expressing human CD37 and GFPSpark(R) (CHO-K1_human CD37/GFPSpark cells) and T cells isolated from human PBMCs were evaluated.

### 1: Preparation of CHO-K1_human CD37/GFPSpark cell

CHO-K1_human CD37/GFPSpark cells were prepared by the following method: A human CD37 expression vector was constructed by inserting DNA encoding human CD37 (NCBI Accession No. NP_001765.1) into a pcDNA3.4 TOPO vector according to a conventional method. A GFPSpark expression vector was constructed by removing the PD-L1 sequence from the PD-L1-GFPSpark expression vector (HG10084-ACG, available from SinoBiological) according to a conventional method. These two plasmid vectors were transfected into CHO-K1 cells (American Type Culture Collection, CCL-61) using Lipofectamine 3000 Transfection Reagent (L3000015, available from Thermo Fisher Scientific) and cultured in a medium containing Geneticin (10131-027, available from Thermo Fisher Scientific) and Hygromycin B Gold (ant-hg-5, available from InvivoGen) to select transfected cells. Cells in which expression of human CD37 and GFPSpark were confirmed by flow cytometry after isolation of single clonal cells are used as CHO-K1_human CD37/GFPSpark cells in the following experiments.

### 2: Preparation of Expanded pan T cell

Pan T cells (including both CD4T cells and CD8T cells) were isolated from human PBMC (PB003F, available from ALLCELLS) using Pan T Cell Isolation Kit, human (130-096-535, available from Miltenyi Biotec) according to the attached protocol. The isolated pan T cells were diluted to 1 × 10⁶ cells/mL, Dynabeads Human T-Activator CD3/CD28 for T Cell Expansion and Activation (11131D, available from Thermo Fisher Scientific) in the same number as the pan T cells and human IL-2 (200-02, available from PeproTech) at a final concentration of 30 U/mL were added thereto, and the cells were cultured in a 5% CO₂ incubator at 37°C, which were passaged at a concentration of 5 × 10⁵ cells/mL at 4 days, 6 days, and 8 days after the start of culture. Human IL-2 was added at a final concentration of 30 U/mL at the time of passage 4 and 6 days after the start of culture. The cells were collected 11 days after the start of culture, cleared of Dynabeads Human T-Activator CD3/CD28 for T Cell Expansion and Activation with a magnet, and then suspended in CELLBANKER1 (CB011, available from Nihon Zenyaku Kogyo Co., Ltd.) and cryopreserved. The prepared cells are referred to as expanded pan T cells.

### 3: RTCC assay

The suspension of the CHO-K1_human CD37/GFPSpark cells prepared at 5 × 10⁴ cells/mL in the culture medium was seeded in a flat bottom 384 well plate (781182, available from Greiner) in an amount of 20 µL (1 × 10³ cells) for each and cultured in a 5% CO₂ incubator at 37°C for 3 hours. BTE-0136, 0138, 0141 to 0145, 0147, 0148, and 0151 were each diluted to 2.9 pg/mL at a 4-fold common ratio with a maximum final concentration of 3.0 µg/mL (converted to a final concentration of 0.95 pg/mL to 1.0 µg/mL). BTE-0137, 0139, 0140, 0146, 0149, and 0152 were each diluted to 29 pg/mL at a 4-fold common ratio with a maximum final concentration of 30 µg/mL (converted to a final concentration of 9.5 pg/mL to 10 µg/mL). The diluted antibodies were added to the 384 well plate under cultivation in an amount of 20 µL for each, to which the expanded pan T cells prepared at 5 × 10⁵ cells/mL in the culture medium were added in an amount of 20 µL for each and cultured in a 5% CO₂ incubator at 37°C for 3 days. The GFPSpark fluorescence area of each well was then measured as an indicator of viable cell count using IncuCyte(R) ZOOM (available from Sartorius AG). The relationship between the concentrations of various anti-CD37/anti-CD3 bispecific antibodies and GFPSpark fluorescence areas is shown in Figs. 1-1 and 1-2. The results showed that 15 anti-CD37/anti-CD3 bispecific antibodies, except for BTE-0137, reduced the viable cell count of CHO-K1_human CD37/GFPSpark cells.

### 4: Evaluation of T-Cell Activating Effect of 16 Anti-CD37/Anti-CD3 Bispecific Antibodies

IFN-y and TNF-α contained in the culture supernatant of the RTCC assay of Example 4-3-3 were measured by ELISA to evaluate the T cell activation effect of the anti-CD37/anti-CD3 bispecific antibodies. BD OptEIA Human IFN-y ELISA (555142, available from BD Biosciences) and Human TNF-alpha DuoSet ELISA (DY210-5, available from R&D Systems) were used for the measurement. Graphs plotting the amount of detected IFN-y and TNF-α against the concentration of added anti-CD37/anti-CD3 bispecific antibody are shown in Fig. 2-1 to 2-4. The results showed that 15 anti-CD37/anti-CD3 bispecific antibodies, except for BTE-0137, induced the production of IFN-y and TNF-α, whereas the amounts of IFN-y and TNF-α produced were decreased in many antibodies in the presence of antibodies at a certain concentration or higher. In general, it is known that an antibody designed to form a crosslink between cells by recognizing two antigen molecules is inhibited from forming a crosslink between cells under a high concentration condition because the antigen is occupied by an antibody bound to only one antigen molecule (AAPS J., 2019, Vol. 21(4), p. 66). A decrease in the amount of IFN-y and TNF-α produced in the high antibody concentration range is a result of such inhibition of intercellular crosslink formation, and antibodies with such characteristics are considered to be antibodies that are likely to attenuate the cytotoxic effect of T cells, and are therefore not preferable as antibodies to be used for treating human cancer. In view of the above, BTE-0136, 0142, 0148 were selected as candidate antibodies for use in human therapy as a result of a comprehensive selection of antibodies with diverse CD3 binding affinities, stability, and a small reduction rate in IFN-y and TNF-α production even at high concentrations in the RTCC assay. The results suggest that anti-TAA-anti-CD3 scFv bispecific antibodies can be prepared by combination with 16 anti-CD3 scFvs having diverse binding affinity for CD3, thereby obtaining anti-TAA/anti-CD3 scFv bispecific antibodies having preferred profiles for use in human therapy.

### [Example 5: In Vivo Antitumor Evaluation of Anti-CD37/Anti-CD3 Bispecific Antibody]

Six-week-old NOD/Shi-scid, IL-2RγKO (NOG) female mice (available from In-Vivo Science Inc.) were transplanted with 2.5 × 10⁶ cells/100 µL of human PBMC (33000-10M, available from Precision for Medicine) suspended in PBS from the tail vein. Subsequently, 13 days after transplantation of the human PBMC, 2 × 10⁶ cells/100 µL of SU-DHL-6 cells (CRL-2959, available from American Type Culture Collection) suspended in Corning Matrigel Basement Membrane Matrix, Phenol Red-Free (356237, available from Corning) were transplanted subcutaneously into mice. Tumor volume and body weight were measured 8 days after transplantation of the SU-DHL-6 cells, and the mice were divided into groups so that the tumor volume became equal in each group. BTE-0136, 0142, and 0148 diluted in PBS were administered from the tail vein at doses of 5, 1, 0.1, 0.01, and 0.001 mg/kg on the day of and 7 days after grouping. PBS was administered to the control group. Tumor volumes were measured on the day of and 4, 7, and 11 days after grouping. The test was carried out with 8 mice in each group. However, one case in each of the 0.001 mg/kg BTE-0136 and 0.001 mg/kg BTE-0148 treated groups that died before 11 days after the start of antibody administration was excluded from the analysis. The results are shown in Figs. 3-1 to 3-3. Tumor volume [mm³] was calculated by the following formula: (Length [mm] of long axis of tumor) × (length [mm] of short axis of tumor)² × 0.5

Tumor volumes 11 days after the start of antibody administration were compared between the control group and each antibody-treated group, and * was assigned to a group with a P <0.05 and * * to a group with a P <0.01 by Dunnett's multiple comparison test. The results showed significant anti-tumor effects of BTE-0136 and BTE-0142 at doses of 0.001 mg / kg or more and BTE-0148 at doses of 0.01 mg/kg or more.

### [Example 6: Obtaining Anti-SLC34A2/Anti-CD3 Bispecific Antibody]

To confirm whether the bispecific antibodies of any anti-TAA antibody and the 16 anti-CD3 scFvs obtained in Examples 2-4 had various binding activities to CD3 and good stability, an attempt was made to obtain an anti-SLC34A2/anti-CD3 bispecific antibody using a SLC34A2 antibody.

### [Example 6-1: Obtaining SLC34A2 Antibody]

The present inventors prepared an expression vector carrying a polynucleotide encoding human SLC34A2 or monkey SLC34A2 to prepare human SLC34A2 or monkey SLC34A2 expressing cells using the expression vector. AlivaMab mice (available from Ablexis, US Patent No. 9346873) were immunized with the expression vector or expression cells to prepare hybridomas according to a conventional method. Antibodies produced by the hybridomas were screened using binding to SLC34A2 expressing cells as an indicator to obtain a plurality of SLC34A2 antibodies.

### [Example 6-2: Preparation of Anti-SLC34A2/Anti-CD3 Bispecific Antibody]

Anti-SLC34A2/anti-CD3 bispecific antibodies were prepared using the anti-SLC34A2 antibodies consisting of the amino acid sequences of SEQ ID NO: 65 and SEQ ID NO: 67, which were obtained by the method described in Example 6-1.

DNA was synthesized based on the DNA sequences of SEQ ID NO: 64 and SEQ ID NO: 66) and inserted into a pcDNA3.4 TOPO vector. The thus prepared vectors are designated as SLC_32O14A_HC and SLC_32O14A_LC. ExpiCHO-S (R) cells were transfected with SLC_32O14A_HC, SLC_32O14A_LC, and one of ApiD00087, ApiD00145, ApiD00100, ApiD00105 to ApiD00109, and ApiD00119 to ApiD00121 encoding anti-CD3 scFv. The culture supernatant was purified using Protein A cartridges (G5496-60018, available from Agilent) according to a conventional method. The resulting 11 anti-SLC34A2/anti-CD3 bispecific antibodies are referred to as BTE-0271 to 0281 (hereinafter, also referred to as the "11 anti-SLC34A2/anti-CD3 bispecific antibodies"). The names of the vectors used for the production of the anti-SLC34A2/anti-CD3 bispecific antibodies, the modified amino acid number of the anti-CD3 scFv, and mutation information are shown in Table 7.

**[Table 7]**

| Antibody name | Heavy chain (anti-SLC34A2) | Light chain (anti-SLC34A2) | Heavy chain (anti-CD3) | Mutation of anti-CD3 variable region (amino acid before mutation_Kabat number_amino acid after mutation) |
|---|---|---|---|---|
| BTE-0271 | SLC_32O14A_HC | SLC_32O14A_LC | ApiD00087 | N_H100_D, S_H100A_D |
| BTE-0272 | SLC_32O14A_HC | SLC_32O14A_LC | ApiD00145 | S_H100A_D, T_L58_V |
| BTE-0273 | SLC_32014A_HC | SLC_32014A_LC | ApiD00100 | N_H30_S, T_H31_D, Y_H32_H, L_L75_I |
| BTE-0274 | SLC_32O14A_HC | SLC_32O14A_LC | ApiD00105 | Y_H52C_A, L_L75_I |
| BTE-0275 | SLC_32O14A_HC | SLC_32O14A_LC | ApiD00106 | Y_H58_E, L_L75_I |
| BTE-0276 | SLC_32O14A_HC | SLC_32O14A_LC | ApiD00107 | Y_H58_S, L_L75_I |
| BTE-0277 | SLC_32O14A_HC | SLC_32O14A_LC | ApiD00108 | S_H100A_E, L_L75_I |
| BTE-0278 | SLC_32O14A_HC | SLC_32O14A_LC | ApiD00109 | S_H100A_D, L_L75_I |
| BTE-0279 | SLC_32O14A_HC | SLC_32O14A_LC | ApiD00119 | S_H100A_E, E_L85_D |
| BTE-0280 | SLC_32O14A_HC | SLC_32O14A_LC | ApiD00120 | S_H100A_D, E_L85_D |
| BTE-0281 | SLC_32O14A_HC | SLC_32O14A_LC | ApiD00121 | A_H101_D, E_L85_D |

### [Example 6-3: Evaluation of CD3 Binding Activity of Anti-SLC34A2/Anti-CD3 Bispecific Antibody]

Binding activities of the 11 anti-SLC34A2/anti-CD3 bispecific antibodies to CD3 were evaluated by the ELISA described in Example 1-4 (Fig. 4). As a result, it was confirmed that the 11 anti-SLC34A2/anti-CD3 bispecific antibodies substantially maintained their various binding activities to CD3.

### [Example 6-4: Evaluation of Stability of Anti-SLC34A2/Anti-CD3 Bispecific Antibody]

Stabilities of the 11 anti-SLC34A2/anti-CD3 bispecific antibodies were evaluated by the TSA described in Example 1-5. Tm and the difference dTm from Tm of BTE-0001 are shown in Table 8. All of the 11 antibodies exhibited higher stability than BTE-0001.

**[Table 8]**

| Antibody name | Tm TSA (°C) | dTm TSA (°C) |
|---|---|---|
| BTE-0271 | 64.1 | 4.1 |
| BTE-0272 | 63.4 | 3.4 |
| BTE-0273 | 64.3 | 4.3 |
| BTE-0274 | 63.4 | 3.4 |
| BTE-0275 | 64.0 | 4.0 |
| BTE-0276 | 63.5 | 3.5 |
| BTE-0277 | 63.8 | 3.8 |
| BTE-0278 | 64.3 | 4.3 |
| BTE-0279 | 64.0 | 4.0 |
| BTE-0280 | 64.0 | 4.0 |
| BTE-0281 | 64.4 | 4.4 |

### [Example 6-5: Evaluation of In Vitro Activity of Anti-SLC34A2/Anti-CD3 Bispecific Antibody]

*In vitro* RTCC activities induced by the action of anti-SLC34A2/anti-CD3 bispecific antibodies on a co-culture system of SLC34A2 expressing cells and T cells isolated from human PBMCs were evaluated.

### 1: Preparation of OVCAR-3 GFPSpark cell

The GFPSpark expression vector prepared in Example 4-3 was transfected into OVCAR-3 cells endogenously expressing SLC34A2 using Lipofectamine LTX Reagent with PLUS Reagent (15338100, available from Thermo Fisher Scientific). Cells in which expression of GFPSpark was confirmed by flow cytometry after isolation of single clonal cells are referred to as "OVCAR-3_GFPSpark cells".

### 2: RTCC assay

BTE-0272 to 0280 of the 11 anti-SLC34A2/anti-CD3 bispecific antibodies were used as test antibodies. The OVCAR-3_GFPSpark cells were suspended in a culture medium to give 5 × 10⁴ cells/mL. The suspension was seeded in a flat bottom 384 well plate (781182, available from Greiner) in an amount of 20 µL (1 × 10³ cells) for each and cultured in a 5% CO₂ incubator at 37°C for 2 hours. Subsequently, the test antibodies were each diluted to 29 pg/mL at a 4-fold common ratio with a maximum final concentration of 30 µg/mL (converted to a final concentration of 10 µg/mL to 9.5 pg/mL) and added to the 384 well plate under cultivation in an amount of 20 µL for each, to which the expanded pan T cells (produced in Example 4-3) prepared at 5 × 10⁵ cells/mL in the culture medium were added in an amount of 20 µL for each and cultured in a 5% CO₂ incubator at 37°C for 2 days. The GFPSpark fluorescence area of each well was then measured as an indicator of viable cell count using IncuCyte(R) S3 (available from Sartorius AG). The results showed that all 9 anti-SLC34A2/anti-CD3 bispecific antibodies reduced the viable cell count of OVCAR-3_GFPSpark cells (Fig. 5). The results of Examples 3 to 6 showed that the 16 anti-CD3 scFvs, which were obtained by using the diversity of binding activity to CD3 and the stability of the antibody as indicators in combination with an anti-TSPAN8 antibody, could maintain the diversity of binding activity to CD3 and the stability of the antibody even in a bispecific antibody prepared using an anti-CD37 antibody and an anti-SLC34A2 antibody. From these results, even in combination with other anti-TAA antibodies, the 16 anti-CD3 scFvs are expected to have the diversity of binding activity to CD3 and the stability of antibodies.

### [Example 7: Obtaining Anti-TAA/Anti-CD3/Anti-CD137 Trispecific Antibody]

The present inventors further conceived the idea of using anti-CD3 scFv antibodies in trispecific antibodies. In one embodiment thereof, a study was then conducted to obtain an anti-TAA/anti-CD3/ anti-CD 137 trispecific antibody. Anti-CLDN4 and anti-PD-L1 antibodies were used as anti-TAA antibodies, which are hereinafter referred to as an "anti-CLDN4/anti-CD3/anti-CD137 trispecific antibody" and "anti-PD-L1/anti-CD3/anti-CD137 trispecific antibody", respectively. The antibodies may also be referred to collectively as "anti-TAA/anti-CD3/anti-CD 137 trispecific antibodies".

### [Example 7-1: Obtaining Anti-CLDN4/Anti-CD3 Bispecific Antibody and Anti-PD-L1/Anti-CD3 Bispecific antibody]

To produce an anti-TAA/anti-CD3/anti-CD137 tri-specific antibody, an anti-TAA/anti-CD3 bi-specific antibody was first produced. An anti-CLDN4-anti-CD3 bispecific antibody was designed in the same manner as in Example 3, based on the amino acid sequence of the anti-CLDN4 antibody described in SEQ ID NO: 6 and 8 and International Publication No. WO 2022/224997. An anti-PD-L1./anti-CD3 bispecific antibody was designed based on the amino acid sequence of the anti-PD-L1 antibody described in SEQ ID NO: 22 and 23 of International Publication No. WO 2012/155019.

DNA encoding anti-CLDN4-anti-CD3 and anti-PD-L1-anti-CD3 bispecific antibodies was synthesized according to a conventional method and inserted into the pcDNA3.4 TOPO vector. The vector with the DNA encoding the heavy chain of the anti-CLDN4 antibody was called hKM3900(-), the vector with the DNA encoding the light chain of the anti-CLDN4 antibody was called hKM3900_k, the vector with the DNA encoding the heavy chain of atezolizumab was called atezolizumab_HC (-), and the vector with the DNA encoding the light chain of atezolizumab was called atezolizumab _LC. ExpiCHO-S(R) cells were transfected with hKM3900(-), hKM3900_k, and any one of ApiD00087, ApiD00088, ApiD00100, and ApiD00146 encoding anti-CD3 scFv. According to a conventional method, culture supernatants were purified using MabSelect SuRe to obtain 4 anti-CLDN4/anti-CD3 antibodies. ExpiCHO-S (R) cells were transfected with atezolizumab_HC(-), atezolizumab_LC, and any one of ApiD00087, ApiD00088, ApiD00100, and ApiD00146 encoding anti-CD3 scFv. According to a conventional method, culture supernatants were purified using MabSelect SuRe to obtain 4 anti-PD-L1/anti-CD3 antibodies.

Hereinafter, the anti-CLDN4/anti-CD3 and anti-PD-L1/anti-CD3 bispecific antibodies are also referred to collectively as "anti-TAA/anti-CD3 bispecific antibodies". The combinations of vectors used for the production of the anti-TAA-anti CD3 bispecific antibodies are shown in Table 9.

**[Table 9]**

| Antibody name | Heavy chain (anti-TAA-Fc) | Light chain (anti-TAA) | Anti-CD3 scFv-Fc (anti-CD3) |
|---|---|---|---|
| CD3_hKM3900_87 | hKM3900(-) | hKM3900_k | ApiD00087 (SEQ ID NO: 14) |
| CD3_hKM3900_88 | hKM3900(-) | hKM3900_k | ApiD00088 (SEQ ID NO: 20) |
| CD3_hKM3900_100 | hKM3900(-) | hKM3900_k | ApiD00100 (SEQ ID NO: 26) |
| CD3_hKM3900_146 | hKM3900(-) | hKM3900_k | ApiD00146 (SEQ ID NO: 24) |
| CD3_atezolizumab_87 | atezolizumab_HC(-) | atezolizumab_LC | ApiD00087 (SEQ ID NO: 14) |
| CD3_atezolizumab_88 | atezolizumab_HC(-) | atezolizumab_LC | ApiD00088 (SEQ ID NO: 20) |
| CD3_atezolizumab_100 | atezolizumab_HC(-) | atezolizumab_LC | ApiD00100 (SEQ ID NO: 26) |
| CD3_atezolizumab_146 | atezolizumab_HC(-) | atezolizumab_LC | ApiD00146 (SEQ ID NO: 24) |

The anti-TAA/anti-CD3 bispecific antibody was analyzed by SDS-PAGE using NuPAGE 4 to 12%, Bis-Tris, 1.0-1.5 mm, Mini Protein Gels (NP0322BOX or NP0329BOX, available from Thermo Fisher Scientific). The anti-TAA/anti-CD3 bispecific antibodies obtained in Example 7-1 were treated with or without addition of NuPAGESample Reducing Agent (10X) (Thermo Fisher Scientific, NP0009), run at 200 V for an appropriate time, and stained with SimplyBlue SafeStain (LC6065, available from Thermo Fisher Scientific), followed by imaging with Gel Doc EZ Imager (available from BioRad) or ChemiDoc Imager (available from BioRad). Precision Plus Protein Unstained Standards (161-0363, available from BIO-RAD) were used as molecular weight markers. In all of the anti-TAA/anti-CD3 bispecific antibodies, a band with mobility corresponding to the molecular weight of interest was observed (Figs. 6-1 and 6-2).

### [Example 7-2: Study of Anti-TAA/Anti-CD3/Anti-CD137 Trispecific Antibody Format]

An anti-TAA/anti-CD3/anti-CD137 trispecific antibody was designed in which a GS linker and the anti-CD 137 scFv antibody described in SEQ ID NO: 26 of International Publication No. WO 2022/224997 were linked in series to the heavy chain carboxyl terminal of the anti-TAA antibody of the anti-TAA/anti-CD3 bispecific antibody described in Example 7-1. DNA encoding the designed polypeptide was synthesized by a conventional method and inserted into a pcDNA 3.4 TOPO vector. The thus prepared vectors are designated as hKM3900(-)_3-34-j6 and atezolizumab(-)_3-34-j6. The vectors were transfected into ExpiCHO-S (R) cells based on combinations shown in Table 10. According to a conventional method, culture supernatants were purified using MabSelect SuRe.

The vectors used for the production of the anti-TAA-anti-CD3/anti-CD137 trispecific antibodies are shown in Table 10.

**[Table 10]**

| Antibody name | Heavy chain | Light chain | Anti-CD3 scFv-Fc |
|---|---|---|---|
| | (anti-TAA-Fc/anti-CD137 scFv) | (anti-TAA) | (anti-CD3) |
| CD3x137_1_hKM3900_87 | hKM3900(-)_3-34-j6 | hKM3900_k | ApiD00087 (SEQ ID NO: 14) |
| CD3x137_1_hKM3900_88 | hKM3900(-)_3-34-j6 | hKM3900_k | ApiD00088 (SEQ ID NO: 20) |
| CD3x137_1_hKM3900_100 | hKM3900(-)_3-34-j6 | hKM3900_k | ApiD00100 (SEQ ID NO: 26) |
| CD3x137_1_hKM3900_146 | hKM3900(-)_3-34-j6 | hKM3900_k | ApiD00146 (SEQ ID NO: 24) |
| CD3x137_1_ atezolizumab_87 | atezolizumab(-)_3-34-j6 | atezolizumab_LC | ApiD00087 (SEQ ID NO: 14) |
| CD3x137_1_ atezolizumab_88 | atezolizumab(-)_3-34-j6 | atezolizumab_LC | ApiD00088 (SEQ ID NO: 20) |
| CD3x137_1_ atezolizumab 100 | atezolizumab(-)_3-34-j6 | atezolizumab_LC | ApiD00100 (SEQ ID NO: 26) |
| CD3x137_1_ atezolizumab 146 | atezolizumab(-)_3-34-j6 | atezolizumab_LC | ApiD00146 (SEQ ID NO: 24) |

The anti-TAA/anti-CD3/anti-CD137 trispecific antibodies were analyzed by SDS-PAGE according to the method described in Example 7-1. In all of the anti-TAA/anti-CD3/anti-CD137 trispecific antibodies, a band with mobility corresponding to the molecular weight of interest was observed (Figs. 7-1 and 7-2).

### [Example 7: In Vitro Activity of Anti-CLDN4/Anti-CD3/Anti-CD137 Trispecific Antibody]

Binding activities of the anti-CLDN4/anti-CD3/anti-CD137 trispecific antibodies to human CD3 were evaluated by the ELISA described in Example 1-4. As a result, even when the anti-CLDN4/anti-CD3 bispecific antibodies were modified, various binding activities to CD3 were not significantly impaired, and the original binding activities were mostly maintained (Fig. 8).

Binding activities of the anti-CLDN4/anti-CD3/anti-CD137 trispecific antibodies to human CD137 were evaluated by the ELISA described in Example 5-2 of International Publication No. WO 2022/224997. All of the anti-CLDN4/anti-CD3/anti-CD137 trispecific antibodies maintained the binding activity to CD137 (Fig. 9).

Binding activities of the anti-CLDN4/anti-CD3/anti-CD137 trispecific antibodies to human CLDN4 were evaluated by cell-based ELISA. Human CLDN4 expressing CHO-K1 described in Example 5-1 of International Publication No. WO 2022/224997 was suspended in MEM α (32571036, available from Thermo Fisher Scientific) containing 10% FBS and 1.5 mg/mL Geneticin (10131027, available from Thermo Fisher Scientific), seeded in a 384 well plate (356662, available from Corning) at 2 × 10⁴ cells/100 uL/well, and cultured overnight. Culture supernatants were removed, and solutions of test antibodies serially dilutions in HBSS (14065056, available from Thermo Fisher Scientific) containing 1% BSA (A3803-50G, available from Sigma-Aldrich) and 10 mM HEPES (17557-94, available from Nacalai Tesque) were added thereto, and the cells were incubated at room temperature for 1 hour. HBSS containing 0.1% BSA and 10 mM HEPES was used as a wash solution, Goat AntiHuman IgG Fc, Multi-Species SP ads-HRP was used as a secondary antibody, TMB (Dako, S1601) was used as a substrate, and 1 mol/L sulfuric acid (198-0959, available from FUJIFILM Wako Pure Chemical Corporation) was used for reaction termination. Absorbance was measured at 450 nm and 570 nm using an Infinite M200PRO (available from TECAN). All of the anti-CLDN4/anti-CD3/anti-CD137 trispecific antibodies maintained the binding activity to CLDN4 (Fig. 10).

### INDUSTRIAL APPLICABILITY

The anti-TAA/anti-CD3 multispecific antibody of the present invention is expected to be useful for treating cancer. The polynucleotide, the expression vector, the host cell containing the expression vector, and the method for producing the antibody of the present invention are also useful for producing the anti-TAA/anti-CD3 multispecific antibody.

### SEQUENCE LISTING FREE TEXT

SEQ ID NOs: 2 and 8 represent heavy chain amino acid sequences of the anti-TSPAN8 antibody, and the nucleotide sequences set forth in SEQ ID NOs: 1 and 7 are nucleotide sequences encoding the heavy chain amino acid sequences of the anti-TSPAN8 antibody set forth in SEQ ID NOs: 2 and 8. SEQ ID NO: 4 represents a light chain amino acid sequence of the anti-TSPAN8 antibody, and the nucleotide sequence set forth in SEQ ID NO: 3 is a nucleotide sequence encoding the light chain amino acid sequence of the anti-TSPAN8 antibody set forth in SEQ ID NO: 4. SEQ ID NO: 10 represents a heavy chain amino acid sequence of the anti-CD37 antibody, and the nucleotide sequence set forth in SEQ ID NO: 9 is a nucleotide sequence encoding the heavy chain amino acid sequence of the anti-CD37 antibody set forth in SEQ ID NO: 10. SEQ ID NO: 12 represents a light chain amino acid sequence of the anti-CD37 antibody, and the nucleotide sequence set forth in SEQ ID NO: 11 is a nucleotide sequence encoding the light chain amino acid sequence of the anti-CD37 antibody set forth in SEQ ID NO: 12. SEQ ID NO: 6, 14, 16, 18, 20, 22, 24, 26, 28, 30, 32, 34, 36, 38, 40, 42, or 44 is an amino acid sequence of a polypeptide in which the anti-CD3 scFv region and the second Fc polypeptide are linked, and the nucleotide sequence set forth in SEQ ID NO: 5, 13, 15, 17, 19, 21, 23, 25, 27, 29, 31, 33, 35, 37, 39, 41, or 43 is a nucleotide sequence encoding the amino acid sequence of the polypeptide, set forth in SEQ ID NO: 6, 14, 16, 18, 20, 22, 24, 26, 28, 30, 32, 34, 36, 38, 40, 42, or 44, in which the anti-CD3 scFv region and the second Fc polypeptide are linked. SEQ ID NO: 46, 48, 50, 52, or 54 is an amino acid sequence of the anti-CD 137 scFv region, and the nucleotide sequence set forth in SEQ ID NO: 45, 47, 49, 51, or 53 is a nucleotide sequence encoding the amino acid sequence of the anti-CD 137 scFv region set forth in SEQ ID NO: 46, 48, 50, 52, or 54. SEQ ID NOs: 55 to 63 are amino acid sequences of the various linkers described in the detailed description of the invention. SEQ ID NO: 65 or 67 is an amino acid sequence of the anti-SLC34A2 antibody, and the nucleotide sequence set forth in SEQ ID NO: 64 or 66 is a nucleotide sequence encoding the amino acid sequence of the anti-SLC34A2 antibody set forth in SEQ ID NO: 65 or 67.

## Claims

1. A multispecific antibody comprising: a heavy chain variable region and a light chain variable region of an anti-tumor associated antigen (TAA) antibody that binds to TAA; and an anti-CD3 scFv region comprising a heavy chain variable region and a light chain variable region of an anti-CD3 antibody that binds to CD3, wherein the anti-CD3 scFv region is any one of (1) to (16):
(1) an anti-CD3 scFv region consisting of an amino acid sequence at amino acid positions 1 to 254 of SEQ ID NO: 14;
(2) an anti-CD3 scFv region consisting of an amino acid sequence at amino acid positions 1 to 254 of SEQ ID NO: 16;
(3) an anti-CD3 scFv region consisting of an amino acid sequence at amino acid positions 1 to 254 of SEQ ID NO: 18;
(4) an anti-CD3 scFv region consisting of an amino acid sequence at amino acid positions 1 to 254 of SEQ ID NO: 20;
(5) an anti-CD3 scFv region consisting of an amino acid sequence at amino acid positions 1 to 254 of SEQ ID NO: 22;
(6) an anti-CD3 scFv region consisting of an amino acid sequence at amino acid positions 1 to 254 of SEQ ID NO: 24;
(7) an anti-CD3 scFv region consisting of an amino acid sequence at amino acid positions 1 to 254 of SEQ ID NO: 26;
(8) an anti-CD3 scFv region consisting of an amino acid sequence at amino acid positions 1 to 254 of SEQ ID NO: 28;
(9) an anti-CD3 scFv region consisting of an amino acid sequence at amino acid positions 1 to 254 of SEQ ID NO: 30;
(10) an anti-CD3 scFv region consisting of an amino acid sequence at amino acid positions 1 to 254 of SEQ ID NO: 32;
(11) an anti-CD3 scFv region consisting of an amino acid sequence at amino acid positions 1 to 254 of SEQ ID NO: 34;
(12) an anti-CD3 scFv region consisting of an amino acid sequence at amino acid positions 1 to 254 of SEQ ID NO: 36;
(13) an anti-CD3 scFv region consisting of an amino acid sequence at amino acid positions 1 to 254 of SEQ ID NO: 38;
(14) an anti-CD3 scFv region consisting of an amino acid sequence at amino acid positions 1 to 254 of SEQ ID NO: 40;
(15) an anti-CD3 scFv region consisting of an amino acid sequence at amino acid positions 1 to 254 of SEQ ID NO: 42; and
(16) an anti-CD3 scFv region consisting of an amino acid sequence at amino acid positions 1 to 254 of SEQ ID NO: 44.

2. The multispecific antibody according to claim 1, wherein the multispecific antibody is a bispecific antibody having structures (a) to (c):
(a) a Fab region of an anti-TAA antibody consisting of a heavy chain fragment comprising a heavy chain variable region of the anti-TAA antibody and a light chain comprising a light chain variable region of the anti-TAA antibody;
(b) an anti-CD3 scFV region comprising a heavy chain variable region and a light chain variable region of an anti-CD3 antibody; and
(c) an Fc region consisting of a first Fc polypeptide linked to the heavy chain fragment of the Fab region (a) and a second Fc polypeptide linked to the anti-CD3 scFv region (b).

3. The multispecific antibody according to claim 2, wherein the Fc region comprises knobs-into-holes mutation or LALA mutation, L234A and L235A, where positions of the mutation are amino acid positions in human Igγ1 constant region according to EU index.

4. The multispecific antibody according to claim 2, wherein the Fc region comprises knobs-into-holes mutation and LALA mutation.

5. The multispecific antibody according to any one of claims 2 to 4, wherein the Fc region comprises P329A mutation and/or P331G mutation, where a position of the mutation is an amino acid position in human Igγ1 constant region according to EU index.

6. The multispecific antibody according to any one of claims 2 to 5, wherein the heavy chain fragment of the Fab region of the anti-TAA antibody and the first Fc polypeptide are linked via a hinge region; and the anti-CD3 scFv region and the second Fc polypeptide are linked via a hinge region.

7. The multispecific antibody according to claim 6, wherein the hinge region linking the anti-CD3 scFv region to the second Fc polypeptide comprises C220S mutation, where a position of the mutation is an amino acid position in human Igγ1 constant region according to EU index.

8. The multispecific antibody according to claim 6, wherein the Fc region comprises knobs-into-holes mutation, LALA mutation, and P329A and/or P331G mutation, wherein the hinge region linking anti-CD3 scFv region to the second Fc polypeptide comprises C220S mutation, where a position of the mutation is an amino acid position in human Igγ1 constant region according to EU index.

9. A multispecific antibody which is an anti-tumor associated antigen (TAA)/anti-CD3 bispecific antibody, comprising: a heavy chain of an anti-TAA antibody that binds to TAA, in which a first Fc polypeptide is linked to a heavy chain fragment comprising a heavy chain variable region of the anti-TAA antibody; a light chain comprising a light chain variable region of the anti-TAA antibody; and a polypeptide in which an anti-CD3 scFv region comprising a heavy chain variable region and a light chain variable region of an anti-CD3 antibody that binds to CD3 and a second Fc polypeptide are linked, wherein the anti-CD3 scFv region is any one of (1) to (16):
(1) an anti-CD3 scFv region consisting of an amino acid sequence at amino acid positions 1 to 254 of SEQ ID NO: 14;
(2) an anti-CD3 scFv region consisting of an amino acid sequence at amino acid positions 1 to 254 of SEQ ID NO: 16;
(3) an anti-CD3 scFv region consisting of an amino acid sequence at amino acid positions 1 to 254 of SEQ ID NO: 18;
(4) an anti-CD3 scFv region consisting of an amino acid sequence at amino acid positions 1 to 254 of SEQ ID NO: 20;
(5) an anti-CD3 scFv region consisting of an amino acid sequence at amino acid positions 1 to 254 of SEQ ID NO: 22;
(6) an anti-CD3 scFv region consisting of an amino acid sequence at amino acid positions 1 to 254 of SEQ ID NO: 24;
(7) an anti-CD3 scFv region consisting of an amino acid sequence at amino acid positions 1 to 254 of SEQ ID NO: 26;
(8) an anti-CD3 scFv region consisting of an amino acid sequence at amino acid positions 1 to 254 of SEQ ID NO: 28;
(9) an anti-CD3 scFv region consisting of an amino acid sequence at amino acid positions 1 to 254 of SEQ ID NO: 30;
(10) an anti-CD3 scFv region consisting of an amino acid sequence at amino acid positions 1 to 254 of SEQ ID NO: 32;
(11) an anti-CD3 scFv region consisting of an amino acid sequence at amino acid positions 1 to 254 of SEQ ID NO: 34;
(12) an anti-CD3 scFv region consisting of an amino acid sequence at amino acid positions 1 to 254 of SEQ ID NO: 36;
(13) an anti-CD3 scFv region consisting of an amino acid sequence at amino acid positions 1 to 254 of SEQ ID NO: 38;
(14) an anti-CD3 scFv region consisting of an amino acid sequence at amino acid positions 1 to 254 of SEQ ID NO: 40;
(15) an anti-CD3 scFv region consisting of an amino acid sequence at amino acid positions 1 to 254 of SEQ ID NO: 42; and
(16) an anti-CD3 scFv region consisting of an amino acid sequence at amino acid positions 1 to 254 of SEQ ID NO: 44.

10. A multispecific antibody which is a trispecific antibody obtained by further adding an anti-CD137 scFv region comprising a heavy chain variable region and a light chain variable region of an anti-CD 137 antibody that binds to CD137 to the multispecific antibody according to any one of claims 2 to 9, wherein the anti-CD137 scFv region is any one of (a) to (e):
(a) an anti-CD137 scFv region consisting of an amino acid sequence at amino acid positions 1 to 242 of SEQ ID NO: 46;
(b) an anti-CD137 scFv region consisting of an amino acid sequence at amino acid positions 1 to 244 of SEQ ID NO: 48;
(c) an anti-CD137 scFv region consisting of an amino acid sequence at amino acid positions 1 to 249 of SEQ ID NO: 50;
(d) an anti-CD137 scFv region consisting of an amino acid sequence at amino acid positions 1 to 244 of SEQ ID NO: 52; and
(e) an anti-CD137 scFv region consisting of an amino acid sequence at amino acid positions 1 to 247 of SEQ ID NO: 54.

11. The multispecific antibody according to claim 10, wherein the anti-CD137 scFv region is linked to one or both of carboxy terminals of the first Fc polypeptide and the second Fc polypeptide.

12. A multispecific antibody which is an anti-tumor associated antigen (TAA)/anti-CD3/anti-CD137 trispecific antibody, comprising: a heavy chain of an anti-TAA antibody that binds to TAA, in which a first Fc polypeptide is linked to a heavy chain fragment comprising a heavy chain variable region of the anti-TAA antibody; a light chain comprising a light chain variable region of the anti-TAA antibody; and a polypeptide in which an anti-CD3 scFv region comprising a heavy chain variable region and a light chain variable region of an anti-CD3 antibody that binds to CD3 and a second Fc polypeptide are linked, wherein an anti-CD137 scFv region comprising a heavy chain variable region and a light chain variable region of an anti-CD137 antibody is also linked to one or both of carboxy terminals of the first Fc polypeptide and the second Fc polypeptide,
wherein the anti-CD3 scFv region is any one of (1) to (16):
(1) an anti-CD3 scFv region consisting of an amino acid sequence at amino acid positions 1 to 254 of SEQ ID NO: 14;
(2) an anti-CD3 scFv region consisting of an amino acid sequence at amino acid positions 1 to 254 of SEQ ID NO: 16;
(3) an anti-CD3 scFv region consisting of an amino acid sequence at amino acid positions 1 to 254 of SEQ ID NO: 18;
(4) an anti-CD3 scFv region consisting of an amino acid sequence at amino acid positions 1 to 254 of SEQ ID NO: 20;
(5) an anti-CD3 scFv region consisting of an amino acid sequence at amino acid positions 1 to 254 of SEQ ID NO: 22;
(6) an anti-CD3 scFv region consisting of an amino acid sequence at amino acid positions 1 to 254 of SEQ ID NO: 24;
(7) an anti-CD3 scFv region consisting of an amino acid sequence at amino acid positions 1 to 254 of SEQ ID NO: 26;
(8) an anti-CD3 scFv region consisting of an amino acid sequence at amino acid positions 1 to 254 of SEQ ID NO: 28;
(9) an anti-CD3 scFv region consisting of an amino acid sequence at amino acid positions 1 to 254 of SEQ ID NO: 30;
(10) an anti-CD3 scFv region consisting of an amino acid sequence at amino acid positions 1 to 254 of SEQ ID NO: 32;
(11) an anti-CD3 scFv region consisting of an amino acid sequence at amino acid positions 1 to 254 of SEQ ID NO: 34;
(12) an anti-CD3 scFv region consisting of an amino acid sequence at amino acid positions 1 to 254 of SEQ ID NO: 36;
(13) an anti-CD3 scFv region consisting of an amino acid sequence at amino acid positions 1 to 254 of SEQ ID NO: 38;
(14) an anti-CD3 scFv region consisting of an amino acid sequence at amino acid positions 1 to 254 of SEQ ID NO: 40;
(15) an anti-CD3 scFv region consisting of an amino acid sequence at amino acid positions 1 to 254 of SEQ ID NO: 42; and
(16) an anti-CD3 scFv region consisting of an amino acid sequence at amino acid positions 1 to 254 of SEQ ID NO: 44, and
wherein the anti-CD 137 scFv region is any one of (a) to (e):
(a) an anti-CD137 scFv region consisting of an amino acid sequence at amino acid positions 1 to 242 of SEQ ID NO: 46;
(b) an anti-CD137 scFv region consisting of an amino acid sequence at amino acid positions 1 to 244 of SEQ ID NO: 48;
(c) an anti-CD137 scFv region consisting of an amino acid sequence at amino acid positions 1 to 249 of SEQ ID NO: 50;
(d) an anti-CD137 scFv region consisting of an amino acid sequence at amino acid positions 1 to 244 of SEQ ID NO: 52; and
(e) an anti-CD137 scFv region consisting of an amino acid sequence at amino acid positions 1 to 247 of SEQ ID NO: 54.

13. The multispecific antibody according to any one of claims 1 to 12, wherein the anti-TAA antibody is an anti-TAA antibody that binds to any one of TAAs:
HER2, HER3, HER4, CD19, CD20, CD22, CD33, CD38, CD40, CD44, CD70, CD123, CD138, CXCR3, CXCR5, CCR3, CCR4, CCR9, CRTH2, PMCH, CD4, CD25, CD200, endoplasmin, BCMA, CD276, TSPAN8, CLDN4, CLDN6, CLDN18.2, ENPP3, SLC34A2, TROP2, MerTK, Nectin-4, ASGR1, mesothelin, PSMA, LIV-1, MUC1, MUC2, MUC4, MUC16, CDH6, CEACAM1, CEACAM3, CEACAM4, CEACAM5, CEACAM6, CEACAM7, CEACAM16, CEACAM18, CEACAM19, CEACAM20, CEACAM21, URLC10, NY-ESO-1, GAA, OFA, cyclinB1, WT-1, CEF, VEGRR1, VEGFR2, TTK, HPV16, HPV16E7, HPV18, CEAIMA910, KOC1, SL-701, MART-1, gp100, tyrosinase, survivin, MAGE-3.1, MAGE-10.A2, OVA BiP, gp209-2M, melan-A, NA17.A2, KOC1, CO16, DEPDC1, MPHOSPH1, MAGE12, ONT-10, GD2L, GD3L, URLC10, CDCA1, TF, PSA, TERT, STF-II, G17DT, ICT-107, Dex2, hTERT, PAP, TRP2, LRRC15, c-Met, PD-L1, FAP, EGFR, B3-H3, GPC3, and 5T4.

14. The multispecific antibody according to any one of claims 1 to 13, which is post-translationally modified.

15. The multispecific antibody according to claim 14, wherein the post-translational modification is pyroglutamylation at an amino terminal of the heavy chain variable region and/or lysine deletion at a heavy chain carboxy terminal.

16. A polynucleotide for use in production of the multispecific antibody according to any one of claims 1 to 15, the polynucleotide being selected from the group consisting of (1) to (16):
(1) a polynucleotide comprising a nucleotide sequence encoding the anti-CD3 scFv region consisting of an amino acid sequence at amino acid positions 1 to 254 of SEQ ID NO: 14;
(2) a polynucleotide comprising a nucleotide sequence encoding the anti-CD3 scFv region consisting of an amino acid sequence at amino acid positions 1 to 254 of SEQ ID NO: 16;
(3) a polynucleotide comprising a nucleotide sequence encoding the anti-CD3 scFv region consisting of an amino acid sequence at amino acid positions 1 to 254 of SEQ ID NO: 18;
(4) a polynucleotide comprising a nucleotide sequence encoding the anti-CD3 scFv region consisting of an amino acid sequence at amino acid positions 1 to 254 of SEQ ID NO: 20;
(5) a polynucleotide comprising a nucleotide sequence encoding the anti-CD3 scFv region consisting of an amino acid sequence at amino acid positions 1 to 254 of SEQ ID NO: 22;
(6) a polynucleotide comprising a nucleotide sequence encoding the anti-CD3 scFv region consisting of an amino acid sequence at amino acid positions 1 to 254 of SEQ ID NO: 24;
(7) a polynucleotide comprising a nucleotide sequence encoding the anti-CD3 scFv region consisting of an amino acid sequence at amino acid positions 1 to 254 of SEQ ID NO: 26;
(8) a polynucleotide comprising a nucleotide sequence encoding the anti-CD3 scFv region consisting of an amino acid sequence at amino acid positions 1 to 254 of SEQ ID NO: 28;
(9) a polynucleotide comprising a nucleotide sequence encoding the anti-CD3 scFv region consisting of an amino acid sequence at amino acid positions 1 to 254 of SEQ ID NO: 30;
(10) a polynucleotide comprising a nucleotide sequence encoding the anti-CD3 scFv region consisting of an amino acid sequence at amino acid positions 1 to 254 of SEQ ID NO: 32;
(11) a polynucleotide comprising a nucleotide sequence encoding the anti-CD3 scFv region consisting of an amino acid sequence at amino acid positions 1 to 254 of SEQ ID NO: 34;
(12) a polynucleotide comprising a nucleotide sequence encoding the anti-CD3 scFv region consisting of an amino acid sequence at amino acid positions 1 to 254 of SEQ ID NO: 36;
(13) a polynucleotide comprising a nucleotide sequence encoding the anti-CD3 scFv region consisting of an amino acid sequence at amino acid positions 1 to 254 of SEQ ID NO: 38;
(14) a polynucleotide comprising a nucleotide sequence encoding the anti-CD3 scFv region consisting of an amino acid sequence at amino acid positions 1 to 254 of SEQ ID NO: 40;
(15) a polynucleotide comprising a nucleotide sequence encoding the anti-CD3 scFv region consisting of an amino acid sequence at amino acid positions 1 to 254 of SEQ ID NO: 42; and
(16) a polynucleotide comprising a nucleotide sequence encoding the anti-CD3 scFv region consisting of an amino acid sequence at amino acid positions 1 to 254 of SEQ ID NO: 44.

17. A polynucleotide for use in production of the multispecific antibody according to any one of claims 10 to 15, the polynucleotide being selected from the group consisting of (a) to (e):
(a) a polynucleotide comprising a nucleotide sequence encoding an anti-CD 137 scFv region consisting of an amino acid sequence at amino acid positions 1 to 242 of SEQ ID NO: 46;
(b) a polynucleotide comprising a nucleotide sequence encoding an anti-CD 137 scFv region consisting of an amino acid sequence at amino acid positions 1 to 244 of SEQ ID NO: 48;
(c) a polynucleotide comprising a nucleotide sequence encoding an anti-CD 137 scFv region consisting of an amino acid sequence at amino acid positions 1 to 249 of SEQ ID NO: 50;
(d) a polynucleotide comprising a nucleotide sequence encoding an anti-CD 137 scFv region consisting of an amino acid sequence at amino acid positions 1 to 244 of SEQ ID NO: 52; and
(e) a polynucleotide comprising a nucleotide sequence encoding an anti-CD 137 scFv region consisting of an amino acid sequence at amino acid positions 1 to 247 of SEQ ID NO: 54.

18. An expression vector comprising the polynucleotide according to claim 16 or 17.

19. A host cell comprising the polynucleotide according to claim 16 or 17 or the expression vector according to claim 18.

20. A production method comprising a step of culturing the host cell according to claim 19.

21. A pharmaceutical composition comprising the multispecific antibody according to any one of claims 1 to 15 and a pharmaceutically acceptable excipient.

22. The multispecific antibody according to any one of claims 1 to 15, for use of treatment cancer.

23. The pharmaceutical composition according to claim 21, for use of treatment cancer.

24. A method for treating cancer, comprising administering, to a subject, a therapeutically effective amount of the multispecific antibody according to any one of claims 1 to 15.

25. Use of the multispecific antibody according to any one of claims 1 to 15 in production of a pharmaceutical composition for the treatment of cancer.
